(19) Europäisches Patentamt
European Patent Office
Office européen des brevets

(11) Publication number: 0 268 892 A2

(12) EUROPEAN PATENT APPLICATION

(21) Application number: 87116271.5

(22) Date of filing: 05.11.87

(51) Int. Cl.4: C07D 409/12 , C07D 405/12 , C07D 231/14 , C07D 413/12 , C07D 417/12 , A61K 31/415 , A61K 31/42 , A61K 31/425

(30) Priority: 06.11.86 JP 264763/86
28.04.87 JP 103044/87
28.05.87 JP 133048/87

(43) Date of publication of application: 01.06.88 Bulletin 88/22

(84) Designated Contracting States: AT BE CH DE ES FR GB GR IT LI LU NL SE

(71) Applicant: NISSAN CHEMICAL INDUSTRIES LTD.
3-7-1, Kanda Nishiki-cho
Chiyoda-ku Tokyo(JP)

(72) Inventor: Suzuki, Hideo Nissan Chemical Ind. Ltd.
Central Research Institute 722-1, Tsuboi-cho
Funabashi-shi Chiba-ken(JP)
Inventor: Mita, Takeshi Nissan Chemical Ind. Ltd.
Central Research Institute 722-1, Tsuboi-cho
Funabashi-shi Chiba-ken(JP)
Inventor: Takeyama, Toshiaki Nissan Chemical Ind. Ltd.
Central Research Institute 722-1, Tsuboi-cho
Funabashi-shi Chiba-ken(JP)
Inventor: Ochciai, Yoshinori c/o Nissan Chem. Ind. Ltd.
1470, Ohaza Shiraoka Shiraoka-machi
Minamisaitama-gun Saitama-ken(JP)
Inventor: Hanaue, Masami c/o Nissan Chem. Ind. Ltd.
1470, Ohaza Shiraoka Shiraoka-machi
Minamisaitama-gun Saitama-ken(JP)
Inventor: Nishikubo, Masao c/o Nissan Chem. Ind. Ltd.
1470, Ohaza Shiraoka Shiraoka-machi
Minamisaitama-gun Saitama-ken(JP)
Inventor: Yamagishi, Kazuhiro c/o Nissan Chem. Ind. Ltd.
1470, Ohaza Shiraoka Shiraoka-machi
Minamisaitama-gun Saitama-ken(JP)

(74) Representative: Patentanwälte Schaad, Balass & Partner
Dufourstrasse 101 Postfach
CH-8034 Zürich(CH)

(54) Substituted-amido derivatives, method for preparation of the same and phytopathogenic fungicides containing the same.



Xerox Copy Centre

(57) Disclosed are substituted-amido derivatives of a general formula (I):

$$A\text{-}CONH\underset{\displaystyle B}{\underset{|}{C}}H\text{-}D \qquad (I)$$

in which A represents a 5-membered hetero-aromatic group as specifically defined herein; B represents a 5-membered hetero-aromatic group as specifically defined herein or represents a group of $OR_7$ or $SR_7$; D represents a cyano, thiocarbamoyl or acylthiocarbamoyl group of $-CN$, $-CSNH_2$ or $-CSNHCOR_8$; and $R_7$ and $R_8$ each represent a group as specifically defiend herein, as well as a method for preparation of the said derivatives and a fungicide containing the said derivative as an active ingredient.

# SUBSTITUTED-AMIDO DERIVATIVES, METHOD FOR PREPARATION OF THE SAME AND PHYTOPATHOGENIC FUNGICIDES CONTAINING THE SAME

## BACKGROUND OF THE INVENTION

### Field of the Invention:

The present invention relates to new substituted-amido derivatives, method for preparation of the said derivatives and fungicides for agricultural and horticultural use containing the same as an active ingredient.

### Description of the Prior Art:

One important problem in agricultural production, especially in culture of fruit trees and vegetables, includes diseases caused by phytopathogenic fungi of Phycomycetes (for example, Pseudoperonospora-caused diseases, Phytophthora-caused diseases, etc.). The diseases caused by the said fungi are known to be hardly protected because of the special physiological and biological properties of the said fungi, and therefore, the development of excepted chemical agents capable of protecting against the said fungi. The extent of damage caused by the said fungi of Pseudoperonospora or Phytophthora is great to broadly cover various crops, and the said damage is extremely serious.

At present, captan (common name), captafol (common name), dithiocarbamate fungicides (for example, zineb (common name), etc.), chlorothalonil (common name), etc. are being widely used against diseases caused by phytopathogenic fungi of Phycomycetes. However, these fungicides are essentially for the purpose of prevention of diseases and could not almost be expected to have a curative effect, and therefore, these have a fatal defect in that these could not display a sufficient effect when these are applied to diseased plants. A fungicide of an acylalanine series compound, which has recently been developed, for example, metalaxyl (common name), etc., has both preventive effect and curative effect. However, tolerant fungi against the said fungicide were already present, and therefore, the protective effect of the said fungicide has become fairly lowered.

Hitherto, substituted-amido acetonitrile derivatives, substituted-amido thioacetamide derivatives and substituted-amido N-acylthioacetamide derivatives, which are considered to be similar to the compounds of the present invention in view of the chemical structures, have already been reported in Japanese Patent Application Laid-Open Nos. 167978/82, 69866/83 and 255759/85 (the term "OPI" as herein referred to means a "published unexamined Japanese patent application"). However, the prior art cannot be said sufficient to provide any practical fungicides for agricultural and horticultural use,and in particular, the said known compounds would impart some chemical phytotoxicity against crops, which is one serious outstanding problem.

## SUMMARY OF THE INVENTION

One object of the present invention is to provide new amido-substituted derivatives having a fungicidal activity.

Another object of the present invention is to provide a method for preparation of the said amido-substituted derivatives.

Still another object of the present invention is to provide a fungicide which has both preventive effect and curative effect against diseases caused by phytopathogenic fungi of Phycomycetes (for example, Pseudoperonospora-caused diseases, Phytophthora-caused diseases, etc.) and which does not impart any chemical phytotoxicity against crops.

Further object of the present invention is to provide a method for protection against phytopathogenic fungi by the use of the above-mentioned amido-substituted derivatives.

These and other objects and many of the attendant advantages of this invention will be readily appreciated as the same becomes better understood by reference to the following detailed description.

DETAILED DESCRIPTION OF THE INVENTION

The present inventors earnestly studied the above-mentioned amido-derivatives in order to overcome the said problems, and as a result, have found that the compounds as represented by the following general formula (I) have both preventive effect and curative effect against Pseudoperonospora-caused diseases, Phytophthora-caused diseases, etc. of various plants with an extremely low chemical phytotoxicity against crops,as the characteristic feature thereof, and therefore have achieved the present invention. The compounds of the present invention are new compounds which are not described in any publications up to the present.

Specifically, the present invention provides new amide derivatives of a general formula (I):

$A\text{-}CONH\underset{\underset{B}{|}}{C}H\text{-}D$ (I)

in which A represents a 5-membered hetero-aromatic group of a formula:

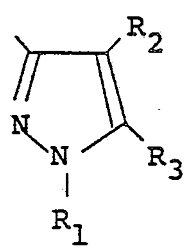 , 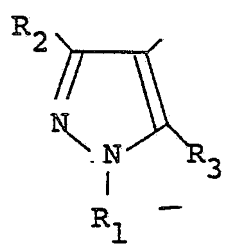 , 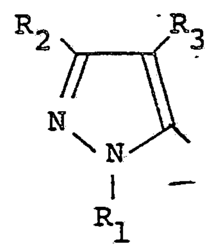 ,

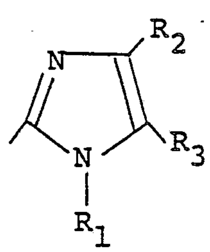 , 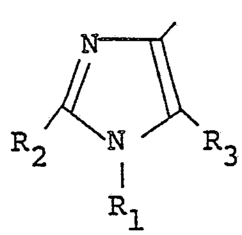 , 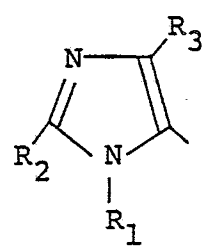 ,

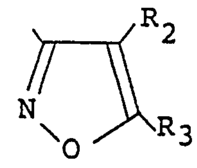 , 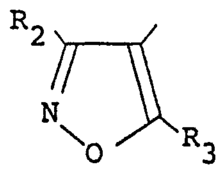 , 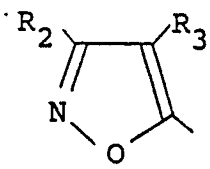 ,

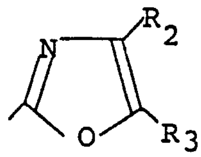 , 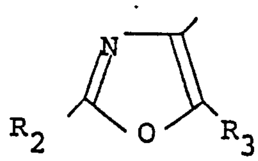 , 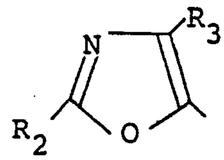 ,

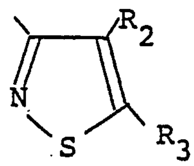 , 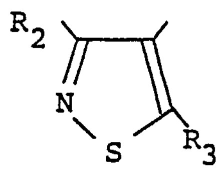 , 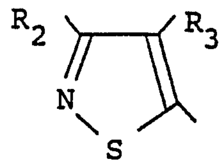 ,

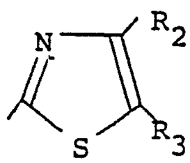 , 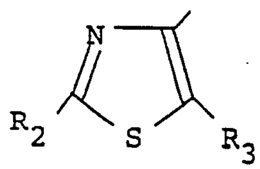 or

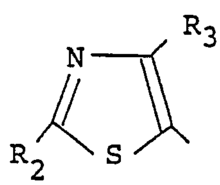 ;

B represents a 5-membered hetero-aromatic group of a formula:

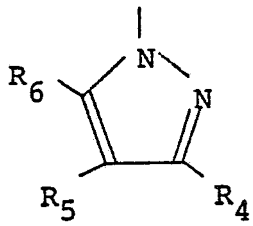 , 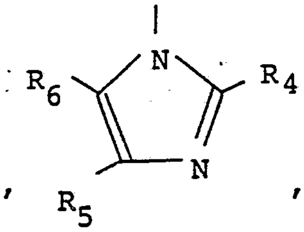 , 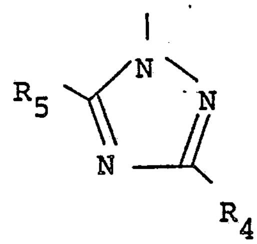

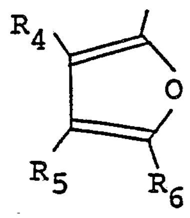 , 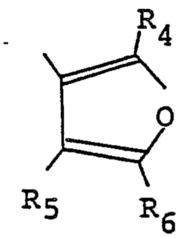 , 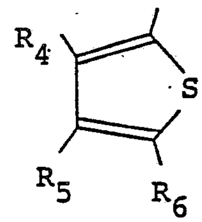

or 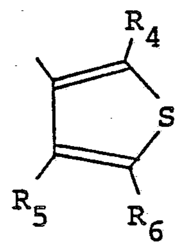 ,

or represents a group of $OR_7$ or $SR_7$;

D represents a cyano, thiocarbamoyl or acylthiocarbamoyl group of $-CN$, $-CSNH_2$ or $-CSNHCOR_8$;

in the said groups, $R_1$ represents a hydrogen atom, a lower alkyl group having from 1 to 4 carbon atoms, a halogenated alkyl group having from 1 to 4 carbon atoms, an alkenyl group having from 2 to 4 carbon atoms, a halogenated alkenyl group having from 2 to 4 carbon atoms, an alkynyl group having from 2 to 4 carbon atoms or an allenyl group having from 3 to 5 carbon atoms;

$R_2$ and $R_3$ independently represent a hydrogen atom, a lower alkyl group having from 1 to 4 carbon atoms, a halogen atom, a halogenated alkyl group having from 1 to 4 carbon atoms, an alkoxy group having from 1 to 4 carbon atoms, a cyano group or a lower alkylthio group having from 1 to 4 carbon atoms;

$R_4$, $R_5$ and $R_6$ independently represent a hydrogen atom, a lower alkyl group having from 1 to 4 carbon atoms, a halogen atom or a halogenated alkyl group having from 1 to 4 carbon atoms;

$R_7$ represents a lower alkyl group having from 1 to 4 carbon atoms, a halogenated alkyl group having from 1 to 4 carbon atoms, an alkenyl group having from 2 to 4 carbon atoms, an alkynyl group having from 2 to 4 carbon atoms, an alkoxyalkyl group having from 3 to 4 carbon atoms, a halogenated alkynyl group having from 3 to 6 carbon atoms, a cyanoalkyl group having from 2 to 4 carbon atoms or a halogenated alkenyl group having from 2 to 4 carbon atoms; and

$R_8$ represents a lower alkyl group having from 1 to 4 carbon atoms, a cyclohexyl group or a phenyl group;

as well as a method for preparation of the said amide derivatives and a phytopaghogenic fungicide containing the said amide derivative as an active ingredient.

Among the above-mentioned amide derivatives of the present invention, compounds which are preferred in the viewpoint of the fungicidal activity are those of the said general formula (I) in which A represents a 5-membered hetero-aromatic group of a formula:

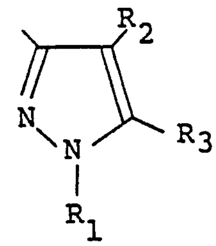,
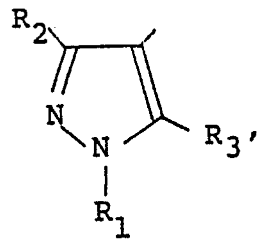,
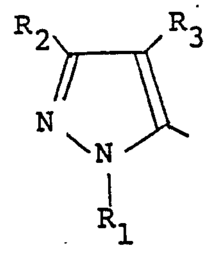,

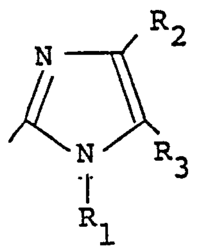,
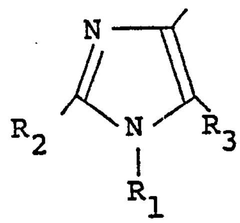,
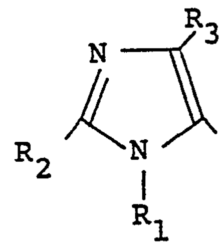,

B represents a 5-membered hetero-aromatic group of a formula:

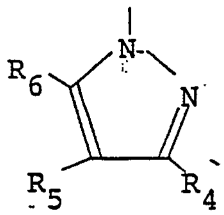,
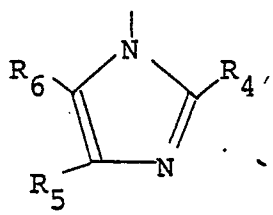,
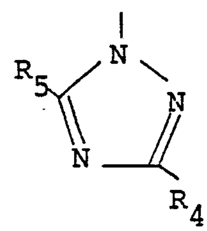,
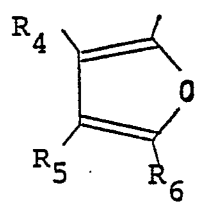,

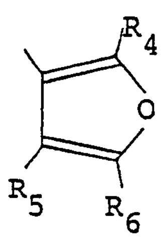,
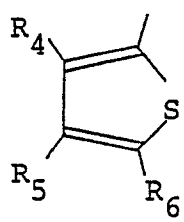
or
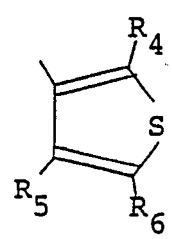,

or represents a group of $OR_7$ or $SR_7$;

D represents a cyano, thiocarbamoyl or acylthiocarbamoyl group of -CN, $-CSNH_2$ or $-CSNHCOR_8$;

in these groups, $R_1$ represents a hydrogen atom, a lower alkyl group having from 1 to 4 carbon atoms, a halogenated alkyl group having from 1 to 4 carbon atoms, an alkenyl group having from 2 to 4 carbon atoms, a halogenated alkenyl group having from 2 to 4 carbon atoms, an alkynyl group having from 2 to 4 carbon atoms or an allenyl group having from 3 to 5 carbon atoms;

$R_2$ and $R_3$ indepedently represent a hydrogen atom, a lower alkyl group having from 1 to 4 carbon atoms, a halogen atom, a halogenated alkyl group having from 1 to 4 carbon atoms, an alkoxy group having from 1 to 4 carbon atoms, a cyano group or a lower alkylthio group having from 1 to 4 carbon atoms;

$R_4$, $R_5$ and $R_6$ independently represent a hydrogen atom, a lower alkyl group having from 1 to 4 carbon atoms, a halogen atom or a halogenated alkyl group having from 1 to 4 carbon atoms;

$R_7$ represents a lower alkyl group having from 1 to 4 carbon atoms, a halogenated alkyl group having from 1 to 4 carbon atoms, an alkenyl group having from 2 to 4 carbon atoms, an alkynyl group having from 2 to 4 carbon atoms, an alkoxyalkyl group having from 3 to 4 carbon atoms, a halogenated alkynyl group having from 3 to 6 carbon atoms, a cyanoalkyl group having from 2 to 4 carbon atoms or a halogenated alkenyl group having from 2 to 4 carbon atoms; and

$R_8$ represents a lower alkyl group having from 1 to 4 carbon atoms, a cyclohexyl group or a phenyl group.

In the practice of the present invention, more preferred compounds are those of a general formula (IA):

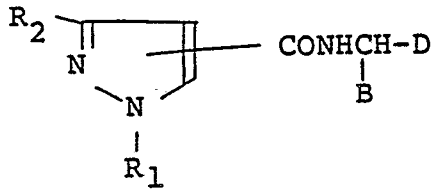

(IA)

in which $R_1$ represents an alkyl group having form 1 to 3 carbon atoms;
$R_2$ represents a hydrogen atom, a methyl group, a methoxy group or a halogen atom;
D represents -CN or $-\overset{\overset{\displaystyle S}{\|}}{C}-NH_2$; and
B represents a group of a formula:

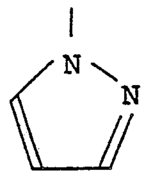,
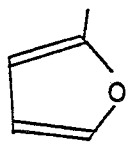,
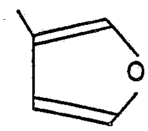,
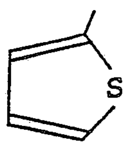
or
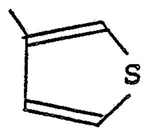.

Among the compounds of the present invention, especially preferred compounds are concretely mentioned hereinafter.

(1) No. 765:

N-(cyano-3-thienylmethyl)-1,3-dimethyl-1H-pyrazole-5-carboxamide.

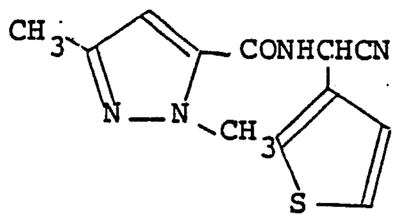

(2) No. 91:

N-(cyano-2-thienylmethyl)-1,3-dimethyl-1H-pyrazole-5-carboxamide.

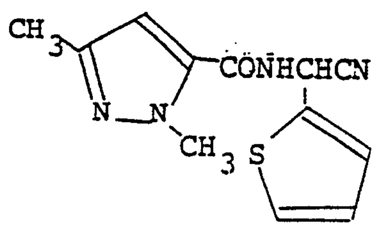

(3) No. 766:

1,3-Dimethyl-N-[3-thienyl(thiocarbamoyl)methyl]-1H-pyrazole-5-carboxamide.

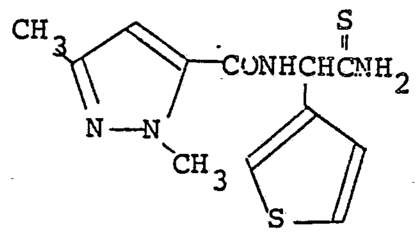

(4) No. 92:

1,3-Dimethyl-N-[2-thienyl(thiocarbamoyl)methyl]-1H-pyrazole-5-carboxamide.

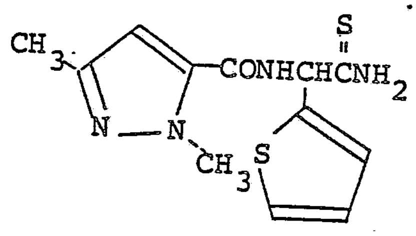

(5) No. 26:

N-(cyano-2-furanylmethyl)-1-methyl-1H-pyrazole-4-carboxamide.

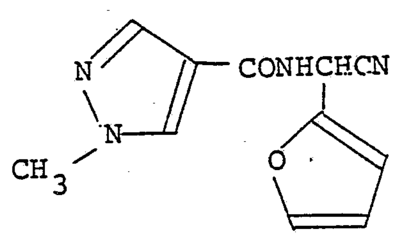

(6) No. 16:

N-(cyano-1H-pyrazol-1-ylmethyl)-1-methyl-1H-pyrazole-4-carboxamide.

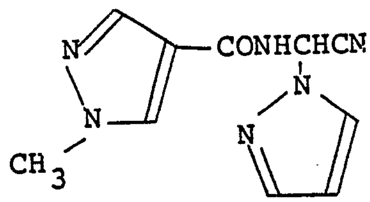

(7) No. 80:

N-(cyano-2-furanylmethyl)-1,3-dimethyl-1H-pyrazole-5-carboxamide.

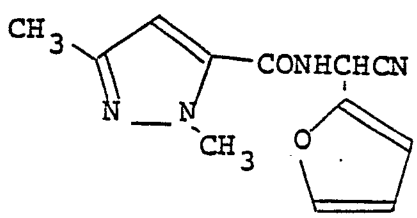

(8) No. 825:

1-Ethyl-3-methyl-N-[3-thienyl(thiocarbamoyl)methyl]-1H-pyrazole-5-carboxamide.

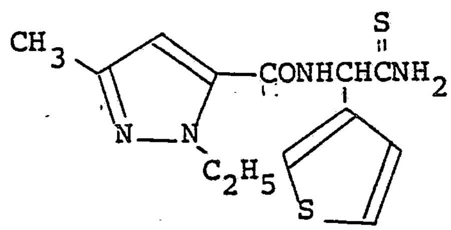

(9) No. 59:

N-(cyano-1H-pyrazol-1-ylmethyl)-1,3-dimethyl-1H-pyrazole-5-carboxamide.

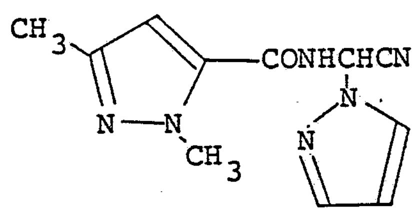

(10) No. 762:

N-(cyano-3-furanylmethyl)-1,3-dimethyl-1H-pyrazole-5-carboxamide.

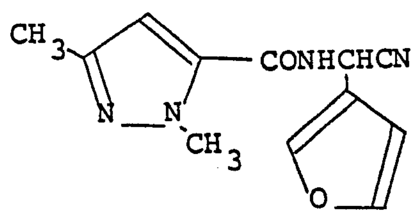

(11) No. 770:

N-(cyano-3-furanylmethyl)-1-ethyl-3-methyl-1H-pyrazole-5-carboxamide.

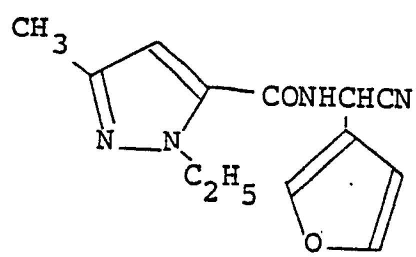

(12) No. 771:

N-(cyano-3-thienylmethyl)-1-ethyl-3-methyl-1H-pyraozle-5-carboxamide.

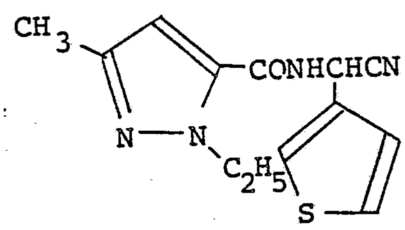

(13) No. 35:

N-(cyano-2-thienylmethyl)-1-methyl-1H-pyrazole-4-carboxamide.

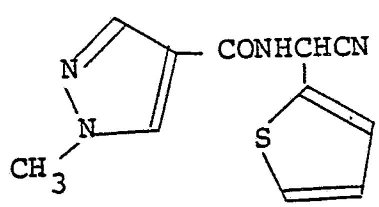

(14) No. 793:

3-Chloro-N-(cyano-3-thienylmethyl)-1-methyl-1H-pyrazole-5-carboxamide.

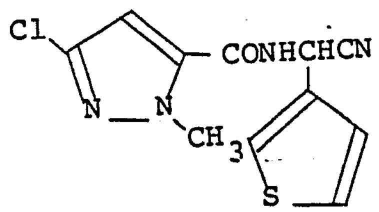

(15) No. 183:

N-(cyano-2-furanylmethyl)-3-methoxy-1-methyl-1H-pyrazole-5-carboxamide.

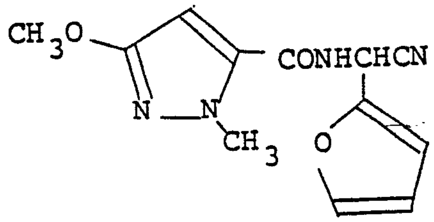

The method for preparation of the compounds of the present invention will be explained hereinafter, by reference to the reaction scheme.

Reaction Scheme:

Method 1:

(a)

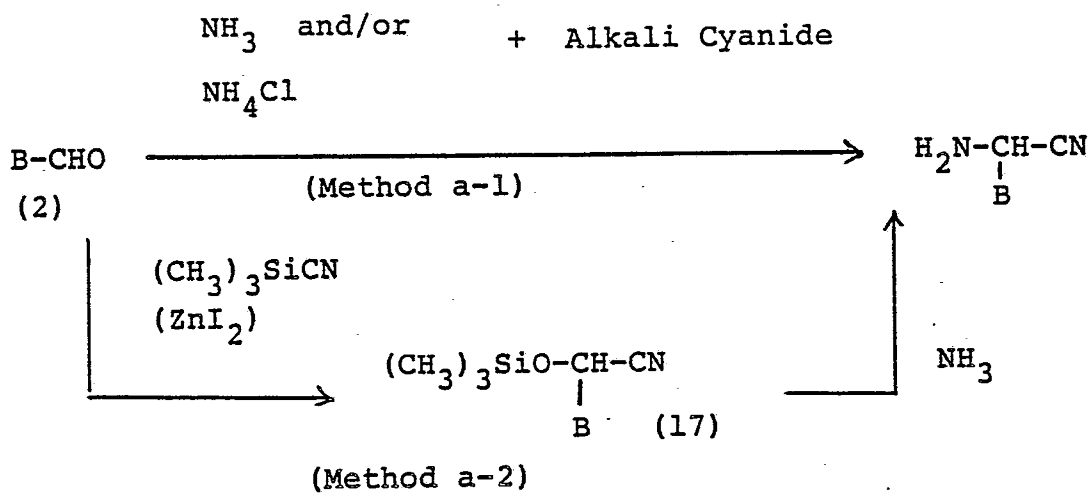


(2) (3) (17)

(b)

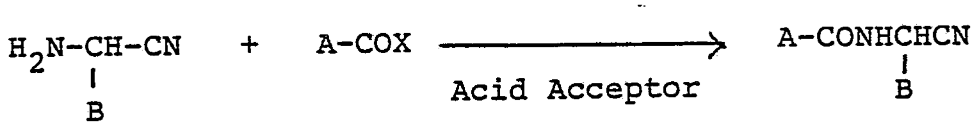


(3) (4) (5)

Method 2:

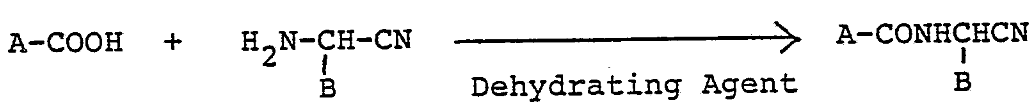


(6) (3) (5)

Method 3:

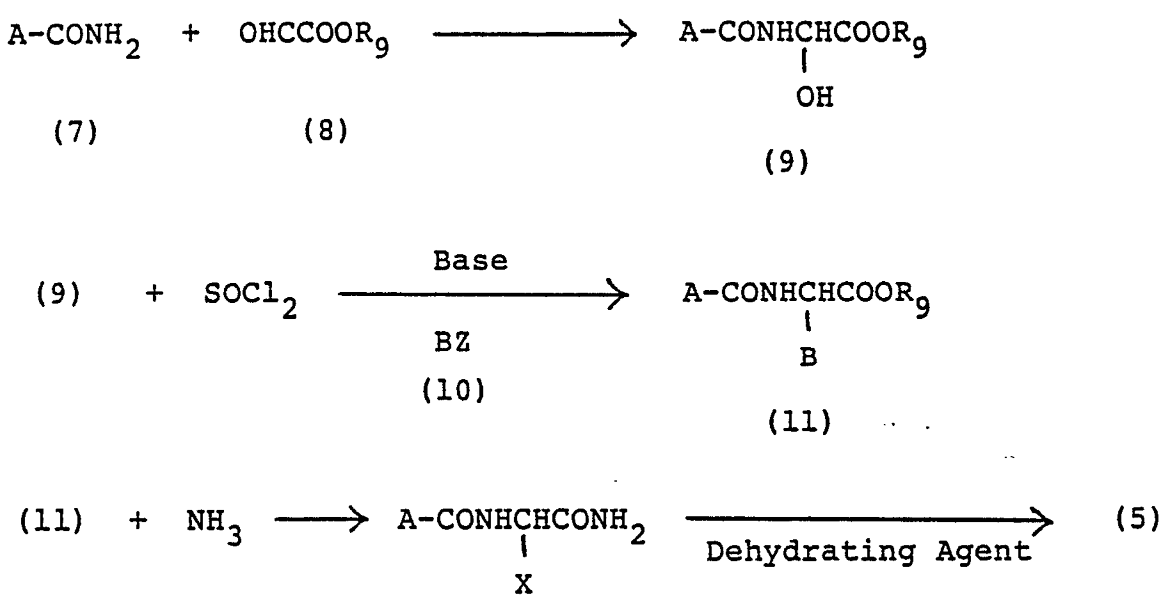


(7) (8) (9) (10) (11) (5)

Method 4:

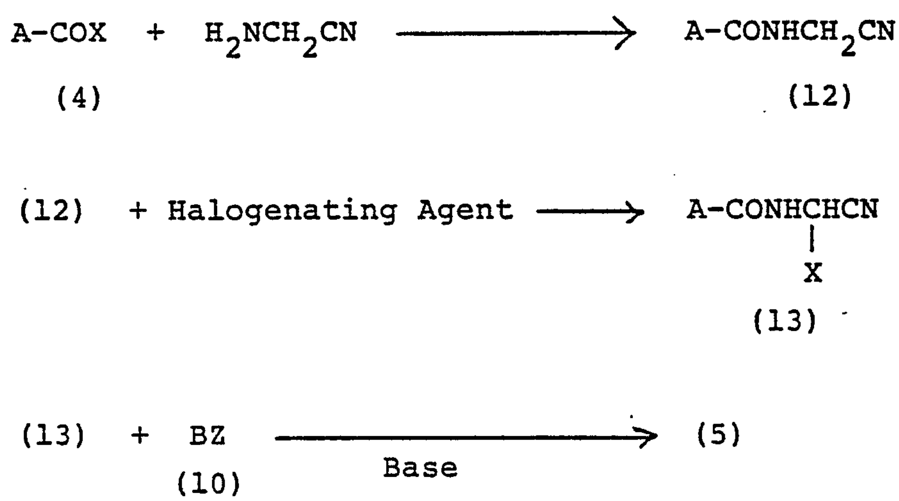


(4) (12) (13) (10) (5)

Method 5:

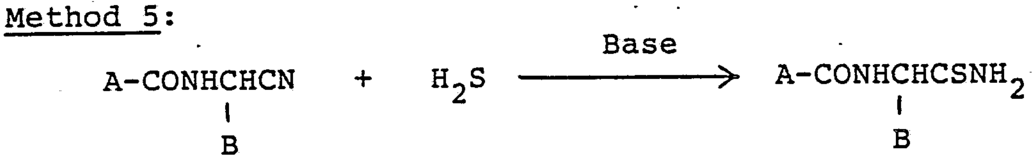


(5) (14)

Method 6:

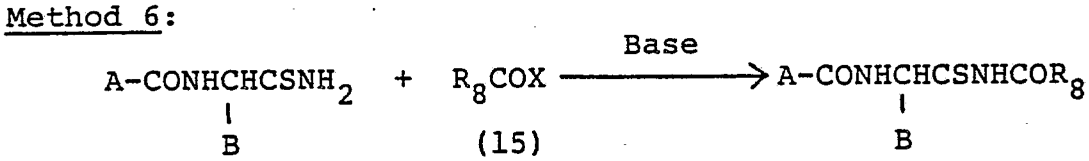


(15)

(14) (16)

In the said formulae, A, B and $R_8$ have the same meanings as mentioned above; $R_9$ represents an alkyl group having from 1 to 4 carbon atoms; Z represents a hydrogen atom or an alkali metal such as lithium, sodium, potassium, etc.; and X represents a halogen atom.

Method 1:

The aminoacetonitrile derivative (3), which is an intermediate in the first step, can be produced by the two ways.

The method (a-1) is one way to produce the said derivative (3) by Strecker reaction, where the aldehyde (2), as a starting material, and aqueous ammonia and/or ammonium chloride and an alkali cyanide, such as sodium cyanide, potassium cyanide, etc., are reacted in a binary solvent system comprising an ether, such as ethyl ether, tetrahydrofuran, etc., or an aromatic hydrocarbon, such as benzene, toluene, etc., and water. The reaction temperature is generally preferably from 0 to 100°C or so.

The method (a-2) is another way to produce the aminoacetonitrile derivative (3), where the aldehyde (2), as a starting material, is reacted with a trialkylsilylnitrile, such as trimethylsilylnitrile, etc., optionally in the presence of a catalytic amount of a Lewis acid, such as zinc iodide, etc., to obtain the intermediate (17), and successively, this (17) is reacted with an ammonia as dissovled in a solvent, such as methanol, ethanol, etc., to obtain the said derivative (3).

The thus-obtained aminoacetonitrile derivative (3) can be purified and isolated by forming a salt with a hydrogen halide, such as hydrogen chloride, etc., in an ether solvent, such as diethyl ether, etc.

Next, in the second step, the aminoacetonitrile derivative (3) as obtained in the first step is reacted with the acid halide having the general formula (4) in the presence of an acid acceptor.

As the acid acceptor, there may be mentioned, for example, organic bases, such as triethylamine, dimethylaniline, pyridine, etc., and inorganic bases, such as ammonia, potassium carbonate, sodium carbonate, ammonium hydrogencarbonate, sodium hydroxide, ammonium carbonate, etc.

The reaction is preferably carried out in the presence of a solvent, for which can be used, for example, ethers, such as diethyl ether, tetrahydrofuran, diisopropyl ether, etc., esters, such as methyl acetate, ethyl acetate, etc., halogenated hydrocarbons, such as methylene chloride, chloroform, 1,2-dichloroethane, etc., acetonitrile and the like. Regarding the reaction temperature, the reaction is desirably carried out under cooling, since this is exothermic, and the reaction temperature is preferably from about -30°C to about 50 °C practically desirable from -20 to 30°C or so. Thus, the intended substituted-amido acetonitrile derivatives can be obtained.

Method 2:

The starting material carboxylic acid derivative (6) is reacted with the aminoacetonitrile derivative (3) as obtained in the (Mehtod 1), in the presence of a dehydrating agent, for dehydrative condensation, to give the intended amido-substituted acetonitrile derivatives.

As the dehydrating agent, carbodiimides, such as dicyclohexylcarbodiimide (DCC), 1-ethyl-3-(3-dimethylaminopropyl)carbodiimide, etc., as well as inorganic dehydrating agents, such as silicon tetrachloride, etc., are especially preferred.

Method 3:

The reaction of the amide derivative (7) with the glyoxylic acid ester (8) is preferably carried out in an ester solvent, such as ethyl acetate, isobutyl acetate, etc. or in an acetone solvent, under heat at from 50 to 100°C or so.

Subsequently, the resulting hydroxyl compound (9) is reacted with an excess amount of thionyl chloride at a temperature from lower than room temperature to room temperature , to give the chlorinated compound. After the reaction, the excess thionyl chloride was evaporated off, and then the resulting compound is reacted with the compound of the formula BZ (10) in a solvent, optionally in the presence of a base, to obtain the ester compound (11).

As the solvent which can be used in the said reaction, there may be mentioned ester solvents, such as ethyl acetate, isobutyl acetate, etc., as well as ethers and acetonitrile, etc. As the base, organic bases, such as triethylamine, pyridine, etc., as well as inorganic bases, such as sodium carbonate, ammonium carbonate, etc., can be used.

Next, the resulting ester compound (11) is reacted with ammonia to give the amide compound. This reaction is preferably carried out in a solvent, for example, alcohols such as methanol, ethanol, etc., as well as ethers such as diethyl ether, tetrahydrofuran, etc., at about -50°C to room temperature or so. The thus-formed amide compound is thereafter further treated with a dehydrating agent, such as anhydrous trifluoroacetic acid, etc., in an organic amine, such as pyridine, or with a dehydrating agent, such as phosphorus oxychloride, etc., in an amide solvent, such as N,N-dimethylformamide (DMF), etc., or with a dehydrating agent, such as a polyphosphoric acid (PPA), a polyphosphoric acid alkyl ester (PPE), a polyphosphoric acid alkylsilyl ester (PPSE), etc., whereby the intended amide derivative (5) can be obtained.

Method 4:

The reaction of the aminoacetonitrile and the acid chloride (4) can easily be carried out under the conventional condensation reaction condition, to give the intended acylamino-acetonitrile derivative (12). In this reaction, the presence of a base is generally preferred; and inorganic bases, such as sodium carbonate, ammonium carbonate, etc., as well as orgnaic bases, such as triethylamine, pyridine, etc., are preferred as the base for use in the said reaction.

The acylaminoacetonitrile derivative (12) can be converted into the halide (13) by halogenation. As the halogenating agent which can be used for the said halogenation, chlorine, bromine, N-chlorosuccinimide, N-bromosuccinimide, etc. are preferred. The resulting halide intermediate is poor in the thermal stability, and therefore, this is, without being isolated and purified, introduced into the next step. Accordingly, the halide intermediate (13) is, immediately after formation by halogenation, reacted with BZ (10), optionally in the presence of a base, to give the intended amide derivative (5). The reaction condition in this step is same as that in the above-mentioned Method 3 .

Method 5:

The substituted-amido acetonitrile derivative (5) as obtained in anyone of the (Method 1) to (method 4) is reacted with hydrogen sulfide in the presence of a tertiary amine, such as triethylamine, pyridine, etc., to obtain the intended thioamide derivative (14).

Method 6 :

The thioamide derivative (14) as obtained in the (Mehtod 5) is reacted with the acid halide derivative (15), optionally in the presence of an effective amount of a tertiary amine, such as triethylamine, pyridine, etc., to obtain the intended acylthioamide derivative (16). As the acid halide derivative for use in this process, there may be mentioned lower alkyl-carboxylic acid halides, such as acetyl chloride, propionyl chloride, etc., as well as cyclic acid halides, such as cyclohexylcarbonyl chloride, benzoyl chloride, etc.

The thus-obtained amide derivative can be purified by means of a conventional purification method, for example, including recrystallization, column chromatography or the like, to obtain a pure product.

Concrete examples are described hereinafter to illustrate the method for preparation of the compounds of the present invention, which, however, are not intended to limit the scope of the present invention.

EXAMPLE 1

Production of Compound No. 35:

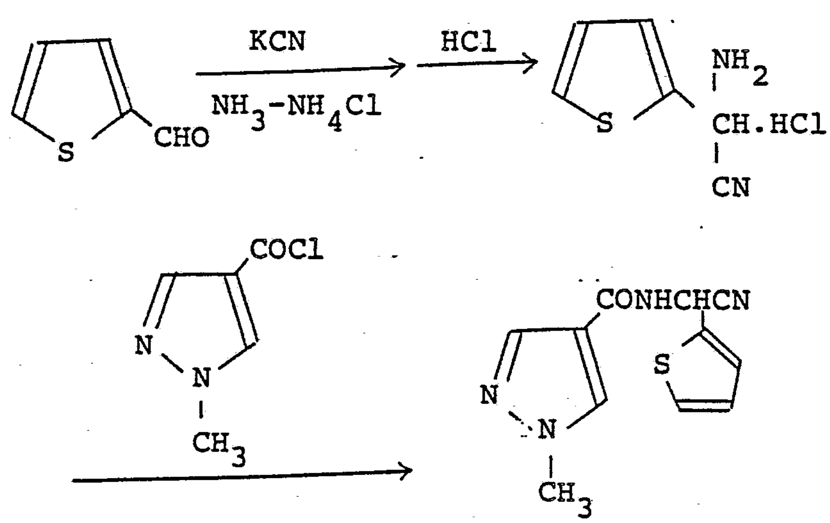


Aqueous ammonia (80 ml) was added to ammonium chloride (7.7 g) and potassium cyanide (4.9 g) under cooling witn ice, and, after stirred, a solution of 2-thiophenaldehyde (8.0 g) as dissolved in toluene (80 ml) was added thereto, and the whole was stirred for 20 hours at room temperature. After the completion of the reaction, the toluene layer was separated out, and the aqueous layer was extracted three times with toluene. These toluene layers were combined, dehydrated and dried, and then the solvent was evaporated out. The reaction product was dissolved in ether, and while cooled with ice, hydrogen chloride-saturated ether was dropwise added thereto. The crystal formed was taken out by filtration and dried, to obtain α-(2-thiophene)aminoacetonitrile hydrochloride salt (7.1 g).

To an acetonitrile solution containing the said α-(2-thiopehne)aminoacetonitrile hydrochloride (3.0g) and triethylamine (3.6 g) was added 1-methyl-1H-pyrazole-4-carboxylic acid chloride (2.2 g) under cooling with ice, and the whole was stirred for 1 hour under cooling with ice and then for 16 hours at room temperature. The solid product formed was taken out by filtration, and then the solvent was evaporated out. The thus-obtained oily product was purified by column chromatography and then washed with isopropyl ether, to obtain 2.1 g of the intended N-(cyano-2-thienylmethyl)-1-methyl-1H-pyrazole-4-carboxamide.

m.p. 161 to 163°C.

$^1$H-NMR : (Solvent $CDCl_3$.DMSDP-$d_6$) δ(ppm) = 9.34 (d,1H, J=8.4Hz), 8.15(s,1H), 7.82(s,1H), 6.8 to 7.6 (m,3H), 6.51(d,1H, J=8.4 Hz), 3.85(s,3H).

EXAMPLE 2

Production of Compound No. 26:

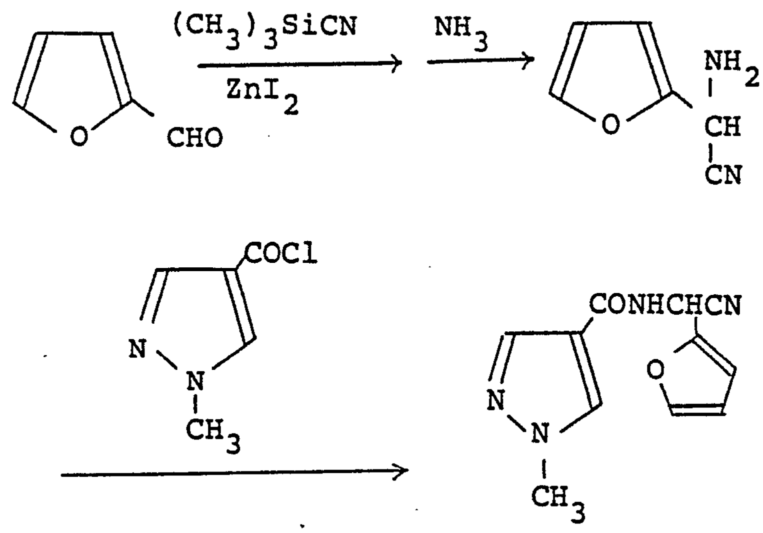


A catalytic amount (50 mg) of zinc iodide was added to furfural (10 g) and trimethylsilyl cyanide (11 g) under cooling with ice and then stirred for 1 hour at room temperature. Afterwards, ammonia-saturated methanol (80 ml) was added thereto and stirred for 2 hours at 40°C. The reaction mixture was concentrated and extracted with ethyl acetate, and then dried, and thereafter the solvent was evaporated out to obtain α-(2-furyl)aminoacetonitrile (11.6 g).

To a THF solution containing the said α-(2-furylaminoacetonitrile (2.15 g) and triethylamine (1.75 g) was added 1-methyl-1H-pyrazole-4-carboxylic acid chloride (2.55 g) under cooling with ice, and the whole was stirred for 1 hour as such and then for 16 hours at room temperature. The solid product formed was taken out by filtration, and the solvent was evaporated out, and then the thus-obtained oily product was purified by column chromatography and then washed with isopropyl ether, to give 2.55 g of the intended N-(cyano-2-furanylmethyl)-1-methyl-1H-pyrazole-4-carboxamide crystal.

m.p. 160 to 162°C.

$^1$H-NMR : (Solvent $CDCl_3$.DMSO-$d_6$) δ (ppm) = 9.16 (d,1H, J=7.2Hz), 8.07(s,1H), 7.84(s,1H), 7.50-(m,1H), 6.3 to 6.6(m,2H), 6.31(d,1H,J=7.2 Hz), 3,84(s,3H).

EXAMPLE 3

Production of Compound No. 30:

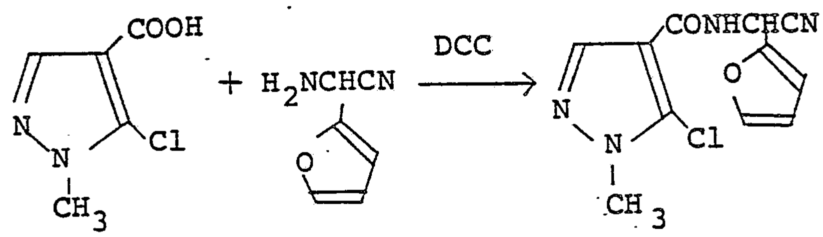


To a dichloromethane solution (20 ml) containing 5-chloro-1-methyl-1H-pyrazole-4-carboxylic acid (1.3 g) was added DDC (dicyclohexylcarbodiimide) (1.7 g) under cooling with ice, and the whole was stirred for 1 hour. To the resulting reaction solution was gradually and dropwise added a dichloromethane solution (6 ml) containing α-(2-furyl)aminoacetonitrile (1.2 g), which had been prepared in the same manner as Example 2. After the completion of the dropwise addition, the whole was stirred for 3 hours at room temperature. The floating solid formed was taken out by filtration, and the solvent was evaporated out. The thus obtained solid product was purified by column chromatography, to obtain 1.2 g of the intended N-(cyano-2-furanylmethyl)-5-chloro-1-methyl-1H-pyrazol-4-carboxamide.

m.p. 104 to 106°C.

$^1$H-NMR : (Solvent $CDCl_2$.DMSO-$d_6$) δ (ppm) = 7.90(s,1H), 7.42(m,1H), 7.23(d,1H,J=8.4Hz), 6.30-(d,1H,J=8.4Hz), 6.2 to 6.8(m,2H), 3.83(s,3H).

EXAMPLE 4

Production of Compound No. 16:

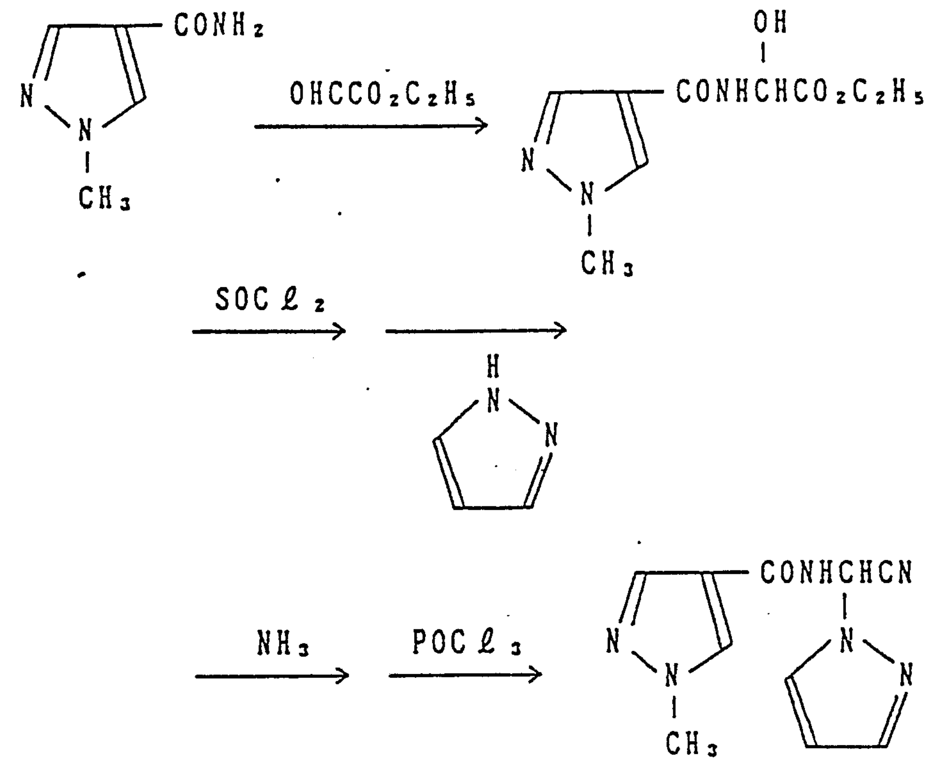


Ethyl glyoxalate (60 g) was added to a mixture comprising 1-methyl-1H-pyrazole-4-carboxamide (25 g) and ethyl acetate (200 ml) and then subjected to continuous reflux for 7 hours.

The reaction mixture thus obtained was separated and purified by column chromatography, to obtain 25.4 g of the intended white crystal ester. (m.p. 97 to 101 °C.)

The ester (5.6 g) and thionyl chloride (30 ml) were stirred for 2 hours at room temperature. The excess thionyl chloride was removed under reduced pressure, and THF (20 ml) was added to the residue and then pyrazole (1.8 g) was added thereto under cooling with ice. Further, triethylamine (4.0 g) was added under cooling with ice, and the resulting whole was stirred for 1 hour at room temperature. The salt as precipitated was separated by filtration and THF was evaporated out, and then the residual solid was dissolved in methanol (100 ml), into which ammonia gas was blown for 1 hour at -30°C to 0°c. The ammonia and the solvent were removed under reduced pressure, to obtain a crude amide (7.1 g). Anhydrous DMF (50 ml) was dropwise added to phosphorus oxychloride (7.5 g). The resulting mixture was dropwise added to a cooled anhydrous DMF solution containing the said crude amide. After the completion of the addition, the reaction mixture was poured into water and extracted with butanol. After the butanol was evaporated out, the product was separated and purified by column chromatography to obtain 2.7 g of the intended N-(cyano-1H-pyrazol-1-ylmethyl)-1-methyl-1H-pyrazol-4-carboxamide of white crystal.

m.p. 188 to 190°C (decomposition)

$^1$H-NMR : (Solvent $CDCl_3$.DMSO-$d_6$) $\delta$ (ppm) = 10.12 (d,1H, J=7.2Hz), 8.21(s,1H), 7.91(s,1H), 7.83-(m,1H), 7.52(m,1H), 7.48(d,1H,J=7.2 Hz), 6.2 to 6.4(m,1H), 3.84(s,3H).

EXAMPLE 5

Production of Compound No. 27:

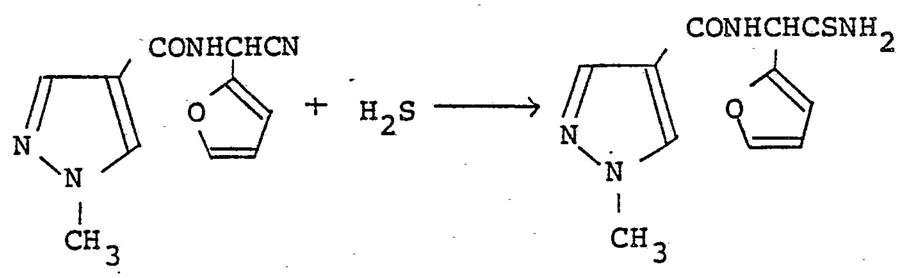

Hydrogen sulfide was blown into an ethanol solution containing 1.4 g of the N-(cyano-2-furanylmethyl)-1-methyl-1H-pyrazol-4-carboxamide as obtained in Example 2 and 0.7 g of triethylamine, for 1 hour, and the whole was stirred for 1 hour under cooling with ice and then for further 2 hours at room temperature. Afterwards, the crystal formed was taken out by filtration, washed with chloroform and then dried, to obtain 1.1 g of the intended N-[2-furanyl(thiocarbamoyl)methyl]-1-methyl-1H-pyrazole-4-carboxamide.

m.p. 203 to 205°C

$^1$H-NMR : (Solvent $CDCl_3$.DMSO-d6) δ (ppm) = 9.79(bs,1H), 9.38(bs,1H), 8.25(d,1H,J = 9Hz), 8.17(s,1H), 7.87(s,1H), 7.46(s,1H), 7.46(m,1H), 6.2 to 6.6(m,2H), 6.04(.d,1H,J = 9 Hz), 3.83(s,3H).

EXAMPLE 6

Production of Compound No. 765:

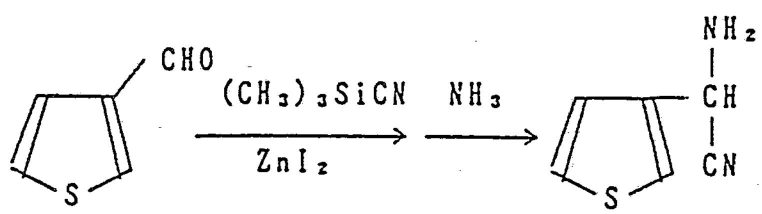


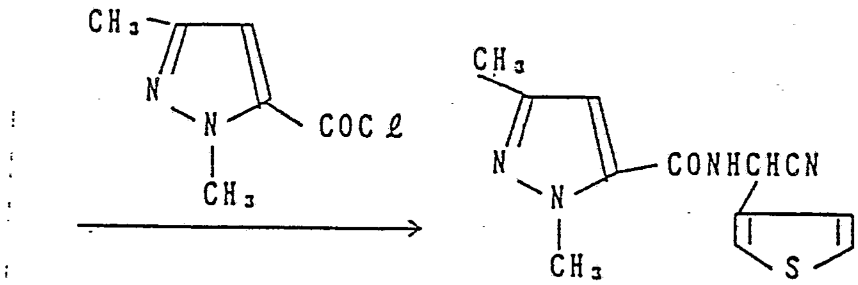

A catalytic amount (about 5 mg) of zinc iodide was added to 3-thiophenaldehyde (11.2 g) and trimethylsilyl cyanide (11 g) under cooling with ice and stirred for 1 hour at room temperature. Afterwards,

ammonia-saturated methanol (80 ml) was added thereto and stirred for 2 hours at 40°C. The reaction mixture was concentrated, extracted with ethyl acetate and dried, and the solvent was evaporated out, to obtain α-(3-thienyl)aminoacetonitrile (13.8 g).

1,3-dimethyl-1H-pyrazole-5-carboxylic acid chloride (2.45 g) was added to a solution containing the said α-(3-thienyl)aminoacetonitrile (2.76 g), pyridine (2 ml) and acetonitrile (30 ml), under cooling with ice, and stirred for 1 hour as such and then for 16 hours at room temperature. After the solid product formed was separated by filtration, the solvent was evaporated out, and the thus-obtained oily product was purified by column chromatography and then washed with isopropyl ether, to obtain 3.95 g of an oily product of the intended N-(cyano-3-thienylmethyl)-1,3-dimethyl-1H-pyrazole-5-carboxamide. This oily product was, after left at room temperature, became solid.

m.p. 107 to 109°C

$^1$H-NMR : (Solvent $CDCl_3$) δ (ppm) = 7.66 (d,1H,J=8.4 Hz), 6.96 to 7.51(m,3H), 6.41(s,1H), 6.24-(d,1H,J=8.4Hz), 4.01(s,3H), 2.16(s,3H).

The compounds of the present invention, which were produced in the same manner, are listed in Tables 1 to 4 below.

Table 1

```
A−CONHCH−D
        |
        B
```

| Compound No. | A | $R_1$ | $R_2$ | $R_3$ | B | $R_4$ | $R_5$ | $R_6$ | D |
|---|---|---|---|---|---|---|---|---|---|
| 1 | A−1 | H | H | H | B−1 | H | H | H | CN |
| 2 | A−1 | H | H | H | B−4 | H | H | H | CN |
| 3 | A−1 | H | H | H | B−5 | H | H | H | CN |
| 4 | A−1 | $CH_3$ | H | H | B−1 | H | H | H | CN |
| 5 | A−1 | $CH_3$ | H | H | B−2 | H | H | H | CN |
| 6 | A−1 | $CH_3$ | H | H | B−3 | H | H | H | CN |
| 7 | A−1 | $CH_3$ | H | H | B−4 | H | H | H | CN |
| 8 | A−1 | $CH_3$ | H | H | B−5 | H | H | H | CN |
| 9 | A−1 | $CH_3$ | H | $CH_3$ | B−1 | H | H | H | CN |
| 10 | A−1 | $CH_3$ | H | $CH_3$ | B−4 | H | H | H | CN |
| 11 | A−1 | $CH_3$ | H | $CH_3$ | B−5 | H | H | H | CN |
| 12 | A−1 | $CH_3$ | H | $CH_3O$ | B−4 | H | H | H | CN |
| 13 | A−2 | H | H | H | B−1 | H | H | H | CN |
| 14 | A−2 | H | H | H | B−4 | H | H | H | CN |
| 15 | A−2 | H | H | H | B−5 | H | H | H | CN |
| 16 | A−2 | $CH_3$ | H | H | B−1 | H | H | H | CN |
| 17 | A−2 | $CH_2F$ | H | H | B−1 | H | H | H | CN |
| 18 | A−2 | $CH_3$ | H | H | B−1 | $CH_3$ | H | H | CN |
| 19 | A−2 | $CH_3$ | H | H | B−1 | H | $CH_3$ | H | CN |
| 20 | A−2 | $CH_3$ | H | H | B−1 | $CH_3$ | H | $CH_3$ | CN |
| 21 | A−2 | $CH_3$ | H | H | B−1 | $CH_3$ | Br | H | CN |
| 22 | A−2 | $CH_2CH_3$ | H | H | B−1 | H | H | H | CN |
| 23 | A−2 | $CH_2CH{=}CH_2$ | H | H | B−1 | H | H | H | CN |
| 24 | A−2 | $CH_3$ | H | H | B−2 | H | H | H | CN |
| 25 | A−2 | $CH_3$ | H | H | B−3 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 26 | A−2 | $CH_3$ | H | H | B−4 | H | H | H | CN |
| 27 | A−2 | $CH_3$ | H | H | B−4 | H | H | H | $CSNH_2$ |
| 28 | A−2 | $CH_3$ | H | H | B−5 | H | H | H | CN |
| 29 | A−2 | $CH_3$ | H | $C\ell$ | B−1 | H | H | H | CN |
| 30 | A−2 | $CH_3$ | H | $C\ell$ | B−4 | H | H | H | CN |
| 31 | A−2 | $CH_3$ | H | H | B−4 | H | H | $CH_3$ | CN |
| 32 | A−2 | $CH_2F$ | H | H | B−4 | H | H | H | CN |
| 33 | A−2 | $CH_2CH_3$ | H | H | B−4 | H | H | H | CN |
| 34 | A−2 | $CH_2CH{=}CH_2$ | H | H | B−4 | H | H | H | CN |
| 35 | A−2 | $CH_3$ | H | H | B−5 | H | H | H | CN |
| 36 | A−2 | $CH_3$ | H | H | B−5 | H | H | H | $CSNH_2$ |
| 37 | A−2 | $CH_3$ | H | H | B−5 | H | H | H | $CSNHCOCH_3$ |
| 38 | A−2 | $CH_3$ | H | H | B−5 | $CH_3$ | H | H | CN |
| 39 | A−2 | $CH_3$ | H | H | B−5 | H | H | $CH_3$ | CN |
| 40 | A−2 | $CH_3$ | $C\ell$ | $C\ell$ | B−4 | H | H | H | CN |
| 41 | A−3 | H | H | H | B−1 | H | H | H | CN |
| 42 | A−3 | H | H | H | B−2 | H | H | H | CN |
| 43 | A−3 | H | H | H | B−3 | H | H | H | CN |
| 44 | A−3 | H | H | H | B−4 | H | H | H | CN |
| 45 | A−3 | H | H | H | B−5 | H | H | H | CN |
| 46 | A−3 | $CH_3$ | H | H | B−1 | H | H | H | CN |
| 47 | A−3 | $CH_3$ | H | H | B−1 | H | H | H | $CSNH_2$ |
| 48 | A−3 | $CH_3$ | H | H | B−1 | H | H | H | $CSNHCOCH_3$ |
| 49 | A−3 | $CH_3$ | H | H | B−1 | $CH_3$ | H | H | CN |
| 50 | A−3 | $CH_3$ | H | H | B−1 | H | H | $CH_3$ | CN |
| 51 | A−3 | $CH_3$ | H | H | B−2 | H | H | H | CN |
| 52 | A−3 | $CH_3$ | H | H | B−3 | H | H | H | CN |
| 53 | A−3 | $CH_3$ | H | H | B−4 | H | H | H | CN |
| 54 | A−3 | $CH_3$ | H | H | B−4 | H | H | H | $CSNH_2$ |
| 55 | A−3 | $CH_3$ | H | H | B−5 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 56 | A−3 | $CH_3$ | H | H | B−5 | H | H | H | $CSNH_2$ |
| 57 | A−3 | $CH_2CH_3$ | H | H | B−4 | H | H | H | CN |
| 58 | A−3 | $CH_2CH_3$ | H | H | B−5 | H | H | H | CN |
| 59 | A−3 | $CH_3$ | $CH_3$ | H | B−1 | H | H | H | CN |
| 60 | A−3 | $CH_3$ | $CH_3$ | H | B−1 | H | H | H | $CSNH_2$ |
| 61 | A−3 | $CH_3$ | $CH_3$ | H | B−1 | H | H | H | $CSNHCOCH_3$ |
| 62 | A−3 | $CH_3$ | $CH_3$ | H | B−1 | H | H | H | $CSNHCOC_6H_{11}$-Cyclo |
| 63 | A−3 | $CH_3$ | $CH_3$ | H | B−1 | H | H | H | $CSNHCOC_6H_5$ |
| 64 | A−3 | $CH_3$ | $CH_3$ | H | B−1 | $CH_3$ | H | H | CN |
| 65 | A−3 | $CH_3$ | $CH_3$ | H | B−1 | H | H | $CH_3$ | CN |
| 66 | A−3 | $CH_3$ | $CH_3$ | H | B−1 | H | H | F | CN |
| 67 | A−3 | $CH_3$ | $CH_3$ | H | B−1 | H | Br | H | CN |
| 68 | A−3 | $CH_2F$ | $CH_3$ | H | B−1 | H | H | H | CN |
| 69 | A−3 | $CHF_2$ | $CH_3$ | H | B−1 | H | H | H | CN |
| 70 | A−3 | $CF_3$ | $CH_3$ | H | B−1 | H | H | H | CN |
| 71 | A−3 | $CH_2CH_3$ | $CH_3$ | H | B−1 | H | H | H | CN |
| 72 | A−3 | $CH=CH_2$ | $CH_3$ | H | B−1 | H | H | H | CN |
| 73 | A−3 | $CF=CF_2$ | $CH_3$ | H | B−1 | H | H | H | CN |
| 74 | A−3 | $CH_2CF_3$ | $CH_3$ | H | B−1 | H | H | H | CN |
| 75 | A−3 | $CH_2CH=CH_2$ | $CH_3$ | H | B−1 | H | H | H | CN |
| 76 | A−3 | $CH_2C\equiv CH$ | $CH_3$ | H | B−1 | H | H | H | CN |
| 77 | A−3 | $CH=C=CH_2$ | $CH_3$ | H | B−1 | H | H | H | CN |
| 78 | A−3 | $CH_3$ | $CH_3$ | H | B−2 | H | H | H | CN |
| 79 | A−3 | $CH_3$ | $CH_3$ | H | B−3 | H | H | H | CN |
| 80 | A−3 | $CH_3$ | $CH_3$ | H | B−4 | H | H | H | CN |
| 81 | A−3 | $CH_3$ | $CH_3$ | H | B−4 | H | H | H | $CSNH_2$ |
| 82 | A−3 | $CH_2F$ | $CH_3$ | H | B−4 | H | H | H | CN |
| 83 | A−3 | $CHF_2$ | $CH_3$ | H | B−4 | H | H | H | CN |
| 84 | A−3 | $CF_3$ | $CH_3$ | H | B−4 | H | H | H | CN |
| 85 | A−3 | $CH_2CH_3$ | $CH_3$ | H | B−4 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 86 | A−3 | $CH=CH_2$ | $CH_3$ | H | B−4 | H | H | H | CN |
| 87 | A−3 | $CH_2CH=CH_2$ | $CH_3$ | H | B−4 | H | H | H | CN |
| 88 | A−3 | $CH_2C\equiv CH$ | $CH_3$ | H | B−4 | H | H | H | CN |
| 89 | A−3 | $CH_3$ | $CH_3$ | H | B−4 | H | H | $CH_3$ | CN |
| 90 | A−3 | $CH_3$ | $CH_3$ | H | B−4 | H | H | Br | CN |
| 91 | A−3 | $CH_3$ | $CH_3$ | H | B−5 | H | H | H | CN |
| 92 | A−3 | $CH_3$ | $CH_3$ | H | B−5 | H | H | H | $CSNH_2$ |
| 93 | A−3 | $CH_3$ | $CH_3$ | H | B−5 | H | H | H | $CSNHCOCH_3$ |
| 94 | A−3 | $CH_3$ | $CH_3$ | H | B−5 | $CH_3$ | H | H | CN |
| 95 | A−3 | $CH_3$ | $CH_3$ | H | B−5 | H | H | $CH_3$ | CN |
| 96 | A−3 | $CH_2F$ | $CH_3$ | H | B−5 | H | H | H | CN |
| 97 | A−3 | $CHF_2$ | $CH_3$ | H | B−5 | H | H | H | CN |
| 98 | A−3 | $CF_3$ | $CH_3$ | H | B−5 | H | H | H | CN |
| 99 | A−3 | $CH_2CH_3$ | $CH_3$ | H | B−5 | H | H | H | CN |
| 100 | A−3 | $CH=CH_2$ | $CH_3$ | H | B−5 | H | H | H | CN |
| 101 | A−3 | $CH_2CH=CH_2$ | $CH_3$ | H | B−5 | H | H | H | CN |
| 102 | A−3 | $CH_2C\equiv CH$ | $CH_3$ | H | B−5 | H | H | H | CN |
| 103 | A−3 | $CH_3$ | H | $CH_3$ | B−1 | H | H | H | CN |
| 104 | A−3 | $CH_3$ | H | $CH_3$ | B−1 | H | H | H | $CSNH_2$ |
| 105 | A−3 | $CH_3$ | H | $CH_3$ | B−2 | H | H | H | CN |
| 106 | A−3 | $CH_3$ | H | $CH_3$ | B−3 | H | H | H | CN |
| 107 | A−3 | $CH_3$ | H | $CH_3$ | B−4 | H | H | H | CN |
| 108 | A−3 | $CH_3$ | H | $CH_3$ | B−4 | H | H | H | $CSNH_2$ |
| 109 | A−3 | $CH_3$ | H | $CH_3$ | B−5 | H | H | H | CN |
| 110 | A−3 | $CH_3$ | H | $CH_3$ | B−5 | H | H | H | $CSNH_2$ |
| 111 | A−3 | $CH_3$ | $CH_3$ | $CH_3$ | B−1 | H | H | H | CN |
| 112 | A−3 | $CH_3$ | $CH_3$ | $CH_3$ | B−2 | H | H | H | CN |
| 113 | A−3 | $CH_3$ | $CH_3$ | $CH_3$ | B−3 | H | H | H | CN |
| 114 | A−3 | $CH_3$ | $CH_3$ | $CH_3$ | B−4 | H | H | H | CN |
| 115 | A−3 | $CH_3$ | $CH_3$ | $CH_3$ | B−5 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 116 | A−3 | $CH_3$ | $CF_3$ | H | B−1 | H | H | H | CN |
| 117 | A−3 | $CH_3$ | $CF_3$ | H | B−1 | H | H | H | $CSNH_2$ |
| 118 | A−3 | $CH_3$ | $CF_3$ | H | B−2 | H | H | H | CN |
| 119 | A−3 | $CH_3$ | $CF_3$ | H | B−3 | H | H | H | CN |
| 120 | A−3 | $CH_3$ | $CF_3$ | H | B−4 | H | H | H | CN |
| 121 | A−3 | $CH_3$ | $CF_3$ | H | B−4 | H | H | H | $CSNH_2$ |
| 122 | A−3 | $CH_3$ | $CF_3$ | H | B−5 | H | H | H | CN |
| 123 | A−3 | $CH_3$ | $CF_3$ | H | B−5 | H | H | H | $CSNH_2$ |
| 124 | A−3 | $CH_3$ | Cℓ | H | B−1 | H | H | H | CN |
| 125 | A−3 | $CH_3$ | Cℓ | H | B−1 | H | H | H | $CSNH_2$ |
| 126 | A−3 | $CH_3$ | Cℓ | H | B−2 | H | H | H | CN |
| 127 | A−3 | $CH_3$ | Cℓ | H | B−2 | H | H | H | $CSNH_2$ |
| 128 | A−3 | $CH_3$ | Cℓ | H | B−3 | H | H | H | CN |
| 129 | A−3 | $CH_3$ | Cℓ | H | B−4 | H | H | H | CN |
| 130 | A−3 | $CH_3$ | Cℓ | H | B−5 | H | H | H | CN |
| 131 | A−3 | $CH_3$ | Br | H | B−1 | H | H | H | CN |
| 132 | A−3 | $CH_3$ | Br | H | B−2 | H | H | H | CN |
| 133 | A−3 | $CH_3$ | Br | H | B−3 | H | H | H | CN |
| 134 | A−3 | $CH_3$ | Br | H | B−4 | H | H | H | CN |
| 135 | A−3 | $CH_3$ | Br | H | B−5 | H | H | H | CN |
| 136 | A−3 | $CH_3$ | $CH_3$ | F | B−1 | H | H | H | CN |
| 137 | A−3 | $CH_3$ | $CH_3$ | F | B−2 | H | H | H | CN |
| 138 | A−3 | $CH_3$ | $CH_3$ | F | B−3 | H | H | H | CN |
| 139 | A−3 | $CH_3$ | $CH_3$ | F | B−4 | H | H | H | CN |
| 140 | A−3 | $CH_3$ | $CH_3$ | F | B−5 | H | H | H | CN |
| 141 | A−3 | $CH_3$ | $CH_3$ | Cℓ | B−1 | H | H | H | CN |
| 142 | A−3 | $CH_3$ | $CH_3$ | Cℓ | B−2 | H | H | H | CN |
| 143 | A−3 | $CH_3$ | $CH_3$ | Cℓ | B−3 | H | H | H | CN |
| 144 | A−3 | $CH_3$ | $CH_3$ | Cℓ | B−4 | H | H | H | CN |
| 145 | A−3 | $CH_3$ | $CH_3$ | Cℓ | B−5 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 146 | A−3 | $CH_3$ | $CH_3$ | Br | B−1 | H | H | H | CN |
| 147 | A−3 | $CH_3$ | $CH_3$ | Br | B−2 | H | H | H | CN |
| 148 | A−3 | $CH_3$ | $CH_3$ | Br | B−3 | H | H | H | CN |
| 149 | A−3 | $CH_3$ | $CH_3$ | Br | B−4 | H | H | H | CN |
| 150 | A−3 | $CH_3$ | $CH_3$ | Br | B−5 | H | H | H | CN |
| 151 | A−3 | $CH_3$ | CN | H | B−1 | H | H | H | CN |
| 152 | A−3 | $CH_3$ | CN | H | B−2 | H | H | H | CN |
| 153 | A−3 | $CH_3$ | CN | H | B−3 | H | H | H | CN |
| 154 | A−3 | $CH_3$ | CN | H | B−4 | H | H | H | CN |
| 155 | A−3 | $CH_3$ | CN | H | B−5 | H | H | H | CN |
| 156 | A−3 | $CH_3$ | H | CN | B−1 | H | H | H | CN |
| 157 | A−3 | $CH_3$ | H | CN | B−2 | H | H | H | CN |
| 158 | A−3 | $CH_3$ | H | CN | B−3 | H | H | H | CN |
| 159 | A−3 | $CH_3$ | H | CN | B−4 | H | H | H | CN |
| 160 | A−3 | $CH_3$ | H | CN | B−5 | H | H | H | CN |
| 161 | A−3 | $CH_3$ | $CH_3$ | CN | B−1 | H | H | H | CN |
| 162 | A−3 | $CH_3$ | $CH_3$ | CN | B−2 | H | H | H | CN |
| 163 | A−3 | $CH_3$ | $CH_3$ | CN | B−3 | H | H | H | CN |
| 164 | A−3 | $CH_3$ | $CH_3$ | CN | B−4 | H | H | H | CN |
| 165 | A−3 | $CH_3$ | $CH_3$ | CN | B−5 | H | H | H | CN |
| 166 | A−3 | $CH_3$ | $CH_2CH_3$ | H | B−1 | H | H | H | CN |
| 167 | A−3 | $CH_3$ | $CH_2CH_3$ | H | B−2 | H | H | H | CN |
| 168 | A−3 | $CH_3$ | $CH_2CH_3$ | H | B−3 | H | H | H | CN |
| 169 | A−3 | $CH_3$ | $CH_2CH_3$ | H | B−4 | H | H | H | CN |
| 170 | A−3 | $CH_3$ | $CH_2CH_3$ | H | B−5 | H | H | H | CN |
| 171 | A−3 | $CH_3$ | H | Cℓ | B−1 | H | H | H | CN |
| 172 | A−3 | $CH_3$ | H | Cℓ | B−2 | H | H | H | CN |
| 173 | A−3 | $CH_3$ | H | Cℓ | B−4 | H | H | H | CN |
| 174 | A−3 | $CH_3$ | H | Cℓ | B−5 | H | H | H | CN |
| 175 | A−3 | $CH_3$ | H | Br | B−1 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 176 | A−3 | $CH_3$ | H | Br | B−2 | H | H | H | CN |
| 177 | A−3 | $CH_3$ | H | Br | B−3 | H | H | H | CN |
| 178 | A−3 | $CH_3$ | H | Br | B−4 | H | H | H | CN |
| 179 | A−3 | $CH_3$ | H | Br | B−5 | H | H | H | CN |
| 180 | A−3 | $CH_3$ | $CH_3O$ | H | B−1 | H | H | H | CN |
| 181 | A−3 | $CH_3$ | $CH_3O$ | H | B−2 | H | H | H | CN |
| 182 | A−3 | $CH_3$ | $CH_3O$ | H | B−3 | H | H | H | CN |
| 183 | A−3 | $CH_3$ | $CH_3O$ | H | B−4 | H | H | H | CN |
| 184 | A−3 | $CH_3$ | $CH_3O$ | H | B−5 | H | H | H | CN |
| 185 | A−3 | $CH_3$ | H | $CH_3O$ | B−1 | H | H | H | CN |
| 186 | A−3 | $CH_3$ | H | $CH_3O$ | B−2 | H | H | H | CN |
| 187 | A−3 | $CH_3$ | H | $CH_3O$ | B−3 | H | H | H | CN |
| 188 | A−3 | $CH_3$ | H | $CH_3O$ | B−4 | H | H | H | CN |
| 189 | A−3 | $CH_3$ | H | $CH_3O$ | B−5 | H | H | H | CN |
| 190 | A−3 | $CH_3$ | $CH_3S$ | H | B−1 | H | H | H | CN |
| 191 | A−3 | $CH_3$ | $CH_3S$ | H | B−2 | H | H | H | CN |
| 192 | A−3 | $CH_3$ | $CH_3S$ | H | B−3 | H | H | H | CN |
| 193 | A−3 | $CH_3$ | $CH_3S$ | H | B−4 | H | H | H | CN |
| 194 | A−3 | $CH_3$ | $CH_3S$ | H | B−5 | H | H | H | CN |
| 195 | A−3 | $CH_3$ | P-Cl-$C_6H_4$- | H | B−4 | H | H | H | CN |
| 196 | A−4 | H | H | H | B−1 | H | H | H | CN |
| 197 | A−4 | H | H | H | B−2 | H | H | H | CN |
| 198 | A−4 | H | H | H | B−3 | H | H | H | CN |
| 199 | A−4 | H | H | H | B−4 | H | H | H | CN |
| 200 | A−4 | H | H | H | B−5 | H | H | H | CN |
| 201 | A−4 | $CH_3$ | H | H | B−1 | H | H | H | CN |
| 202 | A−4 | $CH_3$ | H | H | B−2 | H | H | H | CN |
| 203 | A−4 | $CH_3$ | H | H | B−3 | H | H | H | CN |
| 204 | A−4 | $CH_3$ | H | H | B−4 | H | H | H | CN |
| 205 | A−4 | $CH_3$ | H | H | B−5 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 206 | A−4 | $CH_3$ | $CH_3$ | H | B−1 | H | H | H | CN |
| 207 | A−4 | $CH_3$ | $CH_3$ | H | B−2 | H | H | H | CN |
| 208 | A−4 | $CH_3$ | $CH_3$ | H | B−3 | H | H | H | CN |
| 209 | A−4 | $CH_3$ | $CH_3$ | H | B−4 | H | H | H | CN |
| 210 | A−4 | $CH_3$ | $CH_3$ | H | B−5 | H | H | H | CN |
| 211 | A−4 | $CH_3$ | Cℓ | H | B−5 | H | H | H | CN |
| 212 | A−4 | $CH_3$ | H | $CH_3$ | B−1 | H | H | H | CN |
| 213 | A−4 | $CH_3$ | H | $CH_3$ | B−2 | H | H | H | CN |
| 214 | A−4 | $CH_3$ | H | $CH_3$ | B−3 | H | H | H | CN |
| 215 | A−4 | $CH_3$ | H | $CH_3$ | B−4 | H | H | H | CN |
| 216 | A−4 | $CH_3$ | H | $CH_3$ | B−5 | H | H | H | CN |
| 217 | A−4 | $CH_3$ | H | Cℓ | B−5 | H | H | H | CN |
| 218 | A−5 | H | H | H | B−1 | H | H | H | CN |
| 219 | A−5 | H | H | H | B−2 | H | H | H | CN |
| 220 | A−5 | H | H | H | B−3 | H | H | H | CN |
| 221 | A−5 | H | H | H | B−4 | H | H | H | CN |
| 222 | A−5 | H | H | H | B−5 | H | H | H | CN |
| 223 | A−5 | $CH_3$ | H | H | B−1 | H | H | H | CN |
| 224 | A−5 | $CH_3$ | H | H | B−2 | H | H | H | CN |
| 225 | A−5 | $CH_3$ | H | H | B−3 | H | H | H | CN |
| 226 | A−5 | $CH_3$ | H | H | B−4 | H | H | H | CN |
| 227 | A−5 | $CH_3$ | H | H | B−5 | H | H | H | CN |
| 228 | A−5 | $CH_3$ | $CH_3$ | H | B−1 | H | H | H | CN |
| 229 | A−5 | $CH_3$ | $CH_3$ | H | B−2 | H | H | H | CN |
| 230 | A−5 | $CH_3$ | $CH_3$ | H | B−3 | H | H | H | CN |
| 231 | A−5 | $CH_3$ | $CH_3$ | H | B−4 | H | H | H | CN |
| 232 | A−5 | $CH_3$ | $CH_3$ | H | B−5 | H | H | H | CN |
| 233 | A−5 | $CH_3$ | Cℓ | H | B−5 | H | H | H | CN |
| 234 | A−5 | $CH_3$ | H | $CH_3$ | B−1 | H | H | H | CN |
| 235 | A−5 | $CH_3$ | H | $CH_3$ | B−2 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 236 | A−5 | $CH_3$ | H | $CH_3$ | B−3 | H | H | H | CN |
| 237 | A−5 | $CH_3$ | H | $CH_3$ | B−4 | H | H | H | CN |
| 238 | A−5 | $CH_3$ | H | $CH_3$ | B−5 | H | H | H | CN |
| 239 | A−5 | $CH_3$ | H | Cℓ | B−5 | H | H | H | CN |
| 240 | A−6 | H | H | H | B−1 | H | H | H | CN |
| 241 | A−6 | H | H | H | B−2 | H | H | H | CN |
| 242 | A−6 | H | H | H | B−3 | H | H | H | CN |
| 243 | A−6 | H | H | H | B−4 | H | H | H | CN |
| 244 | A−6 | H | H | H | B−5 | H | H | H | CN |
| 245 | A−6 | H | H | $CH_3$ | B−4 | H | H | H | CN |
| 246 | A−6 | $CH_3$ | H | H | B−1 | H | H | H | CN |
| 247 | A−6 | $CH_3$ | H | H | B−2 | H | H | H | CN |
| 248 | A−6 | $CH_3$ | H | H | B−3 | H | H | H | CN |
| 249 | A−6 | $CH_3$ | H | H | B−4 | H | H | H | CN |
| 250 | A−6 | $CH_3$ | H | H | B−5 | H | H | H | CN |
| 251 | A−6 | $CH_3$ | $CH_3$ | H | B−1 | H | H | H | CN |
| 252 | A−6 | $CH_3$ | $CH_3$ | H | B−2 | H | H | H | CN |
| 253 | A−6 | $CH_3$ | $CH_3$ | H | B−3 | H | H | H | CN |
| 254 | A−6 | $CH_3$ | $CH_3$ | H | B−4 | H | H | H | CN |
| 255 | A−6 | $CH_3$ | $CH_3$ | H | B−5 | H | H | H | CN |
| 256 | A−6 | $CH_3$ | Cℓ | H | B−5 | H | H | H | CN |
| 257 | A−6 | $CH_3$ | H | $CH_3$ | B−1 | H | H | H | CN |
| 258 | A−6 | $CH_3$ | H | $CH_3$ | B−2 | H | H | H | CN |
| 259 | A−6 | $CH_3$ | H | $CH_3$ | B−3 | H | H | H | CN |
| 260 | A−6 | $CH_3$ | H | $CH_3$ | B−4 | H | H | H | CN |
| 261 | A−6 | $CH_3$ | H | $CH_3$ | B−5 | H | H | H | CN |
| 262 | A−6 | $CH_3$ | H | Cℓ | B−5 | H | H | H | CN |

| Compound No. | A | $R_2$ | $R_3$ | B | $R_4$ | $R_5$ | $R_6$ | D |
|---|---|---|---|---|---|---|---|---|
| 263 | A−7 | H | H | B−1 | H | H | H | CN |
| 264 | A−7 | H | H | B−2 | H | H | H | CN |
| 265 | A−7 | H | H | B−3 | H | H | H | CN |
| 266 | A−7 | H | H | B−4 | H | H | H | CN |
| 267 | A−7 | H | H | B−5 | H | H | H | CN |
| 268 | A−7 | $CH_3$ | H | B−1 | H | H | H | CN |
| 269 | A−7 | $CH_3$ | H | B−2 | H | H | H | CN |
| 270 | A−7 | $CH_3$ | H | B−3 | H | H | H | CN |
| 271 | A−7 | $CH_3$ | H | B−4 | H | H | H | CN |
| 272 | A−7 | $CH_3$ | H | B−5 | H | H | H | CN |
| 273 | A−7 | Cℓ | H | B−5 | H | H | H | CN |
| 274 | A−7 | H | $CH_3$ | B−1 | H | H | H | CN |
| 275 | A−7 | H | $CH_3$ | B−2 | H | H | H | CN |
| 276 | A−7 | H | $CH_3$ | B−3 | H | H | H | CN |
| 277 | A−7 | H | $CH_3$ | B−4 | H | H | H | CN |
| 278 | A−7 | H | $CH_3$ | B−5 | H | H | H | CN |
| 279 | A−7 | H | Cℓ | B−5 | H | H | H | CN |
| 280 | A−8 | H | H | B−1 | H | H | H | CN |
| 281 | A−8 | H | H | B−2 | H | H | H | CN |
| 282 | A−8 | H | H | B−3 | H | H | H | CN |
| 283 | A−8 | H | H | B−4 | H | H | H | CN |
| 284 | A−8 | H | H | B−5 | H | H | H | CN |
| 285 | A−8 | $CH_3$ | H | B−1 | H | H | H | CN |
| 286 | A−8 | $CH_3$ | H | B−2 | H | H | H | CN |
| 287 | A−8 | $CH_3$ | H | B−3 | H | H | H | CN |
| 288 | A−8 | $CH_3$ | H | B−4 | H | H | H | CN |
| 289 | A−8 | $CH_3$ | H | B−5 | H | H | H | CN |
| 290 | A−8 | Cℓ | H | B−5 | H | H | H | CN |
| 291 | A−8 | H | $CH_3$ | B−1 | H | H | H | CN |
| 292 | A−8 | H | $CH_3$ | B−2 | H | H | H | CN |

| Compound No. | A | $R_2$ | $R_3$ | B | $R_4$ | $R_5$ | $R_6$ | D |
|---|---|---|---|---|---|---|---|---|
| 293 | A−8 | H | $CH_3$ | B−3 | H | H | H | CN |
| 294 | A−8 | H | $CH_3$ | B−4 | H | H | H | CN |
| 295 | A−8 | H | $CH_3$ | B−5 | H | H | H | CN |
| 296 | A−8 | H | Cℓ | B−5 | H | H | H | CN |
| 297 | A−9 | H | H | B−1 | H | H | H | CN |
| 298 | A−9 | H | H | B−2 | H | H | H | CN |
| 299 | A−9 | H | H | B−3 | H | H | H | CN |
| 300 | A−9 | H | H | B−4 | H | H | H | CN |
| 311 | A−9 | H | H | B−5 | H | H | H | CN |
| 312 | A−9 | $CH_3$ | H | B−1 | H | H | H | CN |
| 313 | A−9 | $CH_3$ | H | B−2 | H | H | H | CN |
| 314 | A−9 | $CH_3$ | H | B−3 | H | H | H | CN |
| 315 | A−9 | $CH_3$ | H | B−4 | H | H | H | CN |
| 316 | A−9 | $CH_3$ | H | B−5 | H | H | H | CN |
| 317 | A−9 | Cℓ | H | B−5 | H | H | H | CN |
| 318 | A−9 | H | $CH_3$ | B−1 | H | H | H | CN |
| 319 | A−9 | H | $CH_3$ | B−2 | H | H | H | CN |
| 320 | A−9 | H | $CH_3$ | B−3 | H | H | H | CN |
| 321 | A−9 | H | $CH_3$ | B−4 | H | H | H | CN |
| 322 | A−9 | H | $CH_3$ | B−5 | H | H | H | CN |
| 323 | A−9 | H | Cℓ | B−5 | H | H | H | CN |
| 324 | A−10 | H | H | B−1 | H | H | H | CN |
| 325 | A−10 | H | H | B−2 | H | H | H | CN |
| 326 | A−10 | H | H | B−3 | H | H | H | CN |
| 327 | A−10 | H | H | B−4 | H | H | H | CN |
| 328 | A−10 | H | H | B−5 | H | H | H | CN |
| 329 | A−10 | $CH_3$ | H | B−1 | H | H | H | CN |
| 330 | A−10 | $CH_3$ | H | B−2 | H | H | H | CN |
| 331 | A−10 | $CH_3$ | H | B−3 | H | H | H | CN |
| 332 | A−10 | $CH_3$ | H | B−4 | H | H | H | CN |

| Compound No. | A | $R_2$ | $R_3$ | B | $R_4$ | $R_5$ | $R_6$ | D |
|---|---|---|---|---|---|---|---|---|
| 333 | A−10 | $CH_3$ | H | B−5 | H | H | H | CN |
| 334 | A−10 | Cℓ | H | B−5 | H | H | H | CN |
| 335 | A−10 | H | $CH_3$ | B−1 | H | H | H | CN |
| 336 | A−10 | H | $CH_3$ | B−2 | H | H | H | CN |
| 337 | A−10 | H | $CH_3$ | B−3 | H | H | H | CN |
| 338 | A−10 | H | $CH_3$ | B−4 | H | H | H | CN |
| 339 | A−10 | H | $CH_3$ | B−5 | H | H | H | CN |
| 340 | A−10 | H | Cℓ | B−5 | H | H | H | CN |
| 341 | A−11 | H | H | B−1 | H | H | H | CN |
| 342 | A−11 | H | H | B−2 | H | H | H | CN |
| 343 | A−11 | H | H | B−3 | H | H | H | CN |
| 344 | A−11 | H | H | B−4 | H | H | H | CN |
| 345 | A−11 | H | H | B−5 | H | H | H | CN |
| 346 | A−11 | $CH_3$ | H | B−1 | H | H | H | CN |
| 347 | A−11 | $CH_3$ | H | B−2 | H | H | H | CN |
| 348 | A−11 | $CH_3$ | H | B−3 | H | H | H | CN |
| 349 | A−11 | $CH_3$ | H | B−4 | H | H | H | CN |
| 350 | A−11 | $CH_3$ | H | B−5 | H | H | H | CN |
| 351 | A−11 | Cℓ | H | B−5 | H | H | H | CN |
| 352 | A−11 | H | $CH_3$ | B−1 | H | H | H | CN |
| 353 | A−11 | H | $CH_3$ | B−2 | H | H | H | CN |
| 354 | A−11 | H | $CH_3$ | B−3 | H | H | H | CN |
| 355 | A−11 | H | $CH_3$ | B−4 | H | H | H | CN |
| 356 | A−11 | H | $CH_3$ | B−5 | H | H | H | CN |
| 357 | A−11 | H | Cℓ | B−5 | H | H | H | CN |
| 358 | A−12 | H | H | B−1 | H | H | H | CN |
| 359 | A−12 | H | H | B−2 | H | H | H | CN |
| 360 | A−12 | H | H | B−3 | H | H | H | CN |
| 361 | A−12 | H | H | B−4 | H | H | H | CN |
| 362 | A−12 | H | H | B−5 | H | H | H | CN |

| Compound No. | A | $R_2$ | $R_3$ | B | $R_4$ | $R_5$ | $R_6$ | D |
|---|---|---|---|---|---|---|---|---|
| 363 | A−12 | $CH_3$ | H | B−1 | H | H | H | CN |
| 364 | A−12 | $CH_3$ | H | B−2 | H | H | H | CN |
| 365 | A−12 | $CH_3$ | H | B−3 | H | H | H | CN |
| 366 | A−12 | $CH_3$ | H | B−4 | H | H | H | CN |
| 367 | A−12 | $CH_3$ | H | B−5 | H | H | H | CN |
| 368 | A−12 | Cℓ | H | B−5 | H | H | H | CN |
| 369 | A−12 | H | $CH_3$ | B−1 | H | H | H | CN |
| 370 | A−12 | H | $CH_3$ | B−2 | H | H | H | CN |
| 371 | A−12 | H | $CH_3$ | B−3 | H | H | H | CN |
| 372 | A−12 | H | $CH_3$ | B−4 | H | H | H | CN |
| 373 | A−12 | H | $CH_3$ | B−5 | H | H | H | CN |
| 374 | A−12 | H | Cℓ | B−5 | H | H | H | CN |
| 375 | A−13 | H | H | B−1 | H | H | H | CN |
| 376 | A−13 | H | H | B−2 | H | H | H | CN |
| 377 | A−13 | H | H | B−3 | H | H | H | CN |
| 378 | A−13 | H | H | B−4 | H | H | H | CN |
| 379 | A−13 | H | H | B−5 | H | H | H | CN |
| 380 | A−13 | $CH_3$ | H | B−1 | H | H | H | CN |
| 381 | A−13 | $CH_3$ | H | B−2 | H | H | H | CN |
| 382 | A−13 | $CH_3$ | H | B−3 | H | H | H | CN |
| 383 | A−13 | $CH_3$ | H | B−4 | H | H | H | CN |
| 384 | A−13 | $CH_3$ | H | B−5 | H | H | H | CN |
| 385 | A−13 | Cℓ | H | B−5 | H | H | H | CN |
| 386 | A−13 | H | $CH_3$ | B−1 | H | H | H | CN |
| 387 | A−13 | H | $CH_3$ | B−2 | H | H | H | CN |
| 388 | A−13 | H | $CH_3$ | B−3 | H | H | H | CN |
| 389 | A−13 | H | $CH_3$ | B−4 | H | H | H | CN |
| 390 | A−13 | H | $CH_3$ | B−5 | H | H | H | CN |
| 391 | A−13 | H | Cℓ | B−5 | H | H | H | CN |
| 392 | A−14 | H | H | B−1 | H | H | H | CN |

| Compound No. | A | $R_2$ | $R_3$ | B | $R_4$ | $R_5$ | $R_6$ | D |
|---|---|---|---|---|---|---|---|---|
| 393 | A−14 | H | H | B−2 | H | H | H | CN |
| 394 | A−14 | H | H | B−3 | H | H | H | CN |
| 395 | A−14 | H | H | B−4 | H | H | H | CN |
| 396 | A−14 | H | H | B−5 | H | H | H | CN |
| 397 | A−14 | $CH_3$ | H | B−1 | H | H | H | CN |
| 398 | A−14 | $CH_3$ | H | B−2 | H | H | H | CN |
| 399 | A−14 | $CH_3$ | H | B−3 | H | H | H | CN |
| 400 | A−14 | $CH_3$ | H | B−4 | H | H | H | CN |
| 401 | A−14 | $CH_3$ | H | B−5 | H | H | H | CN |
| 402 | A−14 | Cℓ | H | B−5 | H | H | H | CN |
| 403 | A−14 | H | $CH_3$ | B−1 | H | H | H | CN |
| 404 | A−14 | H | $CH_3$ | B−2 | H | H | H | CN |
| 405 | A−14 | H | $CH_3$ | B−3 | H | H | H | CN |
| 406 | A−14 | H | $CH_3$ | B−4 | H | H | H | CN |
| 407 | A−14 | H | $CH_3$ | B−5 | H | H | H | CN |
| 408 | A−14 | H | Cℓ | B−5 | H | H | H | CN |
| 409 | A−15 | H | H | B−1 | H | H | H | CN |
| 410 | A−15 | H | H | B−2 | H | H | H | CN |
| 411 | A−15 | H | H | B−3 | H | H | H | CN |
| 412 | A−15 | H | H | B−4 | H | H | H | CN |
| 413 | A−15 | H | H | B−5 | H | H | H | CN |
| 414 | A−15 | $CH_3$ | H | B−1 | H | H | H | CN |
| 415 | A−15 | $CH_3$ | H | B−2 | H | H | H | CN |
| 416 | A−15 | $CH_3$ | H | B−3 | H | H | H | CN |
| 417 | A−15 | $CH_3$ | H | B−4 | H | H | H | CN |
| 418 | A−15 | $CH_3$ | H | B−5 | H | H | H | CN |
| 419 | A−15 | Cℓ | H | B−5 | H | H | H | CN |
| 420 | A−15 | H | $CH_3$ | B−1 | H | H | H | CN |
| 421 | A−15 | H | $CH_3$ | B−2 | H | H | H | CN |
| 422 | A−15 | H | $CH_3$ | B−3 | H | H | H | CN |

| Compound No. | A | $R_2$ | $R_3$ | B | $R_4$ | $R_5$ | $R_6$ | D |
|---|---|---|---|---|---|---|---|---|
| 423 | A−15 | H | $CH_3$ | B−4 | H | H | H | CN |
| 424 | A−15 | H | $CH_3$ | B−5 | H | H | H | CN |
| 425 | A−15 | H | Cℓ | B−5 | H | H | H | CN |
| 426 | A−16 | H | H | B−1 | H | H | H | CN |
| 427 | A−16 | H | H | B−2 | H | H | H | CN |
| 428 | A−16 | H | H | B−3 | H | H | H | CN |
| 429 | A−16 | H | H | B−4 | H | H | H | CN |
| 430 | A−16 | H | H | B−5 | H | H | H | CN |
| 431 | A−16 | $CH_3$ | H | B−1 | H | H | H | CN |
| 432 | A−16 | $CH_3$ | H | B−2 | H | H | H | CN |
| 433 | A−16 | $CH_3$ | H | B−3 | H | H | H | CN |
| 434 | A−16 | $CH_3$ | H | B−4 | H | H | H | CN |
| 435 | A−16 | $CH_3$ | H | B−5 | H | H | H | CN |
| 436 | A−16 | Cℓ | H | B−5 | H | H | H | CN |
| 437 | A−16 | H | $CH_3$ | B−1 | H | H | H | CN |
| 438 | A−16 | H | $CH_3$ | B−2 | H | H | H | CN |
| 439 | A−16 | H | $CH_3$ | B−3 | H | H | H | CN |
| 440 | A−16 | H | $CH_3$ | B−4 | H | H | H | CN |
| 441 | A−16 | H | $CH_3$ | B−5 | H | H | H | CN |
| 442 | A−16 | H | Cℓ | B−5 | H | H | H | CN |
| 443 | A−17 | H | H | B−1 | H | H | H | CN |
| 444 | A−17 | H | H | B−2 | H | H | H | CN |
| 445 | A−17 | H | H | B−3 | H | H | H | CN |
| 446 | A−17 | H | H | B−4 | H | H | H | CN |
| 447 | A−17 | H | H | B−5 | H | H | H | CN |
| 448 | A−17 | $CH_3$ | H | B−1 | H | H | H | CN |
| 449 | A−17 | $CH_3$ | H | B−2 | H | H | H | CN |
| 450 | A−17 | $CH_3$ | H | B−3 | H | H | H | CN |
| 451 | A−17 | $CH_3$ | H | B−4 | H | H | H | CN |
| 452 | A−17 | $CH_3$ | H | B−5 | H | H | H | CN |

| Compound No. | A | $R_2$ | $R_3$ | B | $R_4$ | $R_5$ | $R_6$ | D |
|---|---|---|---|---|---|---|---|---|
| 453 | A−17 | Cℓ | H | B−5 | H | H | H | CN |
| 454 | A−17 | H | $CH_3$ | B−1 | H | H | H | CN |
| 455 | A−17 | H | $CH_3$ | B−2 | H | H | H | CN |
| 456 | A−17 | H | $CH_3$ | B−3 | H | H | H | CN |
| 457 | A−17 | H | $CH_3$ | B−4 | H | H | H | CN |
| 458 | A−17 | H | $CH_3$ | B−5 | H | H | H | CN |
| 459 | A−17 | H | Cℓ | B−5 | H | H | H | CN |
| 460 | A−18 | H | H | B−1 | H | H | H | CN |
| 461 | A−18 | H | H | B−2 | H | H | H | CN |
| 462 | A−18 | H | H | B−3 | H | H | H | CN |
| 463 | A−18 | H | H | B−4 | H | H | H | CN |
| 464 | A−18 | H | H | B−5 | H | H | H | CN |
| 465 | A−18 | $CH_3$ | H | B−1 | H | H | H | CN |
| 466 | A−18 | $CH_3$ | H | B−2 | H | H | H | CN |
| 467 | A−18 | $CH_3$ | H | B−3 | H | H | H | CN |
| 468 | A−18 | $CH_3$ | H | B−4 | H | H | H | CN |
| 469 | A−18 | $CH_3$ | H | B−5 | H | H | H | CN |
| 470 | A−18 | Cℓ | H | B−5 | H | H | H | CN |
| 471 | A−18 | H | $CH_3$ | B−1 | H | H | H | CN |
| 472 | A−18 | H | $CH_3$ | B−2 | H | H | H | CN |
| 473 | A−18 | H | $CH_3$ | B−3 | H | H | H | CN |
| 474 | A−18 | H | $CH_3$ | B−4 | H | H | H | CN |
| 475 | A−18 | H | $CH_3$ | B−5 | H | H | H | CN |
| 476 | A−18 | H | Cℓ | B−5 | H | H | H | CN |

Table 2

$$A-CONH\underset{\displaystyle B}{\underset{|}{C}}H-D$$

| Compound No. | A | $R_1$ | $R_2$ | $R_3$ | B | D |
|---|---|---|---|---|---|---|
| 477 | A-1 | H | H | H | $OCH_2CH_3$ | CN |
| 478 | A-1 | $CH_3$ | H | H | $OCH_2CH_3$ | CN |
| 479 | A-1 | $CH_3$ | H | H | $OCH_2CH_3$ | $CSNH_2$ |
| 480 | A-1 | $CH_3$ | H | H | $OCH_2CH_2F$ | CN |
| 481 | A-1 | $CH_3$ | H | H | $OCH_2CH_2F$ | $CSNH_2$ |
| 482 | A-1 | $CH_3$ | H | H | $OCH_2CH_2C\ell$ | CN |
| 483 | A-1 | $CH_3$ | H | H | $OCH(CH_3)_2$ | CN |
| 484 | A-1 | $CH_3$ | H | H | $OCH_2C\equiv CH$ | CN |
| 485 | A-1 | $CH_3$ | $CH_3$ | H | $OCH_2CH_3$ | CN |
| 486 | A-1 | $CH_3$ | H | $CH_3$ | $OCH_2CH_3$ | CN |
| 487 | A-2 | H | H | H | $OCH_2CH_3$ | CN |
| 488 | A-2 | $CH_3$ | H | H | $OCH_2CH_3$ | CN |
| 489 | A-2 | $CH_3$ | H | H | $OCH_2CH_3$ | $CSNH_2$ |
| 490 | A-2 | $CH_3$ | H | H | $OCH_2CH_2F$ | CN |
| 491 | A-2 | $CH_3$ | H | H | $OCH_2CH_2F$ | $CSNH_2$ |
| 492 | A-2 | $CH_3$ | H | H | $OCH_2CH_2C\ell$ | CN |
| 493 | A-2 | $CH_3$ | H | H | $OCH(CH_3)_2$ | CN |
| 494 | A-2 | $CH_3$ | H | H | $OCH_2C\equiv CH$ | CN |
| 495 | A-2 | $CH_3$ | $CH_3$ | H | $OCH_2CH_3$ | CN |
| 496 | A-2 | $CH_3$ | H | $CH_3$ | $OCH_2CH_3$ | CN |
| 497 | A-3 | H | H | H | $OCH_2CH_3$ | CN |
| 498 | A-3 | $CH_3$ | H | H | $OCH_2CH_3$ | CN |
| 499 | A-3 | $CH_3$ | H | H | $OCH_2CH_3$ | $CSNH_2$ |
| 500 | A-3 | $CH_3$ | H | H | $SCH_2CH_3$ | CN |
| 501 | A-3 | $CH_3$ | H | H | $SCH_2CH_3$ | $CSNH_2$ |

| Compound No. | A | $R_1$ | $R_2$ | $R_3$ | B | D |
|---|---|---|---|---|---|---|
| 502 | A−3 | $CH_3$ | H | H | $OCH_2CH_2F$ | CN |
| 503 | A−3 | $CH_3$ | H | H | $OCH_2CH_2F$ | $CSNH_2$ |
| 504 | A−3 | $CH_3$ | H | H | $OCH_2CH_2C\ell$ | CN |
| 505 | A−3 | $CH_3$ | H | H | $OCH_2C\equiv CH$ | CN |
| 506 | A−3 | $CH_3$ | H | H | $OCH(CH_3)_2$ | CN |
| 507 | A−3 | $CH_2F$ | H | H | $OCH_2CH_3$ | CN |
| 508 | A−3 | $CF_3$ | H | H | $OCH_2CH_3$ | CN |
| 509 | A−3 | $CH_2CH_3$ | H | H | $OCH_2CH_3$ | CN |
| 510 | A−3 | $CH_2CH_3$ | H | H | $OCH_2CH_3$ | $CSNH_2$ |
| 511 | A−3 | $CH_3$ | $CH_3$ | H | $OCH_3$ | CN |
| 512 | A−3 | $CH_3$ | $CH_3$ | H | $OCH_2CH_3$ | CN |
| 513 | A−3 | $CH_3$ | $CH_3$ | H | $OCH_2CH_3$ | $CSNH_2$ |
| 514 | A−3 | $CH_3$ | $CH_3$ | H | $OCH_2CH_3$ | $CSNHCOCH_3$ |
| 515 | A−3 | $CH_3$ | $CH_3$ | H | $SCH_2CH_3$ | CN |
| 516 | A−3 | $CH_3$ | $CH_3$ | H | $SCH_2CH_3$ | $CSNH_2$ |
| 517 | A−3 | $CH_3$ | $CH_3$ | H | $SCH_2CH_3$ | $CSNHCOCH_3$ |
| 518 | A−3 | $CH_3$ | $CH_3$ | H | $OCH_2CH_2F$ | CN |
| 519 | A−3 | $CH_3$ | $CH_3$ | H | $OCH_2CH_2F$ | $CSNH_2$ |
| 520 | A−3 | $CH_3$ | $CH_3$ | H | $OCH(CH_3)_2$ | CN |
| 521 | A−3 | $CH_3$ | $CH_3$ | H | $SCH(CH_3)_2$ | CN |
| 522 | A−3 | $CH_3$ | $CH_3$ | H | $OCH_2CH_2CH_3$ | CN |
| 523 | A−3 | $CH_3$ | $CH_3$ | H | $SCH_2CH_2CH_3$ | CN |
| 524 | A−3 | $CH_3$ | $CH_3$ | H | $OCH_2CH_2C\ell$ | CN |
| 525 | A−3 | $CH_3$ | $CH_3$ | H | 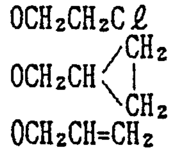 | CN |
| 526 | A−3 | $CH_3$ | $CH_3$ | H | $OCH_2CH=CH_2$ | CN |
| 527 | A−3 | $CH_3$ | $CH_3$ | H | $OCH_2C\equiv CH$ | CN |
| 528 | A−3 | $CH_3$ | $CH_3$ | H | $OCH_2C\equiv CCH_3$ | CN |
| 529 | A−3 | $CH_3$ | $CH_3$ | H | $OCH_2CH_2OCH_3$ | CN |
| 530 | A−3 | $CH_3$ | $CH_3$ | H | $OCH_2CH_2CH_2CH_3$ | CN |
| 531 | A−3 | $CH_3$ | $CH_3$ | H | $SCH_2CH_2CH_2CH_3$ | CN |

| Compound No. | A | $R_1$ | $R_2$ | $R_3$ | B | D |
|---|---|---|---|---|---|---|
| 532 | A－3 | $CH_3$ | $CH_3$ | H | $OCH(CH_3)CN$ | CN |
| 533 | A－3 | $CH_3$ | $CH_3$ | H | $OCH_2CH_2CN$ | CN |
| 534 | A－3 | $CH_3$ | $CH_3$ | H | 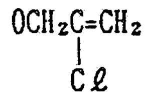 | CN |
| 535 | A－3 | $CH_3$ | $CH_3$ | H | 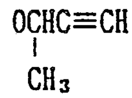 | CN |
| 536 | A－3 | $CH_2F$ | $CH_3$ | H | $OCH_2CH_3$ | CN |
| 537 | A－3 | $CHF_2$ | $CH_3$ | H | $OCH_2CH_3$ | CN |
| 538 | A－3 | $CF_3$ | $CH_3$ | H | $OCH_2CH_3$ | CN |
| 539 | A－3 | $CH_2CH_3$ | $CH_3$ | H | $OCH_2CH_3$ | CN |
| 540 | A－3 | $CH_2CH_2F$ | $CH_3$ | H | $OCH_2CH_3$ | CN |
| 541 | A－3 | $CH=CH_2$ | $CH_3$ | H | $OCH_2CH_3$ | CN |
| 542 | A－3 | $CH_2CH=CH_2$ | $CH_3$ | H | $OCH_2CH_3$ | CN |
| 543 | A－3 | $CH_3$ | H | $CH_3$ | $OCH_2CH_3$ | CN |
| 544 | A－3 | $CH_3$ | H | $CH_3$ | $OCH_2CH_3$ | $CSNH_2$ |
| 545 | A－3 | $CH_3$ | Cℓ | H | $OCH_2CH_3$ | CN |
| 546 | A－3 | $CH_3$ | $CF_3$ | H | $OCH_2CH_3$ | CN |
| 547 | A－3 | $CH_3$ | $CH_3O$ | H | $OCH_2CH_3$ | CN |
| 548 | A－3 | $CH_3$ | CN | H | $OCH_2CH_3$ | CN |
| 549 | A－3 | $CH_3$ | $CH_3$ | Br | $OCH_2CH_3$ | CN |
| 550 | A－3 | $CH_3$ | $CH_3$ | $CH_3$ | $OCH_2CH_3$ | CN |
| 551 | A－3 | $CH_3$ | $CH_2CH_3$ | H | $OCH_2CH_3$ | CN |
| 552 | A－4 | H | H | H | $OCH_2CH_3$ | CN |
| 553 | A－4 | $CH_3$ | H | H | $OCH_2CH_3$ | CN |
| 554 | A－4 | $CH_3$ | H | H | $OCH_2CH_3$ | $CSNH_2$ |
| 555 | A－4 | $CH_3$ | H | H | $SCH_2CH_3$ | CN |
| 556 | A－4 | $CH_3$ | H | H | $SCH_2CH_3$ | $CSNH_2$ |
| 557 | A－4 | $CH_3$ | H | H | $OCH_2CH_2F$ | CN |
| 558 | A－4 | $CH_3$ | H | H | $OCH_2CH_2Cℓ$ | CN |
| 559 | A－4 | $CH_3$ | H | H | $OCH(CH_3)_2$ | CN |

| Compound No. | A | $R_1$ | $R_2$ | $R_3$ | B | D |
|---|---|---|---|---|---|---|
| 560 | A－4 | $CH_3$ | H | H | $SCH(CH_3)_2$ | CN |
| 561 | A－4 | $CH_3$ | H | H | $OCH_2C\equiv CH$ | CN |
| 562 | A－4 | $CH_3$ | $CH_3$ | H | $OCH_2CH_3$ | CN |
| 563 | A－4 | $CH_3$ | H | $CH_3$ | $OCH_2CH_3$ | CN |
| 564 | A－5 | H | H | H | $OCH_2CH_3$ | CN |
| 565 | A－5 | $CH_3$ | H | H | $OCH_2CH_3$ | CN |
| 566 | A－5 | $CH_3$ | H | H | $OCH_2CH_3$ | $CSNH_2$ |
| 567 | A－5 | $CH_3$ | H | H | $SCH_2CH_3$ | CN |
| 568 | A－5 | $CH_3$ | H | H | $SCH_2CH_3$ | $CSNH_2$ |
| 569 | A－5 | $CH_3$ | H | H | $OCH_2CH_2F$ | CN |
| 570 | A－5 | $CH_3$ | H | H | $OCH_2CH_2C\ell$ | CN |
| 571 | A－5 | $CH_3$ | H | H | $OCH(CH_3)_2$ | CN |
| 572 | A－5 | $CH_3$ | H | H | $SCH(CH_3)_2$ | CN |
| 573 | A－5 | $CH_3$ | H | H | $OCH_2C\equiv CH$ | CN |
| 574 | A－5 | $CH_3$ | $CH_3$ | H | $OCH_2CH_3$ | CN |
| 575 | A－5 | $CH_3$ | H | $CH_3$ | $OCH_2CH_3$ | CN |
| 576 | A－6 | H | H | H | $OCH_2CH_3$ | CN |
| 577 | A－6 | $CH_3$ | H | H | $OCH_2CH_3$ | CN |
| 578 | A－6 | $CH_3$ | H | H | $OCH_2CH_3$ | $CSNH_2$ |
| 579 | A－6 | $CH_3$ | H | H | $SCH_2CH_3$ | CN |
| 580 | A－6 | $CH_3$ | H | H | $SCH_2CH_3$ | $CSNH_2$ |
| 581 | A－6 | $CH_3$ | H | H | $OCH_2CH_2F$ | CN |
| 582 | A－6 | $CH_3$ | H | H | $OCH_2CH_2C\ell$ | CN |
| 583 | A－6 | $CH_3$ | H | H | $OCH(CH_3)_2$ | CN |
| 584 | A－6 | $CH_3$ | H | H | $SCH(CH_3)_2$ | CN |
| 585 | A－6 | $CH_3$ | H | H | $OCH_2C\equiv CH$ | CN |
| 586 | A－6 | $CH_3$ | $CH_3$ | H | $OCH_2CH_3$ | CN |
| 587 | A－6 | $CH_3$ | H | $CH_3$ | $OCH_2CH_3$ | CN |

| Compound No. | A | $R_2$ | $R_3$ | B | D |
|---|---|---|---|---|---|
| 588 | A−7 | H | H | $OCH_2CH_3$ | CN |
| 589 | A−7 | H | H | $OCH_2CH_3$ | $CSNH_2$ |
| 590 | A−7 | H | H | $SCH_2CH_3$ | CN |
| 591 | A−7 | H | H | $SCH_2CH_3$ | $CSNH_2$ |
| 592 | A−7 | H | H | $OCH_2CH_2F$ | CN |
| 593 | A−7 | H | H | $OCH_2CH_2C\ell$ | CN |
| 594 | A−7 | H | H | $OCH(CH_3)_2$ | CN |
| 595 | A−7 | H | H | $SCH(CH_3)_2$ | CN |
| 596 | A−7 | H | H | $OCH_2C\equiv CH$ | CN |
| 597 | A−7 | $CH_3$ | H | $OCH_2CH_3$ | CN |
| 598 | A−7 | H | $CH_3$ | $OCH_2CH_3$ | CN |
| 599 | A−8 | H | H | $OCH_2CH_3$ | CN |
| 600 | A−8 | H | H | $OCH_2CH_3$ | $CSNH_2$ |
| 601 | A−8 | H | H | $SCH_2CH_3$ | CN |
| 602 | A−8 | H | H | $SCH_2CH_3$ | $CSNH_2$ |
| 603 | A−8 | H | H | $OCH_2CH_2F$ | CN |
| 604 | A−8 | H | H | $OCH_2CH_2C\ell$ | CN |
| 605 | A−8 | H | H | $OCH(CH_3)_2$ | CN |
| 606 | A−8 | H | H | $SCH(CH_3)_2$ | CN |
| 607 | A−8 | H | H | $OCH_2C\equiv CH$ | CN |
| 608 | A−8 | $CH_3$ | H | $OCH_2CH_3$ | CN |
| 609 | A−8 | H | $CH_3$ | $OCH_2CH_3$ | CN |
| 610 | A−9 | H | H | $OCH_2CH_3$ | CN |
| 611 | A−9 | H | H | $OCH_2CH_3$ | $CSNH_2$ |
| 612 | A−9 | H | H | $SCH_2CH_3$ | CN |
| 613 | A−9 | H | H | $SCH_2CH_3$ | $CSNH_2$ |
| 614 | A−9 | H | H | $OCH_2CH_2F$ | CN |
| 615 | A−9 | H | H | $OCH_2CH_2C\ell$ | CN |
| 616 | A−9 | H | H | $OCH(CH_3)_2$ | CN |
| 617 | A−9 | H | H | $SCH(CH_3)_2$ | CN |

| Compound No. | A | $R_2$ | $R_3$ | B | D |
|---|---|---|---|---|---|
| 618 | A−9 | H | H | $OCH_2C\equiv CH$ | CN |
| 619 | A−9 | $CH_3$ | H | $OCH_2CH_3$ | CN |
| 620 | A−9 | H | $CH_3$ | $OCH_2CH_3$ | CN |
| 621 | A−10 | H | H | $OCH_2CH_3$ | CN |
| 622 | A−10 | H | H | $OCH_2CH_3$ | $CSNH_2$ |
| 623 | A−10 | H | H | $SCH_2CH_3$ | CN |
| 624 | A−10 | H | H | $SCH_2CH_3$ | $CSNH_2$ |
| 625 | A−10 | H | H | $OCH_2CH_2F$ | CN |
| 626 | A−10 | H | H | $OCH_2CH_2Cl$ | CN |
| 627 | A−10 | H | H | $OCH(CH_3)_2$ | CN |
| 628 | A−10 | H | H | $SCH(CH_3)_2$ | CN |
| 629 | A−10 | H | H | $OCH_2C\equiv CH$ | CN |
| 630 | A−10 | $CH_3$ | H | $OCH_2CH_3$ | CN |
| 631 | A−10 | H | $CH_3$ | $OCH_2CH_3$ | CN |
| 632 | A−11 | H | H | $OCH_2CH_3$ | CN |
| 633 | A−11 | H | H | $OCH_2CH_3$ | $CSNH_2$ |
| 634 | A−11 | H | H | $SCH_2CH_3$ | CN |
| 635 | A−11 | H | H | $SCH_2CH_3$ | $CSNH_2$ |
| 636 | A−11 | H | H | $OCH_2CH_2F$ | CN |
| 637 | A−11 | H | H | $OCH_2CH_2Cl$ | CN |
| 638 | A−11 | H | H | $OCH(CH_3)_2$ | CN |
| 639 | A−11 | H | H | $SCH(CH_3)_2$ | CN |
| 640 | A−11 | H | H | $OCH_2C\equiv CH$ | CN |
| 641 | A−11 | $CH_3$ | H | $OCH_2CH_3$ | CN |
| 642 | A−11 | H | $CH_3$ | $OCH_2CH_3$ | CN |
| 643 | A−12 | H | H | $OCH_2CH_3$ | CN |
| 644 | A−12 | H | H | $OCH_2CH_3$ | $CSNH_2$ |
| 645 | A−12 | H | H | $SCH_2CH_3$ | CN |
| 646 | A−12 | H | H | $SCH_2CH_3$ | $CSNH_2$ |
| 647 | A−12 | H | H | $OCH_2CH_2F$ | CN |

| Compound No. | A | $R_2$ | $R_3$ | B | D |
|---|---|---|---|---|---|
| 648 | A−12 | H | H | $OCH_2CH_2Cl$ | CN |
| 649 | A−12 | H | H | $OCH(CH_3)_2$ | CN |
| 650 | A−12 | H | H | $SCH(CH_3)_2$ | CN |
| 651 | A−12 | H | H | $OCH_2C\equiv CH$ | CN |
| 652 | A−12 | $CH_3$ | H | $OCH_2CH_3$ | CN |
| 653 | A−12 | H | $CH_3$ | $OCH_2CH_3$ | CN |
| 654 | A−13 | H | H | $OCH_2CH_3$ | CN |
| 655 | A−13 | H | H | $OCH_2CH_3$ | $CSNH_2$ |
| 656 | A−13 | H | H | $SCH_2CH_3$ | CN |
| 657 | A−13 | H | H | $SCH_2CH_3$ | $CSNH_2$ |
| 658 | A−13 | H | H | $OCH_2CH_2F$ | CN |
| 659 | A−13 | H | H | $OCH_2CH_2Cl$ | CN |
| 660 | A−13 | H | H | $OCH(CH_3)_2$ | CN |
| 661 | A−13 | H | H | $SCH(CH_3)_2$ | CN |
| 662 | A−13 | H | H | $OCH_2C\equiv CH$ | CN |
| 663 | A−13 | $CH_3$ | H | $OCH_2CH_3$ | CN |
| 664 | A−13 | H | $CH_3$ | $OCH_2CH_3$ | CN |
| 665 | A−14 | H | H | $OCH_2CH_3$ | CN |
| 666 | A−14 | H | H | $OCH_2CH_3$ | $CSNH_2$ |
| 667 | A−14 | H | H | $SCH_2CH_3$ | CN |
| 668 | A−14 | H | H | $SCH_2CH_3$ | $CSNH_2$ |
| 669 | A−14 | H | H | $OCH_2CH_2F$ | CN |
| 670 | A−14 | H | H | $OCH_2CH_2Cl$ | CN |
| 671 | A−14 | H | H | $OCH(CH_3)_2$ | CN |
| 672 | A−14 | H | H | $SCH(CH_3)_2$ | CN |
| 673 | A−14 | H | H | $OCH_2C\equiv CH$ | CN |
| 674 | A−14 | $CH_3$ | H | $OCH_2CH_3$ | CN |
| 675 | A−14 | H | $CH_3$ | $OCH_2CH_3$ | CN |
| 676 | A−15 | H | H | $OCH_2CH_3$ | CN |
| 677 | A−15 | H | H | $OCH_2CH_3$ | $CSNH_2$ |

| Compound No. | A | $R_2$ | $R_3$ | B | D |
|---|---|---|---|---|---|
| 678 | A−15 | H | H | $SCH_2CH_3$ | CN |
| 679 | A−15 | H | H | $SCH_2CH_3$ | $CSNH_2$ |
| 680 | A−15 | H | H | $OCH_2CH_2F$ | CN |
| 681 | A−15 | H | H | $OCH_2CH_2C\ell$ | CN |
| 682 | A−15 | H | H | $OCH(CH_3)_2$ | CN |
| 683 | A−15 | H | H | $SCH(CH_3)_2$ | CN |
| 684 | A−15 | H | H | $OCH_2C\equiv CH$ | CN |
| 685 | A−15 | $CH_3$ | H | $OCH_2CH_3$ | CN |
| 686 | A−15 | H | $CH_3$ | $OCH_2CH_3$ | CN |
| 687 | A−16 | H | H | $OCH_2CH_3$ | CN |
| 688 | A−16 | H | H | $OCH_2CH_3$ | $CSNH_2$ |
| 689 | A−16 | H | H | $SCH_2CH_3$ | CN |
| 690 | A−16 | H | H | $SCH_2CH_3$ | $CSNH_2$ |
| 691 | A−16 | H | H | $OCH_2CH_2F$ | CN |
| 692 | A−16 | H | H | $OCH_2CH_2C\ell$ | CN |
| 693 | A−16 | H | H | $OCH(CH_3)_2$ | CN |
| 694 | A−16 | H | H | $SCH(CH_3)_2$ | CN |
| 695 | A−16 | H | H | $OCH_2C\equiv CH$ | CN |
| 696 | A−16 | $CH_3$ | H | $OCH_2CH_3$ | CN |
| 697 | A−16 | H | $CH_3$ | $OCH_2CH_3$ | CN |
| 698 | A−17 | H | H | $OCH_2CH_3$ | CN |
| 699 | A−17 | H | H | $OCH_2CH_3$ | $CSNH_2$ |
| 700 | A−17 | H | H | $SCH_2CH_3$ | CN |
| 701 | A−17 | H | H | $SCH_2CH_3$ | $CSNH_2$ |
| 702 | A−17 | H | H | $OCH_2CH_2F$ | CN |
| 703 | A−17 | H | H | $OCH_2CH_2C\ell$ | CN |
| 704 | A−17 | H | H | $OCH(CH_3)_2$ | CN |
| 705 | A−17 | H | H | $SCH(CH_3)_2$ | CN |
| 706 | A−17 | H | H | $OCH_2C\equiv CH$ | CN |
| 707 | A−17 | $CH_3$ | H | $OCH_2CH_3$ | CN |

| Compound No. | A | $R_2$ | $R_3$ | B | D |
|---|---|---|---|---|---|
| 708 | A−17 | H | $CH_3$ | $OCH_2CH_3$ | CN |
| 709 | A−18 | H | H | $OCH_2CH_3$ | CN |
| 710 | A−18 | H | H | $OCH_2CH_3$ | $CSNH_2$ |
| 711 | A−18 | H | H | $SCH_2CH_3$ | CN |
| 712 | A−18 | H | H | $SCH_2CH_3$ | $CSNH_2$ |
| 713 | A−18 | H | H | $OCH_2CH_2F$ | CN |
| 714 | A−18 | H | H | $OCH_2CH_2C\ell$ | CN |
| 715 | A−18 | H | H | $OCH(CH_3)_2$ | CN |
| 716 | A−18 | H | H | $SCH(CH_3)_2$ | CN |
| 717 | A−18 | H | H | $OCH_2C\equiv CH$ | CN |
| 718 | A−18 | $CH_3$ | H | $OCH_2CH_3$ | CN |
| 719 | A−18 | H | $CH_3$ | $OCH_2CH_3$ | CN |

Table 3

A−CONHCH−D
　　　　|
　　　　B

| Compound No. | A | $R_1$ | $R_2$ | $R_3$ | B | $R_4$ | $R_5$ | $R_6$ | D |
|---|---|---|---|---|---|---|---|---|---|
| 720 | A−1 | H | H | H | B−6 | H | H | H | CN |
| 721 | A−1 | H | H | H | B−7 | H | H | H | CN |
| 722 | A−1 | $CH_3$ | H | H | B−6 | H | H | H | CN |
| 723 | A−1 | $CH_3$ | H | H | B−7 | H | H | H | CN |
| 724 | A−1 | $CH_3$ | $CH_3$ | H | B−6 | H | H | H | CN |
| 725 | A−1 | $CH_3$ | $CH_3$ | H | B−7 | H | H | H | CN |
| 726 | A−1 | $CH_3$ | H | $CH_3$ | B−6 | H | H | H | CN |
| 727 | A−1 | $CH_3$ | H | $CH_3$ | B−7 | H | H | H | CN |
| 728 | A−1 | $CH_3$ | Cℓ | H | B−6 | H | H | H | CN |
| 729 | A−1 | $CH_3$ | Cℓ | H | B−7 | H | H | H | CN |
| 730 | A−1 | $CH_3$ | $CF_3$ | H | B−6 | H | H | H | CN |
| 731 | A−1 | $CH_3$ | $CF_3$ | H | B−7 | H | H | H | CN |
| 732 | A−1 | $CH_3$ | H | $CH_3O$ | B−6 | H | H | H | CN |
| 733 | A−1 | $CH_3$ | H | $CH_3O$ | B−7 | H | H | H | CN |
| 734 | A−2 | H | H | H | B−6 | H | H | H | CN |
| 735 | A−2 | H | H | H | B−7 | H | H | H | CN |
| 736 | A−2 | $CH_3$ | H | H | B−6 | H | H | H | CN |
| 737 | A−2 | $CH_3$ | H | H | B−6 | H | H | H | $CSNH_2$ |
| 738 | A−2 | $CH_3$ | H | H | B−7 | H | H | H | CN |
| 739 | A−2 | $CH_3$ | H | H | B−7 | H | H | H | $CSNH_2$ |
| 740 | A−2 | $CH_3$ | $CH_3$ | H | B−6 | H | H | H | CN |
| 741 | A−2 | $CH_3$ | $CH_3$ | H | B−7 | H | H | H | CN |
| 742 | A−2 | $CH_3$ | H | $CH_3$ | B−6 | H | H | H | CN |
| 743 | A−2 | $CH_3$ | H | $CH_3$ | B−7 | H | H | H | CN |
| 744 | A−2 | $CH_2CH_3$ | H | H | B−6 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 745 | A−2 | $CH_2CH_3$ | H | H | B−7 | H | H | H | CN |
| 746 | A−2 | $CH_3$ | Cℓ | H | B−6 | H | H | H | CN |
| 747 | A−2 | $CH_3$ | Cℓ | H | B−7 | H | H | H | CN |
| 748 | A−2 | $CH_3$ | H | Cℓ | B−6 | H | H | H | CN |
| 749 | A−2 | $CH_3$ | H | Cℓ | B−7 | H | H | H | CN |
| 750 | A−2 | $CH_3$ | Cℓ | Cℓ | B−6 | H | H | H | CN |
| 751 | A−2 | $CH_3$ | Cℓ | Cℓ | B−7 | H | H | H | CN |
| 752 | A−2 | $CH_3$ | $CF_3$ | H | B−6 | H | H | H | CN |
| 753 | A−2 | $CH_3$ | $CF_3$ | H | B−7 | H | H | H | CN |
| 754 | A−2 | $CH_2F$ | $CH_3$ | H | B−6 | H | H | H | CN |
| 755 | A−2 | $CH_2F$ | $CH_3$ | H | B−7 | H | H | H | CN |
| 756 | A−2 | $CF_3$ | $CH_3$ | H | B−6 | H | H | H | CN |
| 757 | A−2 | $CF_3$ | $CH_3$ | H | B−7 | H | H | H | CN |
| 758 | A−3 | H | H | H | B−6 | H | H | H | CN |
| 759 | A−3 | H | H | H | B−7 | H | H | H | CN |
| 760 | A−3 | $CH_3$ | H | H | B−6 | H | H | H | CN |
| 761 | A−3 | $CH_3$ | H | H | B−7 | H | H | H | CN |
| 762 | A−3 | $CH_3$ | $CH_3$ | H | B−6 | H | H | H | CN |
| 763 | A−3 | $CH_3$ | $CH_3$ | H | B−6 | H | H | H | $CSNH_2$ |
| 764 | A−3 | $CH_3$ | $CH_3$ | H | B−6 | H | H | H | $CSNHCOCH_3$ |
| 765 | A−3 | $CH_3$ | $CH_3$ | H | B−7 | H | H | H | CN |
| 766 | A−3 | $CH_3$ | $CH_3$ | H | B−7 | H | H | H | $CSNH_2$ |
| 767 | A−3 | $CH_3$ | $CH_3$ | H | B−7 | H | H | H | $CSNHCOCH_3$ |
| 768 | A−3 | $CH_3$ | H | $CH_3$ | B−6 | H | H | H | CN |
| 769 | A−3 | $CH_3$ | H | $CH_3$ | B−7 | H | H | H | CN |
| 770 | A−3 | $CH_2CH_3$ | $CH_3$ | H | B−6 | H | H | H | CN |
| 771 | A−3 | $CH_2CH_3$ | $CH_3$ | H | B−7 | H | H | H | CN |
| 772 | A−3 | $CH_2F$ | $CH_3$ | $CH_3$ | B−6 | H | H | H | CN |
| 773 | A−3 | $CH_2F$ | $CH_3$ | $CH_3$ | B−7 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 774 | A-3 | $CHF_2$ | $CH_3$ | $CH_3$ | B-6 | H | H | H | CN |
| 775 | A-3 | $CHF_2$ | $CH_3$ | $CH_3$ | B-7 | H | H | H | CN |
| 776 | A-3 | $CF_3$ | $CH_3$ | $CH_3$ | B-6 | H | H | H | CN |
| 777 | A-3 | $CF_3$ | $CH_3$ | $CH_3$ | B-7 | H | H | H | CN |
| 778 | A-3 | $CH=CH_2$ | $CH_3$ | $CH_3$ | B-6 | H | H | H | CN |
| 779 | A-3 | $CH=CH_2$ | $CH_3$ | $CH_3$ | B-7 | H | H | H | CN |
| 780 | A-3 | $CF=CF_2$ | $CH_3$ | $CH_3$ | B-6 | H | H | H | CN |
| 781 | A-3 | $CF=CF_2$ | $CH_3$ | $CH_3$ | B-7 | H | H | H | CN |
| 782 | A-3 | $CH_2CH=CH_2$ | $CH_3$ | H | B-6 | H | H | H | CN |
| 783 | A-3 | $CH_2CH=CH_2$ | $CH_3$ | H | B-7 | H | H | H | CN |
| 784 | A-3 | $CH_2C\equiv CH$ | $CH_3$ | H | B-6 | H | H | H | CN |
| 785 | A-3 | $CH_2C\equiv CH$ | $CH_3$ | H | B-7 | H | H | H | CN |
| 786 | A-3 | $CH=C=CH_2$ | $CH_3$ | H | B-6 | H | H | H | CN |
| 787 | A-3 | $CH=C=CH_2$ | $CH_3$ | H | B-7 | H | H | H | CN |
| 788 | A-3 | $CH_3$ | $CF_3$ | H | B-6 | H | H | H | CN |
| 789 | A-3 | $CH_3$ | $CF_3$ | H | B-6 | H | H | H | $CSNH_2$ |
| 790 | A-3 | $CH_3$ | $CF_3$ | H | B-7 | H | H | H | CN |
| 791 | A-3 | $CH_3$ | $CF_3$ | H | B-7 | H | H | H | $CSNH_2$ |
| 792 | A-3 | $CH_3$ | Cℓ | H | B-6 | H | H | H | CN |
| 793 | A-3 | $CH_3$ | Cℓ | H | B-7 | H | H | H | CN |
| 794 | A-3 | $CH_3$ | Cℓ | Cℓ | B-6 | H | H | H | CN |
| 795 | A-3 | $CH_3$ | Cℓ | Cℓ | B-7 | H | H | H | CN |
| 796 | A-3 | $CH_3$ | Br | H | B-6 | H | H | H | CN |
| 797 | A-3 | $CH_3$ | Br | H | B-7 | H | H | H | CN |
| 798 | A-3 | $CH_3$ | H | Br | B-6 | H | H | H | CN |
| 799 | A-3 | $CH_3$ | H | Br | B-7 | H | H | H | CN |
| 800 | A-3 | $CH_3$ | CN | H | B-6 | H | H | H | CN |
| 801 | A-3 | $CH_3$ | CN | H | B-7 | H | H | H | CN |
| 802 | A-3 | $CH_3$ | H | CN | B-6 | H | H | H | CN |
| 803 | A-3 | $CH_3$ | H | CN | B-7 | H | H | H | CN |
| 804 | A-3 | $CH_3$ | $CH_3$ | $CH_3$ | B-6 | H | H | H | CN |
| 805 | A-3 | $CH_3$ | $CH_3$ | $CH_3$ | B-7 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 806 | A−3 | $CH_3$ | $CH_3$ | F | B−6 | H | H | H | CN |
| 807 | A−3 | $CH_3$ | $CH_3$ | F | B−7 | H | H | H | CN |
| 808 | A−3 | $CH_3$ | $CH_3$ | Cℓ | B−6 | H | H | H | CN |
| 809 | A−3 | $CH_3$ | $CH_3$ | Cℓ | B−7 | H | H | H | CN |
| 810 | A−3 | $CH_3$ | $CH_3$ | Br | B−6 | H | H | H | CN |
| 811 | A−3 | $CH_3$ | $CH_3$ | Br | B−7 | H | H | H | CN |
| 812 | A−3 | $CH_3$ | $CH_2CH_3$ | H | B−6 | H | H | H | CN |
| 813 | A−3 | $CH_3$ | $CH_2CH_3$ | H | B−7 | H | H | H | CN |
| 814 | A−3 | $CH_3$ | $CH_3O$ | H | B−6 | H | H | H | CN |
| 815 | A−3 | $CH_3$ | $CH_3O$ | H | B−7 | H | H | H | CN |
| 816 | A−3 | $CH_3$ | H | $CH_3O$ | B−6 | H | H | H | CN |
| 817 | A−3 | $CH_3$ | H | $CH_3O$ | B−7 | H | H | H | CN |
| 818 | A−3 | $CH_3$ | $CH_3S$ | H | B−6 | H | H | H | CN |
| 819 | A−3 | $CH_3$ | $CH_3S$ | H | B−7 | H | H | H | CN |
| 820 | A−3 | H | $CH_3$ | H | B−6 | H | H | H | CN |
| 821 | A−3 | H | $CH_3$ | H | B−7 | H | H | H | CN |
| 822 | A−3 | $CH_3$ | $C(CH_3)_3$ | H | B−6 | H | H | H | CN |
| 823 | A−3 | $CH_3$ | $C(CH_3)_3$ | H | B−7 | H | H | H | CN |
| 824 | A−3 | $CH_2CH_3$ | $CH_3$ | H | B−6 | H | H | H | $CSNH_2$ |
| 825 | A−3 | $CH_2CH_3$ | $CH_3$ | H | B−7 | H | H | H | $CSNH_2$ |
| 826 | A−3 | $CH_2CH_3$ | H | H | B−6 | H | H | H | CN |
| 827 | A−3 | $CH_2CH_3$ | H | H | B−7 | H | H | H | CN |
| 828 | A−3 | $CH_3$ | $CH_3$ | H | B−7 | Cℓ | H | H | CN |
| 829 | A−3 | $CH_3$ | $CH_3$ | H | B−7 | H | Cℓ | H | CN |
| 830 | A−3 | $CH_3$ | $CH_3$ | H | B−7 | H | H | Cℓ | CN |
| 831 | A−3 | $CH_3$ | $CH_3$ | H | B−7 | Br | H | H | CN |
| 832 | A−3 | $CH_3$ | $CH_3$ | H | B−7 | H | Br | H | CN |
| 833 | A−3 | $CH_3$ | $CH_3$ | H | B−7 | H | H | Br | CN |
| 834 | A−3 | $CH_3$ | $CH_3$ | H | B−7 | $CH_3$ | H | H | CN |
| 835 | A−3 | $CH_3$ | $CH_3$ | H | B−7 | H | $CH_3$ | H | CN |
| 836 | A−3 | $CH_3$ | $CH_3$ | H | B−7 | H | H | $CH_3$ | CN |

| Compound No. | A | $R_1$ | $R_2$ | $R_3$ | B | $R_4$ | $R_5$ | $R_6$ | D |
|---|---|---|---|---|---|---|---|---|---|
| 837 | A－4 | H | H | H | B－6 | H | H | H | CN |
| 838 | A－4 | H | H | H | B－7 | H | H | H | CN |
| 839 | A－4 | $CH_3$ | H | H | B－6 | H | H | H | CN |
| 840 | A－4 | $CH_3$ | H | H | B－7 | H | H | H | CN |
| 841 | A－4 | $CH_3$ | $CH_3$ | H | B－6 | H | H | H | CN |
| 842 | A－4 | $CH_3$ | $CH_3$ | H | B－7 | H | H | H | CN |
| 843 | A－4 | $CH_3$ | H | $CH_3$ | B－6 | H | H | H | CN |
| 844 | A－4 | $CH_3$ | H | $CH_3$ | B－7 | H | H | H | CN |
| 845 | A－4 | $CH_3$ | Cℓ | H | B－6 | H | H | H | CN |
| 846 | A－4 | $CH_3$ | Cℓ | H | B－7 | H | H | H | CN |
| 847 | A－4 | $CH_3$ | H | Cℓ | B－7 | H | H | H | CN |
| 848 | A－4 | $CH_3$ | H | Cℓ | B－7 | H | H | H | CN |
| 849 | A－4 | $CH_3$ | $CH_3$ | $CH_3$ | B－6 | H | H | H | CN |
| 850 | A－4 | $CH_3$ | $CH_3$ | $CH_3$ | B－7 | H | H | H | CN |
| 851 | A－5 | H | H | H | B－6 | H | H | H | CN |
| 852 | A－5 | H | H | H | B－7 | H | H | H | CN |
| 853 | A－5 | $CH_3$ | H | H | B－6 | H | H | H | CN |
| 854 | A－5 | $CH_3$ | H | H | B－7 | H | H | H | CN |
| 855 | A－5 | $CH_3$ | $CH_3$ | H | B－6 | H | H | H | CN |
| 856 | A－5 | $CH_3$ | $CH_3$ | H | B－7 | H | H | H | CN |
| 857 | A－5 | $CH_3$ | H | $CH_3$ | B－6 | H | H | H | CN |
| 858 | A－5 | $CH_3$ | H | $CH_3$ | B－7 | H | H | H | CN |
| 859 | A－5 | $CH_3$ | Cℓ | H | B－6 | H | H | H | CN |
| 860 | A－5 | $CH_3$ | Cℓ | H | B－7 | H | H | H | CN |
| 861 | A－5 | $CH_3$ | H | Cℓ | B－6 | H | H | H | CN |
| 862 | A－5 | $CH_3$ | H | Cℓ | B－7 | H | H | H | CN |
| 863 | A－5 | $CH_3$ | $CH_3$ | $CH_3$ | B－6 | H | H | H | CN |
| 864 | A－5 | $CH_3$ | $CH_3$ | $CH_3$ | B－7 | H | H | H | CN |

| Compound No. | A | $R_1$ | $R_2$ | $R_3$ | B | $R_4$ | $R_5$ | $R_6$ | D |
|---|---|---|---|---|---|---|---|---|---|
| 865 | A-6 | H | H | H | B-6 | H | H | H | CN |
| 866 | A-6 | H | H | H | B-7 | H | H | H | CN |
| 867 | A-6 | $CH_3$ | H | H | B-6 | H | H | H | CN |
| 868 | A-6 | $CH_3$ | H | H | B-7 | H | H | H | CN |
| 869 | A-6 | $CH_3$ | $CH_3$ | H | B-6 | H | H | H | CN |
| 870 | A-6 | $CH_3$ | $CH_3$ | H | B-7 | H | H | H | CN |
| 871 | A-6 | $CH_3$ | H | $CH_3$ | B-6 | H | H | H | CN |
| 872 | A-6 | $CH_3$ | H | $CH_3$ | B-7 | H | H | H | CN |
| 873 | A-6 | $CH_3$ | Cℓ | H | B-6 | H | H | H | CN |
| 874 | A-6 | $CH_3$ | Cℓ | H | B-7 | H | H | H | CN |
| 875 | A-6 | $CH_3$ | H | Cℓ | B-6 | H | H | H | CN |
| 876 | A-6 | $CH_3$ | H | Cℓ | B-7 | H | H | H | CN |
| 877 | A-6 | $CH_3$ | $CH_3$ | $CH_3$ | B-6 | H | H | H | CN |
| 878 | A-6 | $CH_3$ | $CH_3$ | $CH_3$ | B-7 | H | H | H | CN |
| 879 | A-7 | | H | H | B-6 | H | H | H | CN |
| 880 | A-7 | | H | H | B-7 | H | H | H | CN |
| 881 | A-7 | | $CH_3$ | H | B-6 | H | H | H | CN |
| 882 | A-7 | | $CH_3$ | H | B-7 | H | H | H | CN |
| 883 | A-7 | | H | $CH_3$ | B-6 | H | H | H | CN |
| 884 | A-7 | | H | $CH_3$ | B-7 | H | H | H | CN |
| 885 | A-7 | | Cℓ | H | B-6 | H | H | H | CN |
| 886 | A-7 | | Cℓ | H | B-7 | H | H | H | CN |
| 887 | A-7 | | H | Cℓ | B-6 | H | H | H | CN |
| 888 | A-7 | | H | Cℓ | B-7 | H | H | H | CN |
| 889 | A-7 | | $CH_3$ | $CH_3$ | B-6 | H | H | H | CN |
| 890 | A-7 | | $CH_3$ | $CH_3$ | B-7 | H | H | H | CN |
| 891 | A-7 | | H | $CH_3O$ | B-6 | H | H | H | CN |
| 892 | A-7 | | H | $CH_3O$ | B-7 | H | H | H | CN |

| Compound No. | A | $R_2$ | $R_3$ | B | $R_4$ | $R_5$ | $R_6$ | D |
|---|---|---|---|---|---|---|---|---|
| 893 | A−8 | H | H | B−6 | H | H | H | CN |
| 894 | A−8 | H | H | B−7 | H | H | H | CN |
| 895 | A−8 | $CH_3$ | H | B−6 | H | H | H | CN |
| 896 | A−8 | $CH_3$ | H | B−7 | H | H | H | CN |
| 897 | A−8 | H | $CH_3$ | B−6 | H | H | H | CN |
| 898 | A−8 | H | $CH_3$ | B−7 | H | H | H | CN |
| 899 | A−8 | Cℓ | H | B−6 | H | H | H | CN |
| 900 | A−8 | Cℓ | H | B−7 | H | H | H | CN |
| 901 | A−8 | H | Cℓ | B−6 | H | H | H | CN |
| 902 | A−8 | H | Cℓ | B−7 | H | H | H | CN |
| 903 | A−8 | $CH_3$ | $CH_3$ | B−6 | H | H | H | CN |
| 904 | A−8 | $CH_3$ | $CH_3$ | B−7 | H | H | H | CN |
| 905 | A−8 | H | F | B−6 | H | H | H | CN |
| 906 | A−8 | $CH_3$ | F | B−7 | H | H | H | CN |
| 907 | A−9 | H | H | B−6 | H | H | H | CN |
| 908 | A−9 | H | H | B−7 | H | H | H | CN |
| 909 | A−9 | $CH_3$ | H | B−6 | H | H | H | CN |
| 910 | A−9 | $CH_3$ | H | B−7 | H | H | H | CN |
| 911 | A−9 | H | $CH_3$ | B−6 | H | H | H | CN |
| 912 | A−9 | H | $CH_3$ | B−7 | H | H | H | CN |
| 913 | A−9 | Cℓ | H | B−6 | H | H | H | CN |
| 914 | A−9 | Cℓ | H | B−7 | H | H | H | CN |
| 915 | A−9 | H | Cℓ | B−6 | H | H | H | CN |
| 916 | A−9 | H | Cℓ | B−7 | H | H | H | CN |
| 917 | A−9 | $CH_3$ | $CH_3$ | B−6 | H | H | H | CN |
| 918 | A−9 | $CH_3$ | $CH_3$ | B−7 | H | H | H | CN |
| 919 | A−9 | Cℓ | Cℓ | B−6 | H | H | H | CN |
| 920 | A−9 | Cℓ | Cℓ | B−7 | H | H | H | CN |

| Compound No. | A | $R_2$ | $R_3$ | B | $R_4$ | $R_5$ | $R_6$ | D |
|---|---|---|---|---|---|---|---|---|
| 921 | A−10 | H | H | B−6 | H | H | H | CN |
| 922 | A−10 | H | H | B−7 | H | H | H | CN |
| 923 | A−10 | $CH_3$ | H | B−6 | H | H | H | CN |
| 924 | A−10 | $CH_3$ | H | B−7 | H | H | H | CN |
| 925 | A−10 | H | $CH_3$ | B−6 | H | H | H | CN |
| 926 | A−10 | H | $CH_3$ | B−7 | H | H | H | CN |
| 927 | A−10 | Cℓ | H | B−6 | H | H | H | CN |
| 928 | A−10 | Cℓ | H | B−7 | H | H | H | CN |
| 929 | A−10 | H | Cℓ | B−6 | H | H | H | CN |
| 930 | A−10 | H | Cℓ | B−7 | H | H | H | CN |
| 931 | A−10 | $CH_3$ | $CH_3$ | B−6 | H | H | H | CN |
| 932 | A−10 | $CH_3$ | $CH_3$ | B−7 | H | H | H | CN |
| 933 | A−10 | Cℓ | Cℓ | B−6 | H | H | H | CN |
| 934 | A−10 | Cℓ | Cℓ | B−7 | H | H | H | CN |
| 935 | A−11 | H | H | B−6 | H | H | H | CN |
| 936 | A−11 | H | H | B−7 | H | H | H | CN |
| 937 | A−11 | $CH_3$ | H | B−6 | H | H | H | CN |
| 938 | A−11 | $CH_3$ | H | B−7 | H | H | H | CN |
| 939 | A−11 | H | $CH_3$ | B−6 | H | H | H | CN |
| 940 | A−11 | H | $CH_3$ | B−7 | H | H | H | CN |
| 941 | A−11 | Cℓ | H | B−6 | H | H | H | CN |
| 942 | A−11 | Cℓ | H | B−7 | H | H | H | CN |
| 943 | A−11 | H | Cℓ | B−6 | H | H | H | CN |
| 944 | A−11 | H | Cℓ | B−7 | H | H | H | CN |
| 945 | A−11 | $CH_3$ | $CH_3$ | B−6 | H | H | H | CN |
| 946 | A−11 | $CH_3$ | $CH_3$ | B−7 | H | H | H | CN |
| 947 | A−11 | $CH_3O$ | H | B−6 | H | H | H | CN |
| 948 | A−11 | $CH_3O$ | H | B−7 | H | H | H | CN |

| Compound No. | A | $R_2$ | $R_3$ | B | $R_4$ | $R_5$ | $R_6$ | D |
|---|---|---|---|---|---|---|---|---|
| 949 | A−12 | H | H | B−6 | H | H | H | CN |
| 950 | A−12 | H | H | B−7 | H | H | H | CN |
| 951 | A−12 | $CH_3$ | H | B−6 | H | H | H | CN |
| 952 | A−12 | $CH_3$ | H | B−7 | H | H | H | CN |
| 953 | A−12 | H | $CH_3$ | B−6 | H | H | H | CN |
| 954 | A−12 | H | $CH_3$ | B−7 | H | H | H | CN |
| 955 | A−12 | Cℓ | H | B−6 | H | H | H | CN |
| 956 | A−12 | Cℓ | H | B−7 | H | H | H | CN |
| 957 | A−12 | H | Cℓ | B−6 | H | H | H | CN |
| 958 | A−12 | H | Cℓ | B−7 | H | H | H | CN |
| 959 | A−12 | $CH_3$ | $CH_3$ | B−6 | H | H | H | CN |
| 960 | A−12 | $CH_3$ | $CH_3$ | B−7 | H | H | H | CN |
| 961 | A−12 | Cℓ | $CF_3$ | B−6 | H | H | H | CN |
| 962 | A−12 | Cℓ | $CF_3$ | B−7 | H | H | H | CN |
| 963 | A−13 | H | H | B−6 | H | H | H | CN |
| 964 | A−13 | H | H | B−7 | H | H | H | CN |
| 965 | A−13 | $CH_3$ | H | B−6 | H | H | H | CN |
| 966 | A−13 | $CH_3$ | H | B−7 | H | H | H | CN |
| 967 | A−13 | H | $CH_3$ | B−6 | H | H | H | CN |
| 968 | A−13 | H | $CH_3$ | B−7 | H | H | H | CN |
| 969 | A−13 | Cℓ | H | B−6 | H | H | H | CN |
| 970 | A−13 | Cℓ | H | B−7 | H | H | H | CN |
| 971 | A−13 | H | Cℓ | B−6 | H | H | H | CN |
| 972 | A−13 | H | Cℓ | B−7 | H | H | H | CN |
| 973 | A−13 | $CH_3$ | $CH_3$ | B−6 | H | H | H | CN |
| 974 | A−13 | $CH_3$ | $CH_3$ | B−7 | H | H | H | CN |
| 975 | A−13 | Cℓ | Cℓ | B−6 | H | H | H | CN |
| 976 | A−13 | Cℓ | Cℓ | B−7 | H | H | H | CN |

| Compound No. | A | $R_2$ | $R_3$ | B | $R_4$ | $R_5$ | $R_6$ | D |
|---|---|---|---|---|---|---|---|---|
| 977 | A−14 | H | H | B−6 | H | H | H | CN |
| 978 | A−14 | H | H | B−7 | H | H | H | CN |
| 979 | A−14 | $CH_3$ | H | B−6 | H | H | H | CN |
| 980 | A−14 | $CH_3$ | H | B−7 | H | H | H | CN |
| 981 | A−14 | H | $CH_3$ | B−6 | H | H | H | CN |
| 982 | A−14 | H | $CH_3$ | B−7 | H | H | H | CN |
| 983 | A−14 | Cℓ | H | B−6 | H | H | H | CN |
| 984 | A−14 | Cℓ | H | B−7 | H | H | H | CN |
| 985 | A−14 | H | Cℓ | B−6 | H | H | H | CN |
| 986 | A−14 | H | Cℓ | B−7 | H | H | H | CN |
| 987 | A−14 | $CH_3$ | $CH_3$ | B−6 | H | H | H | CN |
| 988 | A−14 | $CH_3$ | $CH_3$ | B−7 | H | H | H | CN |
| 989 | A−14 | Cℓ | Cℓ | B−6 | H | H | H | CN |
| 990 | A−14 | Cℓ | Cℓ | B−7 | H | H | H | CN |
| 991 | A−15 | H | H | B−6 | H | H | H | CN |
| 992 | A−15 | H | H | B−7 | H | H | H | CN |
| 993 | A−15 | $CH_3$ | H | B−6 | H | H | H | CN |
| 994 | A−15 | $CH_3$ | H | B−7 | H | H | H | CN |
| 995 | A−15 | H | $CH_3$ | B−6 | H | H | H | CN |
| 996 | A−15 | H | $CH_3$ | B−7 | H | H | H | CN |
| 997 | A−15 | Cℓ | H | B−6 | H | H | H | CN |
| 998 | A−15 | Cℓ | H | B−7 | H | H | H | CN |
| 999 | A−15 | H | Cℓ | B−6 | H | H | H | CN |
| 1000 | A−15 | H | Cℓ | B−7 | H | H | H | CN |
| 1001 | A−15 | $CH_3$ | $CH_3$ | B−6 | H | H | H | CN |
| 1002 | A−15 | $CH_3$ | $CH_3$ | B−7 | H | H | H | CN |
| 1003 | A−15 | Cℓ | Cℓ | B−6 | H | H | H | CN |
| 1004 | A−15 | Cℓ | Cℓ | B−7 | H | H | H | CN |

| Compound No. | A | $R_2$ | $R_3$ | B | $R_4$ | $R_5$ | $R_6$ | D |
|---|---|---|---|---|---|---|---|---|
| 1005 | A-16 | H | H | B-6 | H | H | H | CN |
| 1006 | A-16 | H | H | B-7 | H | H | H | CN |
| 1007 | A-16 | $CH_3$ | H | B-6 | H | H | H | CN |
| 1008 | A-16 | $CH_3$ | H | B-7 | H | H | H | CN |
| 1009 | A-16 | H | $CH_3$ | B-6 | H | H | H | CN |
| 1010 | A-16 | H | $CH_3$ | B-7 | H | H | H | CN |
| 1011 | A-16 | Cℓ | H | B-6 | H | H | H | CN |
| 1012 | A-16 | Cℓ | H | B-7 | H | H | H | CN |
| 1013 | A-16 | H | Cℓ | B-6 | H | H | H | CN |
| 1014 | A-16 | H | Cℓ | B-7 | H | H | H | CN |
| 1015 | A-16 | $CH_3$ | $CH_3$ | B-6 | H | H | H | CN |
| 1016 | A-16 | $CH_3$ | $CH_3$ | B-7 | H | H | H | CN |
| 1017 | A-16 | Cℓ | Cℓ | B-6 | H | H | H | CN |
| 1018 | A-16 | Cℓ | Cℓ | B-7 | H | H | H | CN |
| 1019 | A-17 | H | H | B-6 | H | H | H | CN |
| 1020 | A-17 | H | H | B-7 | H | H | H | CN |
| 1021 | A-17 | $CH_3$ | H | B-6 | H | H | H | CN |
| 1022 | A-17 | $CH_3$ | H | B-7 | H | H | H | CN |
| 1023 | A-17 | H | $CH_3$ | B-6 | H | H | H | CN |
| 1024 | A-17 | H | $CH_3$ | B-7 | H | H | H | CN |
| 1025 | A-17 | Cℓ | H | B-6 | H | H | H | CN |
| 1026 | A-17 | Cℓ | H | B-7 | H | H | H | CN |
| 1027 | A-17 | H | Cℓ | B-6 | H | H | H | CN |
| 1028 | A-17 | H | Cℓ | B-7 | H | H | H | CN |
| 1029 | A-17 | $CH_3$ | $CH_3$ | B-6 | H | H | H | CN |
| 1030 | A-17 | $CH_3$ | $CH_3$ | B-7 | H | H | H | CN |
| 1031 | A-17 | Cℓ | Cℓ | B-6 | H | H | H | CN |
| 1032 | A-17 | Cℓ | Cℓ | B-7 | H | H | H | CN |

| Compound No. | A | $R_2$ | $R_3$ | B | $R_4$ | $R_5$ | $R_6$ | D |
|---|---|---|---|---|---|---|---|---|
| 1033 | A−18 | H | H | B−6 | H | H | H | CN |
| 1034 | A−18 | H | H | B−7 | H | H | H | CN |
| 1035 | A−18 | $CH_3$ | H | B−6 | H | H | H | CN |
| 1036 | A−18 | $CH_3$ | H | B−7 | H | H | H | CN |
| 1037 | A−18 | H | $CH_3$ | B−6 | H | H | H | CN |
| 1038 | A−18 | H | $CH_3$ | B−7 | H | H | H | CN |
| 1039 | A−18 | Cℓ | H | B−6 | H | H | H | CN |
| 1040 | A−18 | Cℓ | H | B−7 | H | H | H | CN |
| 1041 | A−18 | H | Cℓ | B−6 | H | H | H | CN |
| 1042 | A−18 | H | Cℓ | B−7 | H | H | H | CN |
| 1043 | A−18 | $CH_3$ | $CH_3$ | B−6 | H | H | H | CN |
| 1044 | A−18 | $CH_3$ | $CH_3$ | B−7 | H | H | H | CN |
| 1045 | A−18 | Cℓ | Cℓ | B−6 | H | H | H | CN |
| 1046 | A−18 | Cℓ | Cℓ | B−7 | H | H | H | CN |

Table 4

A−CONHCH−D
　　　　　|
　　　　　B

| Compound No. | A | $R_1$ | $R_2$ | $R_3$ | B | D |
|---|---|---|---|---|---|---|
| 1047 | A−2 | $CH_3$ | H | H | $SCH_2CH=CH_2$ | CN |
| 1048 | A−2 | $CH_3$ | H | H | $SCH_2CH=CH_2$ | $CSNH_2$ |
| 1049 | A−2 | $CH_3$ | H | H | $SCH_2CH=CHCH_3$ | CN |
| 1050 | A−2 | $CH_3$ | H | H | $SCH_2C(C\ell)=CH_2$ | CN |
| 1051 | A−2 | $CH_3$ | H | H | $SCH_2CH=CHC\ell$ | CN |
| 1052 | A−2 | $CH_3$ | H | H | $SCH_2-C(CH_3)=CH_2$ | CN |
| 1053 | A−3 | $CH_3$ | H | H | $SCH_2CH=CH_2$ | CN |
| 1054 | A−3 | $CH_3$ | H | H | $SCH_2CH=CH_2$ | $CSNH_2$ |
| 1055 | A−3 | $CH_3$ | H | H | $SCH_2CH=CHCH_3$ | CN |
| 1056 | A−3 | $CH_3$ | H | H | $SCH_2C(C\ell)=CH_2$ | CN |
| 1057 | A−3 | $CH_3$ | H | H | $SCH_2CH=CHC\ell$ | CN |
| 1058 | A−3 | $CH_3$ | H | H | $SCH_2-C(CH_3)=CH_2$ | CN |
| 1059 | A−3 | $CH_3$ | $CH_3$ | H | $SCH_2CH=CH_2$ | CN |
| 1060 | A−3 | $CH_3$ | $CH_3$ | H | $SCH_2CH=CH_2$ | $CSNH_2$ |
| 1061 | A−3 | $CH_3$ | $CH_3$ | H | $SCH_2CH=CHCH_3$ | CN |
| 1062 | A−3 | $CH_3$ | $CH_3$ | H | $SCH_2C(C\ell)=CH_2$ | CN |
| 1063 | A−3 | $CH_3$ | $CH_3$ | H | $SCH_2CH=CHC\ell$ | CN |
| 1064 | A−3 | $CH_3$ | $CH_3$ | H | $SCH_2-C(CH_3)=CH_2$ | CN |
| 1065 | A−3 | $CH_3$ | $C\ell$ | H | $CH_2CH=CH_2$ | CN |
| 1066 | A−3 | $CH_3$ | $C\ell$ | H | $CH_2CH=CH_2$ | $CSNH_2$ |
| 1067 | A−3 | $CH_3$ | $C\ell$ | H | $SCH_2CH=CHCH_3$ | CN |
| 1068 | A−3 | $CH_3$ | $C\ell$ | H | $SCH_2C(C\ell)=CH_2$ | CN |
| 1069 | A−3 | $CH_3$ | $C\ell$ | H | $SCH_2CH_2=CHC\ell$ | CN |
| 1070 | A−3 | $CH_3$ | $C\ell$ | H | $SCH_2-C(CH_3)=CH_2$ | CN |
| 1071 | A−3 | $CH_3$ | $CF_3$ | H | $SCH_2CH=CH_2$ | CN |
| 1072 | A−3 | $CH_3$ | H | $CH_3$ | $SCH_2CH=CH_2$ | CN |
| 1073 | A−3 | $CH_3$ | H | $C\ell$ | $SCH_2CH=CH_2$ | CN |
| 1074 | A−3 | $CH_3$ | $CH_3$ | $CH_3$ | $SCH_2CH=CH_2$ | CN |

A-1, A-18 and B-1 , B-7 shown in the above-mentioned Tables 1 - 4 are groups represented by the following formulae.

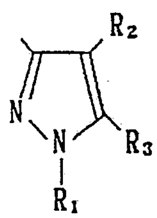
A-1 ,

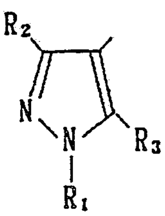
A-2 ,

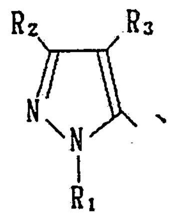
A-3 ,

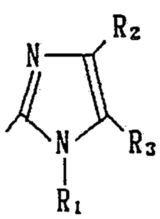
A-4 ,

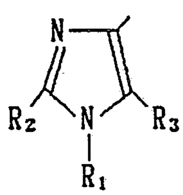
A-5 ,

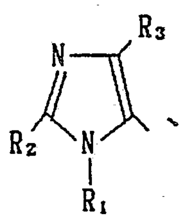
A-6 ,

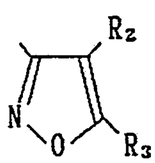
A-7 ,

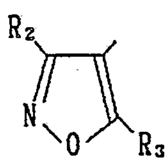
A-8 ,

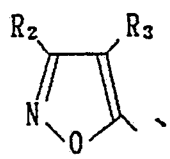
A-9 .

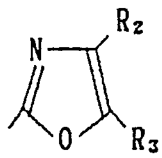 , 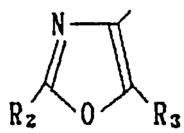 , 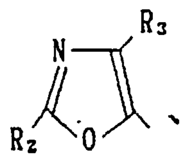 ,

A-10 A-11 A-12

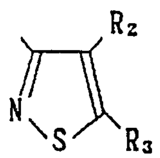 , 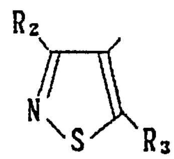 , 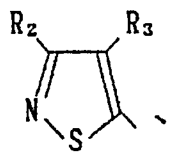 ,

A-13 A-14 A-15

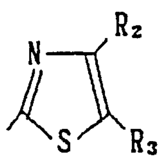 , 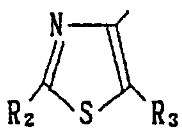 , 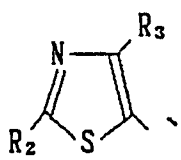 ,

A-16 A-17 A-18

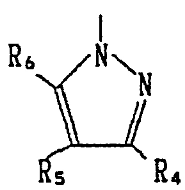 , 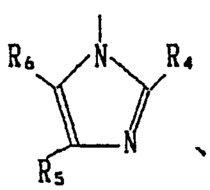 , 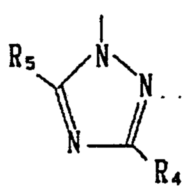 ,

B-1 B-2 B-3

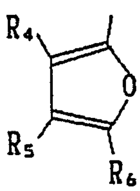 , 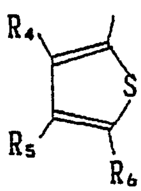 , 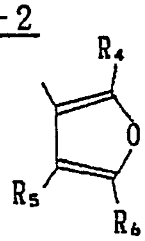 , 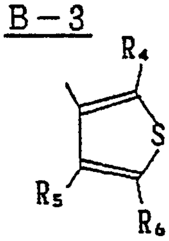

B-4 B-5 B-6 B-7

The data of H-NMR and the melting points of some compounds described in Tables 1 - 4 are shown in Table 5.

Table 5

| Compound No. | Solvent | δ ppm (Standard TMS) | M.P. (℃) |
|---|---|---|---|
| 2 | $CDCl_3$ + DMSO d-6 | 6.20~6.75(m,5H), 7.58(s,1H), 7.70(s,1H), 9.35(d,1H,J=7.2Hz) | 159~160 |
| 7 | $CDCl_3$ DMSO d-6 | 3.92(s,3H), 6.36(d,1H,J=7.8Hz), 6.30~6.80(m,2H), 6.72(d,1H,J=2.4Hz), 7.58(m,1H), 7.66(d,1H,J=2.4Hz), 9.23(d,1H,J=7.8Hz) | 104~106 |
| 8 | $CDCl_3$ DMSO d-6 | 3.91(s,3H), 6.48(d,1H,J=9.0Hz), 6.87(d,1H,J=2.4Hz), 6.90~7.70(m,3H), 7.56(d,1H,J=2.4Hz), 9.12(d,1H,J=9.0Hz) | 123~125 |
| 12 | $CDCl_3$ DMSO d-6 | 3.59(s,3H), 3.85(s,3H), 6.01(d, 1H, J=7.8Hz), 6.10~6.60(m,2H), 7.45(m,1H), 8.61(d,1H,J=7.8Hz) | 100.5~101.5 |
| 17 | $CDCl_3$ DMSO d-6 | 6.13(d,2H,J=51.6Hz), 6.27(m,1H), 7.51(d,1H,J=8.4Hz), 7.57(m,1H), 7.86(m,1H), 8.16(m,1H), 8.67(m,1H), 10.32(d,1H,J=8.4Hz) | 144~146 |
| 18 | $CDCl_3$ DMSO d-6 | 2.20(s,3H), 3.83(s,3H), 6.04(d,1H, J=2.4Hz), 7.33(d,1H,J=8.4Hz), 7.69(d,1H,J=2.4Hz), 7.91(s,1H), 8.18(s,1H), 10.02(d,1H,J=8.4Hz) | 176~181 |

| Compound No. | Solvent | δ ppm (Standard TMS) | M.P. (℃) |
|---|---|---|---|
| 20 | $CDCl_3$ DMSO d-6 | 2.10(s,3H), 2.34(s,3H), 3.83(s,3H), 5.80(s,1H), 6.73(d,1H,J=8.4Hz), 7.98(s,1H), 8.33(s,1H), 9.34(d,1H,J=8.4Hz) | 145~149 |
| 29 | $CDCl_3$ DMSO d-6 | 3.80(s,3H), 6.30(m,1H), 7.48(d, 1H, J=9.0Hz), 7.56(m,1H), 7.90(m,1H), 8.18(s,1H), 10.18(d,1H,J=9.0Hz) | 174~181 |
| 33 | $CDCl_3$ +DMSO d-6 | 1.36(t,3H,J=7.2Hz), 4.53(q,2H,J=7.2Hz), 6.32(d,1H,J=8.4Hz), 6.30~6.65(m,2H), 6.91(d,1H,J=1.8Hz), 7.33(d,1H,J=1.8Hz), 7.49(m,1H), 9.51(d,1H,J=8.4Hz). | 131~134 |
| 36 | $CDCl_3$ DMSO d-6 | 3.84(s,3H), 6.18(d,1H,J=8.4Hz), 6.80~7.50(m,3H), 7.87(s,1H), 8.22(s,1H), 8.26(d,1H,J=8.4Hz), 9.55(bs,1H), 9.87(bs,1H) | 200~203 |

| Compound No. | Solvent | δ ppm (Standard TMS) | M.P. (℃) |
|---|---|---|---|
| 40 | $CDCl_3$ DMSO d-6 | 3.82(s,3H), 6.28(d,1H,J=7.8Hz), 6.30～6.75(m,2H), 7.55(m,1H), 8.91(d,1H,J=7.8Hz) | 159～161 |
| 46 | $CDCl_3$ | 4.12(s,3H), 6.30～6.40(m,1H), 6.80(d,1H,J=3Hz), 7.38(d,1H,J=9Hz), 7.42(d,1H,J=3Hz), 7.60～7.70(m,1H), 7.81(d,1H,J=3Hz), 9.35(d,1H,J=9Hz) | 169～171 |
| 47 | $CD_3OD$ | 3.80(s,3H), 6.00(m,1H), 6.80(m,2H), 7.20(m,2H), 7.67～8.00(m,2H), | 128～129 |
| 53 | $CDCl_3$ DMSO d-6 | 4.08(s,3H), 6.25(d,1H,J=7.2Hz), 6.25～6.60(m,2H), 6.77(d,1H,J=1.8Hz), 7.26(d,1H,J=1.8Hz), 7.39(m,1H), 9.40(d,1H,J=7.2Hz) | 101～104 |

| Compound No. | Solvent | δ ppm (Standard TMS) | M.P. (℃) |
|---|---|---|---|
| 57 | $CDCl_3$<br>DMSO d-6 | 1.34(t,3H,J=7.2Hz), 4.51(q,2H,J=7.2Hz),<br>6.30(d,1H,J=7.8Hz), 6.25~6.60(m,2H),<br>6.88(d,1H,J=2.4Hz), 7.29(d,1H,J=2.4Hz),<br>7.46(m,1H), 9.49(d,1H,J=7.8Hz) | 130~132 |
| 58 | $CDCl_3$<br>DMSO d-6 | 1.39(t,3H,J=7.8Hz), 4.56(q,2H,J=7.8Hz),<br>6.44(d,1H,J=8.4Hz), 6.85~7.15(m,2H),<br>7.15~7.60(m,3H), 9.63(d,1H,J=8.4Hz) | 114~116 |
| 59 | $CDCl_3$ | 2.18(s,3H), 4.07(s,3H), 6.20~6.60(m,2H),<br>7.25~8.00(m,3H), 9.17(d,1H,J=9Hz) | 143~144 |
| 80 | $CDCl_3$ | 2.21(s,3H), 4.06(s,3H), 6.15~6.62(m,4H),<br>6.90~7.50(m,2H) | 102~103 |

| Compound No. | Solvent | δ ppm (Standard TMS) | M.P. (℃) |
|---|---|---|---|
| 91 | $CDC\ell_3$<br>DMSO d-6 | 2.20(s,3H), 4.04(s,3H), 6.45(d,1H,J=9.0Hz), 6.71(s,1H), 6.85~7.15(m,1H), 7.15~7.55 (m,2H), 9.65(d,1H,J=9.0Hz) | oil |
| 169 | $CDC\ell_3$<br>DMSO d-6 | 1.26(t,3H,J=7.2Hz), 2.64(q,2H,J=7.2Hz), 3.79(s,3H), 6.27(d,1H,J=7.8Hz), 6.30~6.70(m,2H), 6.53(s,1H), 7.52(m,1H), 8.87(d,1H,J=7.8Hz) | 95~96 |
| 183 | $CDC\ell_3$ | 3.76(s,3H), 3.94(s,3H), 6.10(s,1H), 6.23(d,1H,J=7.8Hz), 6.25~6.65(m,2H), 7.39(m,1H), 7.94(d,1H,J=7.8Hz) | oil |
| 184 | $CDC\ell_3$ | 3.77(s,3H), 3.94(s,3H), 6.01(s,1H), 6.34(d,1H,J=8.4Hz), 6.80~7.15(m,1H), 7.15~7.60(m,2H), 7.67(d,1H,J=8.4Hz) | oil |

| Compound No. | Solvent | δ ppm (Standard TMS) | M.P. (℃) |
|---|---|---|---|
| 195 | CDCℓ₃ | 4.13(s,3H), 6.24(d,1H,J=8.4Hz), 6.32~6.67(m,2H), 6.78(s,1H), 6.98(d,1H,J=8.4Hz), 7.12~7.77(m,5H) | 162~163 |
| 237 | CDCℓ₃ DMSO d-6 | 2.30(s,3H), 3.73(s,3H), 6.26(d,1H, J=8.4Hz), 6.30~6.70(m,2H), 7.51(s,1H), 7.59(m,1H), 9.12(d,1H,J=8.4Hz) | 96~102 |
| 245 | CDCℓ₃ DMSO d-6 | 2.44(s,3H), 6.24(d,1H,J=7.8Hz), 6.25~6.60(m,2H), 7.35(s,1H), 7.45(m,1H), 8.63(d,1H,J=7.8Hz), 12.33(bs,1H) | 148~152 |
| 277 | CDCℓ₃ | 2.42(s,3H), 6.01~6.59(m,4H), 7.29(bs,1H), 7.75(d,1H,J=7.8Hz) | 106~108 |
| 372 | CDCℓ₃ DMSO d-6 | 2.42(s,3H), 6.41(d,1H,J=8.4Hz), 6.35~6.70(m,2H), 7.65(m,1H), 8.32(s,1H), 9.73(d,1H,J=8.4Hz) | 143~147 |
| 488 | CDCℓ₃ +DMSO d-6 | 1.21(t,3H,J=7.2Hz), 3.67(q,2H,J=7.2Hz), 3.87(s,3H), 6.12(d,1H,J=9Hz), 7.94(s,1H), 8.15(s,1H), 9.55(d,1H,J=9Hz), | 143~145.5 |
| 498 | CDCℓ₃ | 1.15(t,3H,J=7.0Hz), 3.63(q,2H,J=7.0Hz), 4.07(s,3H), 6.08(d,1H,J=9.0Hz), 6.71(d,1H,J=2.0Hz), 7.39(d,1H,J=2.0Hz), 8.47(d,1H,J=9.0Hz) | 61~63 |

| | | | |
|---|---|---|---|
| 722 | $CDCl_3$ + DMSO d-6 | 3.87(s,3H), 6.13(d,1H,J=8.4Hz), 6.57(m,1H), 6.67(d,1H,J=2.4Hz), 7.47(m,1H), 7.54(d,1H,J=2.4Hz), 7.67(m,1H), 9.01(d,1H,J=8.4Hz) | 130~132.5 |
| 723 | $CDCl_3$ + DMSO d-6 | 3.90(s,3H), 6.28(d,1H,J=9.0Hz), 6.74(d,1H,J=2.4Hz), 7.05~7.70 (m,3H), 7.45(d,1H,J=2.4Hz), 8.63(d,1H,J=9.0Hz) | 146.5 ~148 |
| 726 | $CDCl_3$ + DMSO d-6 | 2.27(s,3H), 3.24(s,3H), 6.05(d, 1H,J=7.8Hz), 6.30 ~6.60(m,2H), 7.37(m,1H), 7.54(m,1H), 8.55(d,1H,J=7.8Hz) | 128 ~130 |
| 727 | $CDCl_3$ + DMSO d-6 | 2.28(s,3H), 3.77(s,3H), 6.26(d, 1H,J=8.4Hz), 6.49(s,1H), 7.05~7.65(m,3H), 8.88(d,1H, J=8.4Hz) | oil |

| | | | |
|---|---|---|---|
| 732 | $CDC\ell_3$ + DMSO d-6 | 3.61(s,3H), 3.87(s,3H), 6.03(s,1H), 6.09(d,1H,J=8.4Hz), 6.50(m,1H), 7.40(m,1H), 7.61(m,1H), 8.32(d,1H,J=8.4Hz) | 126~128 |
| 733 | $CDC\ell_3$ + DMSO d-6 | 3.58(s,1H), 3.84(s,3H), 6.02(s,1H), 6.22(d,1H,J=8.4Hz), 7.00~7.65(m,3H), 8.71(d,1H, J=8.4Hz) | 128~131 |
| 736 | DMSO d-6 | 3.73(s,3H), 6.05(d,1H,J=7.8Hz) 6.46(s,1H), 7.41~7.91(m,3H), 8.06(s,1H), 9.02(d,1H,J=7.8Hz) | 159~160 |
| 738 | $CDC\ell_3$ + DMSO d-6 | 3.89(s,3H), 6.31(d,1H,J=8.4Hz), 7.00~7.65(m,3H), 7.92(s,1H), 8.11(s,1H), 8.19(d,1H,J=8.4Hz) | 166~168 |

| | | | |
|---|---|---|---|
| 739 | $CDC\ell_3$ + DMSO d-6 | 3.85(s,3H), 6.02(d,1H,J=8.4Hz), 7.05~7.60(m,3H), 7.85(s,1H), 8.18(s,1H), 8.75(d,1H,J=8.4Hz), 9.44(bs,1H), 9.71(bs,1H) | 224~227 |
| 745 | $CDC\ell_3$ + DMSO d-6 | 1.45(t,3H,J=7.2Hz), 4.12(q,2H, J=7.2Hz), 6.28(d,1H,J=7.8Hz), 6.95~7.65(m,3H), 7.92(s,1H), 8.06(s,1H), 9.04(d,1H,J=7.8Hz) | 126~129 |
| 753 | $CDC\ell_3$ + DMSO d-6 | 3.92(s,3H), 6.24(d,1H,J=7.8Hz), 7.05~7.70(m,3H), 8.34(s,1H), 9.27(d,1H,J=7.8Hz) | 149~153 |
| 762 | $CDC\ell_3$ | 1.90(s,3H), 3.75(s,3H), 5.94(d, 1H,J=7.8Hz), 6.12 ~7.52(m,4H), 8.28(d,1H,J=7.8Hz) | 40~45 |

| | | | |
|---|---|---|---|
| 763 | DMSO d-6 | 2.05(s,3H), 3.83(s,3H), 5.70(d,1H, J=7.8Hz), 6.50(s,1H), 6.57(s,1H), 7.42~7.71(m,2H), 8.35(d,1H, J=7.8Hz),9.37(bs,1H), 9.70(bs,1H) | 41~43 |
| 766 | $CDCl_3$ + DMSO d-6 | 2.23(s,3H), 4.03(s,3H), 6.05(d,1H,J=7.8Hz), 6.51(s,1H), 7.18~7.53(m,3H), 8.10(d,1H, J=7,8Hz), 9.18(s,2H) | 170~171 |
| 770 | $CDCl_3$ | 1.42(t,3H,J=7.2Hz), 2.23(s,3H), 4.51(q,2H,J=7.2Hz), 6.10(d,1H, J=7.8Hz), 6.34(s,1H), 6.48(s,1H), 6.81(d,1H,J=7.8Hz), 7.16~7.76(m,3H) | 103~106 |
| 771 | $CDCl_3$ | 1.36(t,3H,J=7.8Hz), 2.18(s,3H), 4.45(q,2H,J=7.8Hz), 6.22(d,1H, J=7.8Hz), 6.34(s,1H), 6.97~7.57(m,4H) | 118~120 |

| | | | |
|---|---|---|---|
| 790 | $CDC\ell_3$ | 4.12(s,3H), 6.12(d,1H,J=7.5Hz), 6.79(s,1H), 6.85~7.56(m,4H) | 134.5~136.5 |
| 812 | $CDC\ell_3$ + DMSO d-6 | 1.26(t,3H,J=7.2Hz), 2.62(q,2H, J=7.2Hz), 3.76(s,3H), 6.14(d,1H, J=7.8Hz), 6.53(m,2H), 7.44(m,1H), 7.64(m,1H), 8.42(d,1H,J=7.8Hz) | 94~95 |
| 813 | $CDC\ell_3$ + DMSO d-6 | 1.25(t,3H,J=7.2Hz), 2.62(q,2H, J=7.2Hz), 3.78(s,3H), 6.30(d,1H, J=8.4Hz), 6.55(s,1H), 7.05~7.65 (m,3H), 8.89(d,1H,J=8.4Hz) | 132~134 |
| 814 | $CDC\ell_3$ | 3.78(s,3H), 3.94(s,3H), 5.85(s,1H), 5.96(d,1H,J=9.0Hz), 6.35(m,1H), 6.85(d,1H,J=9.0Hz), 7.32(m,1H), 7.48(m,1H) | oil |

| | | | |
|---|---|---|---|
| 815 | $CDC\ell_3$ | 3.72(s,3H), 3.87(s,3H), 5.86(s,1H), 6.08(d,1H,J=8.4Hz), 6.80~7.50(m,4H) | oil |
| 820 | $CDC\ell_3$ | 2.25(s,3H), 6.10(d,1H,J=8.4Hz), 6.35(s,1H), 6.50(s,1H), 7.05~7.67(m,3H), 8.93(d,1H,J=8.4Hz) | 147~149 |
| 821 | $CDC\ell_3$ | 2.25(s,3H), 6.27(d,1H,J=8.4Hz), 6.54(s,1H), 6.94~7.57(m,3H), 8.14(d,1H,J=8.4Hz) | 40~42 |
| 823 | $CDC\ell_3$ + DMSO d-6 | 1.24(s,9H), 4.03(s,3H), 6.20(d,1H, J=7.8Hz), 6.73(s,1H), 7.00~7.55 (m,3H), 9.24(d,1H,J=7.8Hz), | 184~186 |

| | | | |
|---|---|---|---|
| 824 | DMSO d-6 | 1.20(t,3H,J=7.2Hz), 2.10(s,3H), 4.31(q,2H,J=7.2Hz), 5.74(d,1H, J=8.4Hz), 6.52(s,1H), 6.62(s,1H), 7.44~7.81(m,2H), 8.38(d,1H,J=8.4Hz), 9.38(bs,1H), 9.70(bs,1H) | 138~140 |
| 825 | CDC$\ell_3$ + DMSO d-6 | 1.35(t,3H,J=7.2Hz), 2.24(s,3H), 4.45(q,2H,J=7.2Hz), 6.03(d,1H, J=7.8Hz), 6.52(s,1H), 7.08~7.53 (m,3H), 8.15(d,1H,J=7.8Hz), 9.35(s,2H) | 162~163 |
| 827 | CDC$\ell_3$ + DMSO d-6 | 1.37(t,3H,J=7.2Hz), 4.52(q,2H, J=7.2Hz), 6.23(d,1H,J=7.8Hz), 6.85(d,1H,J=2.4Hz), 7.00~7.55 (m,3H), 7.30(d,1H,J=2.4Hz), 9.39(d,1H,J=7.8Hz) | 104~105 |

| | | | |
|---|---|---|---|
| 883 | $CDCl_3$ | 2.43(s,3H), 6.09(d,1H,J=7.8Hz), 6.38(br,s,2H), 7.21 ~7.45(m,1H), 7.52(br,s,1H), 7.72(d,1H,J=7.8Hz) | 92~94 |
| 884 | $CDCl_3$ | 2.42(s,3H), 6.19(d,1H,J=8.0Hz), 6.45(s,1H), 6.81 ~7.49(m,3H), 7.73(d,1H,J=8.0Hz) | 104~105 |
| 898 | $CDCl_3$ | 2.61(s,3H), 6.08(d,1H,J=7.8Hz), 6.81~7.57(m,4H), 8.25(s,1H) | oil |
| 910 | $CDCl_3$ + DMSO d-6 | 2.29(s,3H), 6.70(br,d,1H,J=4.2Hz), 6.76(s,1H), 6.96~7.60(m,3H), 7.85(br,s,1H) | 149~151 |

In addition, the present invention further provides a fungicide for agricultural and horticultural use which is characterized by containing the amido-substituted derivative as represented by the above-mentioned general formula (I) of the present invention, as an active ingredient.

The fungicide for agricultural and horticultural use of the present invention is effective not only against Pseudoperonospora-caused diseases and Phytophthora-caused diseases of various crops but also against other various fungous diseases of various crops. As typical fungous diseases against which the fungicide of the present invention is effective, there may be mentioned, for example, Pseudoperonospora cubensis, Plasmopara viticola, Bremia lacducae, Peronospora brassicae, Psedoperonospora humuli, Phytophthora infestans, Phytophthora capsici (cucumber and green pepper), tomato, cucumber and rice plant damping-off diseases caused by Pythium fungi as well as beet plant damping-off diseases caused by Aphanomyces fungi.

For application of the fungicide of the present invention to crops, the fungicide can be applied to crops by means of seed treatment, foliage treatment and soil treatment. The amount of the fungicide and the concentration thereof to be actually applied to crops vary, in accordance with the crops to be treated, the diseases to be protected, the degree of the occurrence of the diseases, the method of the application of the fungicide, etc. Accordingly, when the fungicide is to be applied over crops, the amount of the active ingredient may be from 2 to 2000 g/ha, preferably from 10 to 1000 g/ha.

The concentration of the fungicide to be applied may be from 1 to 1000 ppm, preferably from 5 to 500 ppm.

Since the fungicide for agricultural and horticultural use of the present invention have both preventive effect and curative effect, this may be applied over crops either for prevention of diseases before the crops are infected or for cure of the diseases of crops after the crops have been infected, and therefore, the proper time for applied the fungicide of the present invention may be wide and broad.

The fungicide of the present invention can be used, if desired, in combination with other biological active compounds, for example, agricultural chemicals such as the similar or complementary fungicides, or insecticides, herbicides, plant growth regulators, etc., and fertilizer substances, soil improving agents and

the like. It is a matter of course that the fungicide of the present invention can be formed into a preparation together with the said biological active compounds, if desired.

The phytopathogenic fungicide of the present invention can be used in combination with a suitable carrier, for example, a solid carrier such as clay, talc, bentonite, diatomaceous earth, etc., or a liquid carrier such as water, alcohols (e.g., methanol, ethanol, etc.), aromatic hydrocarbons (e.g., benzene, toluene, xylene, etc.), chlorinated hydrocarbons, ethers, ketones, esters (e.g., ethyl acetate, etc.), acid amides (e.g., dimethylformamide, etc.), etc., and if desired, an emulsifier, a dispersing agent, a suspension promoter, a permeation promoter, a spreader, a stabilizer, etc. can be added to form preparations in the form of an emulsifiable concentrations, oil solutions, wettable powders, dusts,granulars, flowables, etc. for practical use.

Examples of formulations of compositions of the phytopathogenic fungicide of the present invention are mentioned hereunder, in which the kinds of the ingredients and the amounts thereof are concretely described. Needless to say, these examples do not whatsoever limit the scope of the present invention. The "parts" means "parts by weight" in the formulations, unless otherwise specifically stated.

(1) Wettable Powders: The compound of the invention 5 to 75 parts

| | |
|---|---|
| Solid carrier | 9 to 86 parts |
| Surfactant | 5 to 10 parts |
| Others | 1 to 5 parts |

As the solid carrier, there may be mentioned calcium carbonate, kaolinite, Zeeklite A, Zeeklite PFP, diatomaceous earth, talc, etc.

As the surfactant, there may be mentioned
Lunox 1000C, Sorpol 5039, Sorpol 5050, Sorpol 005D, Sorpol 5029-O, calcium sulfonate, sodium dodecylsulfonate, etc.

Other components include Carplex #80, etc.

(2) Emulsifiable concentrate: The compound of the invention 5 to 50 parts

| | |
|---|---|
| Liquid carrier | 35 to 90 parts |
| Surfactant | 5 to 15 parts |

As the liquid carrier, there may be mentioned
xylene, dimethylformamide, methylnaphthalene, isophorone, etc.

As the surfactant, there may be mentioned
Sorpol 2680, Sorpol 3005X, Sorpol 3346, etc.

(3) Flowables: The compound of the invention 5 to 75 parts

| | |
|---|---|
| Liquid carrier | 14.5 to 68 parts |
| Surfactant | 5 to 10 parts |
| Others | 5 to 10 parts |

As the liquid carrier, water can be used.

As the surfactant, there may be mentioned
Lunox 1000C, Sorpol 3353, Sorpol FL, Nippol , Agrisol S-710, sodium ligninsulfonate, etc.

Others include ethylene glycol, propyelne glycol, Xanthan gum, etc.

The above ingredients were uniformly blended and powdered to form a wettable powders. For the practical use, the wettable powders are diluted to form a 1/1,500 to 1/150,000 solution, and this is sprayed in an amount of from 10 to 100 g/ha, as the active ingredient.

Formulation example 4: Emulsifiable concentrate The compound of the invention 5 parts
Xylene 70 parts
N,N-dimethylformamide 20 parts
Sorpol 2680 5 parts
(Mixture of non-ionic surfactant and anionic surfactant, trade name by Toho Chemical Industrial Co.)

The above ingredients were uniformly blended to form an emulsifiable concentrate. For the practical use, the emulsifiable concentrate is diluted into a 1/100 to 1/10,000 emulsion, and this is sprayed in an amount of from 10 to 1000 g/ha, as the active ingredient.

Formulation example 5: Emulsifiable concentrate The compound of the invention 50 parts
Xylene 25 parts
N,N-dimethylformamide 10 parts
Sorpol 3346 15 parts
(Mixture of non-ionic surfactant and anionic surfactant, trade name by Toho Chemical Industrial Co.)

The above ingredients were uniformly mixed to form an emulsifiable concentrate. For the practical use, the emulsion concentrate is diluted into a 1/1,000 to 1/100,000 emulsion, and this is applied in an amount of from 10 to 1000 g/ha, as the active ingredient.

Formulation example 6: Flowables The compound of the invention 5 parts
Sorpol 3353 5 parts
(Non-ionic surfactant, trade name by Toho Chemical Industrial Co.)
Lunox 1000C 3 parts
(Anionic surfactant, trade name by Toho Chemical Industrial Co.)
1% Aqueous solution of Xanthan gum 20 parts
(Natural high molecular substance)
Water 57 parts
Ethylene glycol 10 parts

The above-mentioned ingredients except the active ingredient (the compound of the invention) were uniformly dissolved, and then the compound of the invention was added thereto and well stirred, and thereafter the resulting mixture was wet-milled in a sand mill, to obtain flowables For the practical use, the flowables are diluted into a 1/100 to 1/10,000 solution, and this is applied in an amount of from 10 to 1000 g/ha, as the active ingredient.

Formulation example 7: Flowables The compound of the invention 75 parts
Sorpol 3353 5 parts
(Non-ionic surfactant, trade name by Toho Chemical Industrial Co.)
Lunox 1000C 0.5 parts
(Anionic surfactant, trade name by Toho Chemical Industrial Co.)
1% Aqueous solution of Xanthan gum 10 parts
(Natural high molecular substance)
Water 4.5 parts
Propylene glycol 5 parts

(4) Dusts: The compound of the invention 0.03 to 3 parts
Solid carrier 94 to 98.97 parts
Others 1 to 3 parts

As the solid carrier, there may be mentioned calcium carbonate, kaolinite, Zeeklite , talc, etc.

Others include diisopropyl phosphate, Carplex #80, etc.

(5) Granulars: The compound of the invention 0.3 to 10 parts
Solid carrier 92 to 98.7 parts
Others 1 to 5 parts

As the solid carrier, there may be mentioned calcium carbonate, kalinite, bentonite, talc, etc.

Others include calcium ligninsulfonate, polyvinyl alcohol, etc.

Next, concrete examples of formulations of fungicides containing the substituted-amido derivative of the formula (I) of the present invention are mentioned hereunder, which, however, are not intended to limit the scope of the present invention. In the formulations, "parts" means "parts by weight", unless otherwise specifically defined.

Formulation example 1: Wettable Powders The compound of the invention 5 parts
Zeeklite PFP 85 parts
(Mixture of kaolinite and sericite, trade name by Zeeklite Co., Ltd. )
Sorpol 5039 5 parts
(Mixture of anionic surfactant and white carbon, trade name by Toho Chemical Industrial Co.)
Carplex #80 3 parts
(White carbon, trade name by Shionogi & Co.)

The above ingredients were uniformly blended and powdered to form wettable powders. For the practical use, the wettable powders are diluted to form a 1/100 to 1/10,000 solution, and this is applied in an amount of from 10 to 1000 g/ha, as the active ingredient.

Formulation example 2: Wettable Powders The compound of the invention 25 parts
Zeeklite PFP 69 parts
(Mixture of kaolinite and sericite, trade name by Zeeklite Co. Ltd. )
Sorpol 5039 3 parts
(Mixture of anionic surfactant and white carbon, trade name by Toho Chemical Industrial Co.)
Carplex #80 3 parts
(White carbon, trade name by Shionogi & Co.)

The above ingredients were uniformly blended and powdered to form wettable powders . For the practical use, the wettable powders are diluted to form a 1/500 to 1/50,000 solution, and this is sprayed in an amount of from 10 to 1000 g/ha, as the active ingredient.

Formulation example 3: Wettable Powders The compound of the invention 75 parts
Calcium carbonate (powder) 69 parts
Sorpol 5050 10 parts
(Mixture of anionic surfactant and white carbon, trade name by Toho Chemical Industrial Co.)
Carplex #80 1 part
(White carbon, trade name by Shionogi & Co.)

The above-mentioned ingredients except the active ingredient (the compound of the invention) were uniformly dissolved, and then the compound of the invention was added thereto and well stirred, and thereafter the resulting mixture was wet-milled in a sand mill, to obtain flowables. For the practical use, the said flowables is diluted into a 1/1,500 to 1/150,000 solution, and this is applied in an amount of form 10 to 1000 g/ha, as the active ingredient.

Formulation example 8: Dust The compound of the invention 10 parts
Clay 90parts

The above ingredients were uniformly blended to obtain dust. For the practical use, this may be applied in an amount of from 10 to 1000 g/ha, as the active ingredient.

Formulation example 9: Granulars The compound of the invention 5 parts
Bentonite 25 parts
Talc 70 parts

The above ingredients were uniformly mixed and ground and then a small amount of water was added thereto and stirred and kneaded. The resulting mixture was granulated from an extrusion granulator and dried to obtain granulars.

For the practical use, the said granulars may be sprayed in an amount of from 10 to 1000 g/ha, as the active ingredient.

Next, the effect of the compounds of the present invention is concretely described hereinafter by reference to biological tests.

Test example 1:

(1) Test of preventive efficacy against Pseudoperonospora cubensis:

When cucumber plants (cultivar: Sagami-hanjiro), which were growing in pots each having a diameter of 7 cm, became 1-to 2-leaf stage, the compound of the present invention, which was in the form of the emulsion concentrate as formed in accordance with the above-mentioned formulation example 2 and which was diluted with water to 500 ppm, was sprayed thereover with a gun type sprayer in an amount of 20 ml/pot. Next day after the spraying, a suspension of spores of Pseudoperonospora cubensis ($2\times10^5$/ml) was sprayed over the plants, and the pots were put in an inoculation box kept at a temperature of 25°C and a humidity of 95 % or more for a whole day and night. Afterwards, the pots were put in a greenhouse, and 7th day after the inoculation, the proportion of the diseased area on the leaf in each plant was measured. The protective value was calculated on the basis of the following formula:

$$\text{Protective Value} = \left(1 - \frac{\text{Proportion of diseased area on the leaves of test plants}}{\text{Proportion of diseased area on the leaves of control plants}}\right) \times 100$$

The test results obtained are shown in Table 6 below.

Table 6
(Concentration on treatment: 500ppm)

| Compound No. | Inhibitory Value | Compound No. | Inhibitory Value |
|---|---|---|---|
| 2 | 100 | 727 | 100 |
| 12 | 100 | 736 | 100 |
| 16 | 100 | 738 | 100 |
| 17 | 100 | 739 | 100 |
| 26 | 100 | 745 | 100 |
| 27 | 100 | 753 | 100 |
| 29 | 100 | 762 | 100 |
| 30 | 100 | 763 | 100 |
| 33 | 100 | 765 | 100 |
| 35 | 100 | 766 | 100 |
| 36 | 100 | 770 | 100 |
| 46 | 100 | 771 | 100 |
| 47 | 100 | 812 | 100 |
| 53 | 100 | 814 | 100 |
| 57 | 100 | 815 | 100 |
| 58 | 100 | 820 | 100 |
| 59 | 100 | 821 | 100 |
| 80 | 100 | 824 | 100 |
| 91 | 100 | 825 | 100 |
| 92 | 100 | 827 | 100 |
| 183 | 100 | 884 | 100 |
| 184 | 100 | 910 | 100 |
| 372 | 100 | zineb | 65 |
| 488 | 100 | Compound A | 100 |
| 498 | 100 | Compound B | 100 |
| 726 | 100 | Compound C | 100 |

The "zineb" in Table 6 is Dithane as a trade name, or zinc ethylene-bis-dithiocarbamate as a chemical name. The Compound A, Compound B and Compound C are control compounds as mentioned hereinafter.

Test example 2:

(2) Test of preventive effeacy against Pseudoperonospora cubensis:

The procedure of Test example 1 was repeated, except that the concentration of the compound in the agent to be applied to plants was varied from 500 ppm to 100 ppm. The test results obtained are shown in Table 7 below.

Table 7
(Concentration on treatment: 100ppm)

| Compound No. | Inhibitory Value | Compound No. | Inhibitory Value |
|---|---|---|---|
| 16 | 100 | 736 | 100 |
| 17 | 100 | 738 | 100 |
| 26 | 100 | 739 | 100 |
| 27 | 100 | 745 | 100 |
| 29 | 100 | 762 | 100 |
| 30 | 100 | 763 | 100 |
| 33 | 100 | 765 | 100 |
| 35 | 100 | 766 | 100 |
| 36 | 100 | 770 | 100 |
| 46 | 100 | 771 | 100 |
| 47 | 100 | 812 | 100 |
| 53 | 100 | 814 | 100 |
| 57 | 100 | 815 | 100 |
| 58 | 100 | 820 | 100 |
| 59 | 100 | 824 | 100 |
| 80 | 100 | 825 | 100 |
| 91 | 100 | 827 | 100 |
| 92 | 100 | 884 | 100 |
| 183 | 100 | 910 | 100 |
| 184 | 100 | zineb | 0 |
| 372 | 100 | Compound A | 90 |
| 488 | 100 | Compound B | 100 |
| 498 | 100 | Compound C | 0 |

Test example 3:

(1) Test of curative efficacy against Pseudoperonospora cubensis:

When cucumber plants (cultivar: Sagami-hanjiro), which were growing in pots each having a diameter of 7 cm, became 1-to 2-leaf stage, a suspension of spores of Pseudoperonospora cubensis ($2 \times 10^5$ /ml) was applied thereover, and the pots were put in an inoculation box kept at a temperature of 25°C and a humidity of 95 % or more for a whole day and night for inoculation of the said spores to the said plants. Next day, the compound of the present invention, which was in the form of the emulsifiable concentrate as formed in accordance with the above-mentioned formulation example 2 and which was diluted with water to 500 ppm, was applied over the plants with a gun type sprayer in an amount of 20 ml/pot. Afterwards, the pot were put in a greenhouse, and 7th day after the inoculation, the proportion of the diseased area on the leaf in each plant was measured. The protective value was calculated on the basis of the following formula:

$$\text{Protective Value} = \left(1 - \frac{\text{Proportion of diseased area on the leaves of test plants}}{\text{Proportion of diseased area on the leaves of control plants}}\right) \times 100$$

The test results obtained are shown in Table 8 below.

Table 8
(Concentration on treatment: 500ppm)

| Compound No. | Inhibitory Value | Compound No. | Inhibitory Value |
|---|---|---|---|
| 2 | 100 | 372 | 100 |
| 16 | 100 | 488 | 100 |
| 17 | 100 | 498 | 100 |
| 26 | 100 | 736 | 100 |
| 27 | 100 | 762 | 100 |
| 29 | 100 | 763 | 100 |
| 30 | 100 | 765 | 100 |
| 33 | 100 | 766 | 100 |
| 35 | 100 | 770 | 100 |
| 36 | 100 | 771 | 100 |
| 46 | 100 | 814 | 100 |
| 47 | 100 | 815 | 100 |
| 53 | 100 | 820 | 100 |
| 57 | 100 | 824 | 100 |
| 58 | 100 | 825 | 100 |
| 59 | 100 | 827 | 100 |
| 80 | 100 | 910 | 100 |
| 91 | 100 | zineb | 0 |
| 92 | 100 | Compound A | 100 |
| 169 | 100 | Compound B | 100 |
| 183 | 100 | Compound C | 50 |
| 184 | 100 | | |
| 277 | 100 | | |

Test example 4:

(2) Cure test for Pseudoperonospora cubensis:

The procedure of Test example 3 was repeated, except that the concentration of the compound in the agent to be applied to plants were varied from 500 ppm to 100 ppm. The test results obtained are shown in Table 9 below.

Table 9
(Concentration on treatment: 100ppm)

| Compound No. | Inhibitory Value | Compound No. | Inhibitory Value |
|---|---|---|---|
| 16 | 100 | 736 | 100 |
| 17 | 100 | 738 | 100 |
| 26 | 100 | 762 | 100 |
| 27 | 100 | 763 | 100 |
| 29 | 100 | 765 | 100 |
| 30 | 100 | 766 | 100 |
| 33 | 100 | 770 | 100 |
| 35 | 100 | 771 | 100 |
| 36 | 100 | 790 | 100 |
| 46 | 100 | 812 | 100 |
| 47 | 100 | 814 | 100 |
| 53 | 100 | 815 | 100 |
| 57 | 100 | 820 | 100 |
| 58 | 100 | 824 | 100 |
| 59 | 100 | 825 | 100 |
| 80 | 100 | 827 | 100 |
| 91 | 100 | 884 | 100 |
| 92 | 100 | 910 | 100 |
| 183 | 100 | zineb | 0 |
| 184 | 100 | Compound A | 78 |
| 277 | 100 | Compound B | 100 |
| 372 | 100 | Compound C | 0 |
| 488 | 100 | | |
| 498 | 100 | | |

Test example 5:

Test of chemical phytotoxicity:

The compound of the present invention, which was in the form of the emulsion concentrate as formed in accordance with the above-mentioned formulation Example 2 and which was adjusted to have a desired concentration, was sprayed over cucumber plants (Sagami-hanjiro 2-ot 3-leaf stage), which were growing in pots each having a diameter of 7 cm, with a gun type sprayer in an amount of 20 ml/pot. The cucumber plants were further grown in a greenhouse for 7 days, and the degree of the test of chemical phytotoxicity of each plant was investigated on the basis of the following evaluation standard. Evaluaiton Standard:

5 : Completely died.
4 : Grwoth was remarkably inhibited and the plants partly died.
3 : Growth was remarkably inhibited.
2 : Growth was somewhat inhibited.

1 : Growth was slightly inhibited.
0 : Growth was normal.
The test results obtained are shown in Table 10 below.

Table 10

| Compound No. | 500 | 100 | 50 (ppm) |
|---|---|---|---|
| 2 | 0 | 0 | 0 |
| 12 | 0 | 0 | 0 |
| 16 | 0 | 0 | 0 |
| 17 | 0 | 0 | 0 |
| 26 | 0 | 0 | 0 |
| 27 | 0 | 0 | 0 |
| 30 | 0 | 0 | 0 |
| 33 | 0 | 0 | 0 |
| 35 | 0 | 0 | 0 |
| 36 | 0 | 0 | 0 |
| 80 | 0 | 0 | 0 |
| 91 | 0 | 0 | 0 |
| 92 | 0 | 0 | 0 |
| 169 | 0 | 0 | 0 |
| 183 | 0 | 0 | 0 |
| 184 | 0 | 0 | 0 |
| 277 | 0 | 0 | 0 |
| 372 | 0 | 0 | 0 |
| 488 | 0 | 0 | 0 |
| 726 | 0 | 0 | 0 |
| 727 | 0 | 0 | 0 |
| 736 | 0 | 0 | 0 |
| 738 | 0 | 0 | 0 |
| 739 | 0 | 0 | 0 |
| 745 | 0 | 0 | 0 |
| 753 | 0 | 0 | 0 |
| 763 | 0 | 0 | 0 |
| 765 | 0 | 0 | 0 |
| 766 | 0 | 0 | 0 |
| 771 | 0 | 0 | 0 |

Table 10 (Continued)

| Compound No. | 500 | 100 | 50 (ppm) |
|---|---|---|---|
| 812 | 0 | 0 | 0 |
| 814 | 0 | 0 | 0 |
| 815 | 0 | 0 | 0 |
| 820 | 0 | 0 | 0 |
| 821 | 0 | 0 | 0 |
| 824 | 0 | 0 | 0 |
| 825 | 0 | 0 | 0 |
| 884 | 0 | 0 | 0 |
| 910 | 0 | 0 | 0 |
| Referential Compound A | 3 | 3 | 2 |
| Referential Compound B | 3 | 3 | 3 |
| Referential Compound C | 3 | 2 | 2 |

The Referential Compound A to C in Tables 6 to 10 are as follows:

Compound A: Described in Japanese Patent Laid-Open Application No. 255759/85.

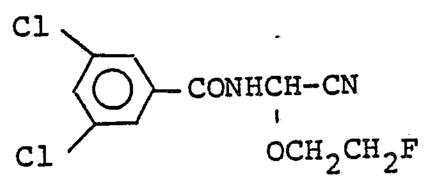

Compound B: Described in Japanese Patent Laid-Open Application No. 69866/83.

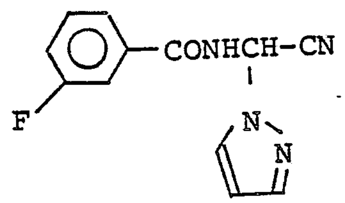

Compound C: Described in Japanese Patent Laid-Open Application No. 167978/82.

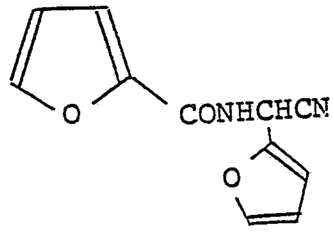

The above-mentioned biological test results apparently indicate that the compounds of the present invention have both preventive effect and curative effect and are noticeably effective against diseases caused by fungi of Phycomycetes, such as, for example, Pseudoperonospora cubensis-caused disease. In addition, the chemical phytotoxicity by the compounds of the present invention is little, as opposed to the other known compounds A to C.

While the invention has been described in detial and with reference to specific embodiemnts thereof, it will be apparent to one skilled in the art that various changes and modifications can be made therein without departing from the spirit and scope thereof.

## Claims

1. An amide derivative of a general formula (I):

A-CONH C H-D (I)
B

in which A represents a 5-membered hetero-aromatic group of a formula:

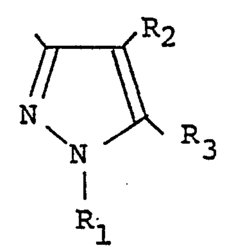,
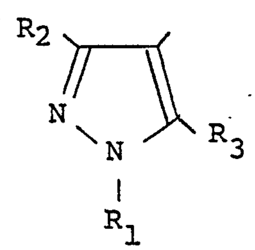,
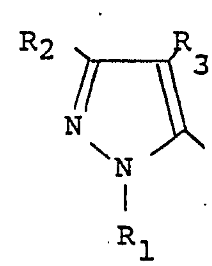,

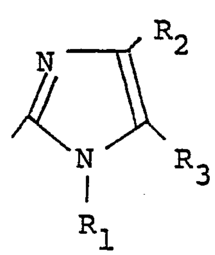,
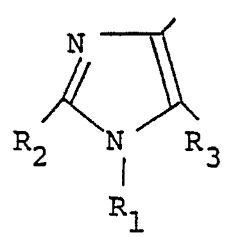,
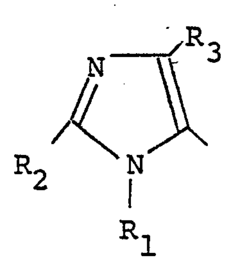,

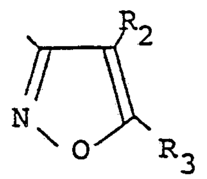
,
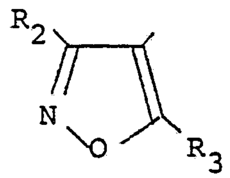
,
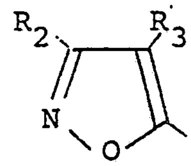
,

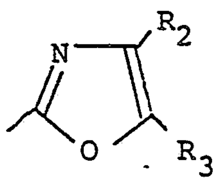
,
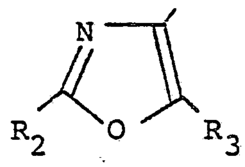
,
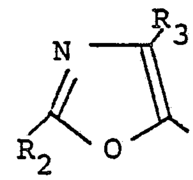
,

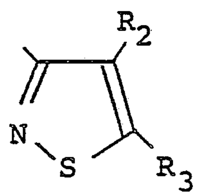
,
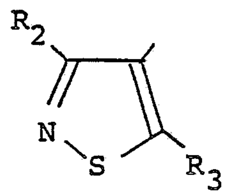
,
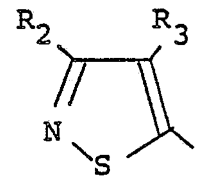
,

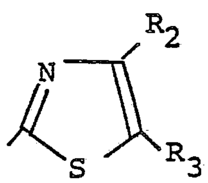
,
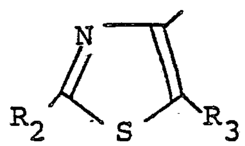
or

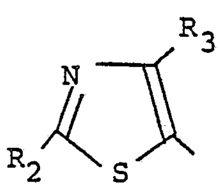
;

B represents a 5-membered hetero-aromatic group of a formula:

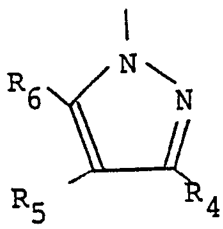,
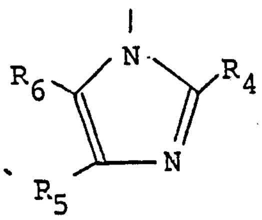,
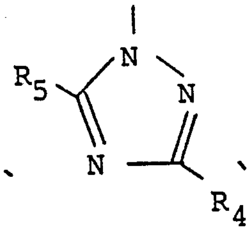,
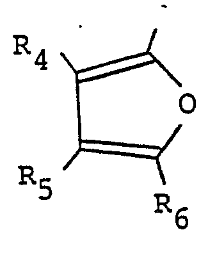

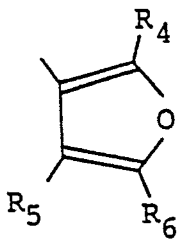,
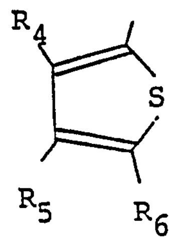
or
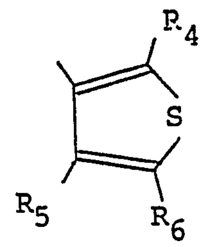

or represents a group of $OR_7$ or $SR_7$;

D represents a cyano, thiocarbamoyl or acylthiocarbamoyl group of -CN, $-CSNH_2$ or $-CSNHCOR_8$;

in the groups, $R_1$ represents a hydrogen atom, a lower alkyl group having from 1 to 4 carbon atoms, a halogenated alkyl group having from 1 to 4 carbon atoms, an alkenyl group having from 2 to 4 carbon atoms, a halogenated alkenyl group having from 2 to 4 carbon atoms, an alkynyl group having from 2 to 4 carbon atoms or an allenyl group having from 3 to 5 carbon atoms;

$R_2$, $R_3$ and $R_4$ independently represent a hydrogen atom, a lower alkyl group having from 1 to 4 carbon atoms, a halogen atom, a halogenated alkyl group having from 1 to 4 carbon atoms, an alkoxy group having from 1 to 4 carbon atoms, a cyano group or a lower alkylthio group having from 1 to 4 carbon atoms;

$R_4$, $R_5$ and $R_6$ independently represent a hydrogen atom, a lower alkyl group having from 1 to 4 carbon atoms, a halogen atom or a halogenated alkyl group having from 1 to 4 carbon atoms;

$R_7$ represents a lower alkyl group having from 1 to 4 carbon atoms, a halogenated alkyl group having from 1 to 4 carbon atoms, an alkenyl group having from 2 to 4 carbon atoms, an alkynyl group having from 2 to 4 carbon atoms, an alkoxyalkyl group having from 3 to 4 carbon atoms, a halogenated alkynyl group having from 3 to 6 carbon atoms, a cyanoalkyl group having from 2 to 4 carbon atoms or a halogenated alkenyl group having from 2 to 4 carbon atoms; and

$R_8$ represents a lower alkyl group having from 1 to 4 carbon atoms, a cyclohexyl group or a phenyl group.

2. An amide derivative as claimed in claim 1, which is represented by a general formula (I):

A-CONH C H-D (I)
B

in which A represents a 5-membered hetero-aromatic group of a formula:

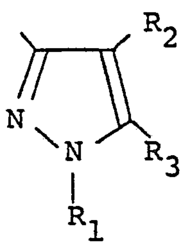, 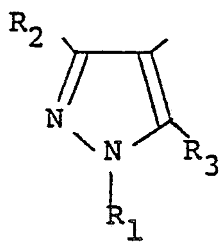, 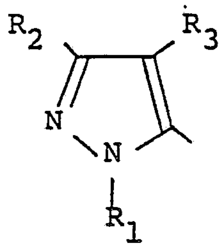, 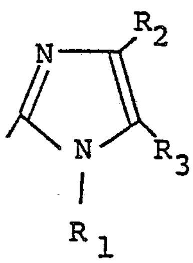, 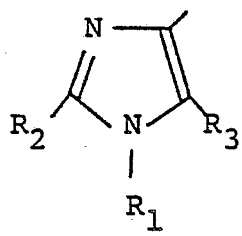, 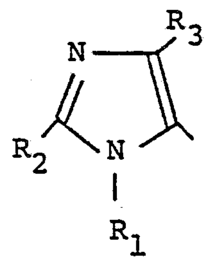,

B represents a 5-membered hetero-aromatic group of a formula:

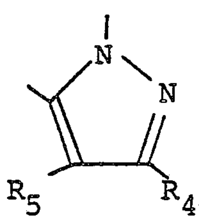, 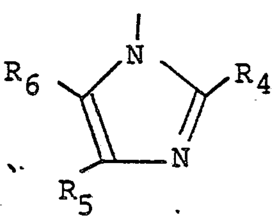, 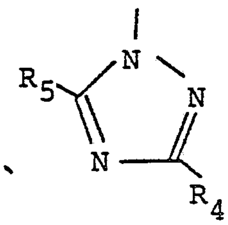, 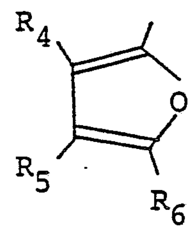, 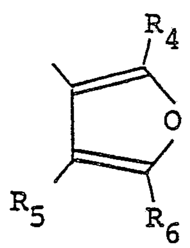, 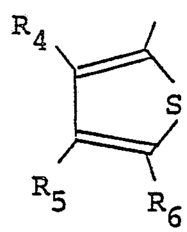 or 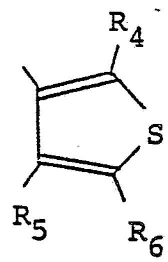

or represents a group of $OR_7$ or $SR_7$;

D represents a cyano, thiocarbamoyl or acylthiocarbamoyl group of -CN, $-CSNH_2$ or $-CSNHCOR_8$;

in the groups, $R_1$ represents a hydrogen atom, a lower alkyl group having from 1 to 4 carbon atoms, a halogenated alkyl group having from 1 to 4 carbon atoms, an alkenyl group having from 2 to 4 carbon atoms, a halogenated alkenyl group having from 2 to 4 carbon atoms, an alkynyl group having from 2 to 4 carbon atoms or an allenyl group having from 3 to 5 carbon atoms;

$R_2$ and $R_3$ independently represent a hydrogen atom, a lower alkyl group having from 1 to 4 carbon atoms, a halogen atom, a halogenated alkyl group having from 1 to 4 carbon atoms, an alkoxy group having from 1 to 4 carbon aotms, a cyano group or a lower alkylthio group having from 1 to 4 carbon atoms;

$R_4$, $R_5$ and $R_6$ independently represent a hydrogen atom, a lower alkyl group having from 1 to 4 carbon atoms, a halogen atom or a halogenated alkyl group having from 1 to 4 carbon atoms;

$R_7$ represents a lower alkyl group having from 1 to 4 carbon atoms, a halogenated alkyl group having from 1 to 4 carbon atoms, an alkenyl group having from 2 to 4 carbon atoms, an alkynyl group having from 2 to 4 carbon atoms, an alkoxyalkyl group having from 3 to 4 carbon atoms, a halogenated alkynyl group having from 3 to 6 carbon atoms, a cyanoalkyl group having form 2 to 4 carbon aotms or a halogenated alkenyl

group having from 2 to 4 carbon atoms; and
$R_8$ represents a lower alkyl group having from 1 to 4 carbon atoms, a cyclohexyl group or a phenyl group.

3. An amide derivative as claimed in claim 1, which is represented by a general formula (IA):

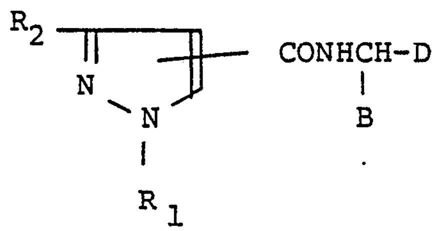

( IA` )

in which $R_1$ represents an alkyl group having from 1 to 3 carbon atoms;
$R_2$ represents a hydrogen atom, a methyl group, a methoxy group or a halogen atom;
D represents -CN or $-\overset{S}{\overset{\|}{C}}-NH_2$; and
B represents

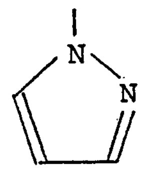
,
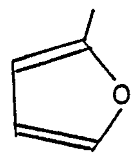
,
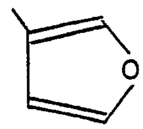
,

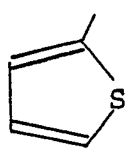
or
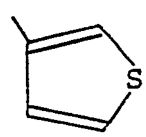

4. An amide derivative as claimed in claim 1, which is selected from the group consisting of:
N-(cyano-3-thienylmethyl)-1,3-dimethyl-1H-pyrazole-5-carboxamide;
N-(cyano-2-thienylmethyl)-1,3-dimethyl-1H-pyrazole-5-carboxamide;
1,3-dimethyl-N-[3-thienyl(thiocarbamoyl)methyl]-1H-pyrazole-5-carboxamide;
1,3-dimethyl-N-[2-thienyl thiocarbamoyl)methyl]-1H-pyrazole-5-carboxamide;
N-(cyano-2-furanylmethyl)-1-methyl-1H-pyrazole-4-carboxamide;
N-(cyano-1H-pyrazol-1-ylmethyl)-1-methyl-1H-pyrazole-4-carboxamide;
N-(cyano-2-furanylmethyl)-1,3-dimethyl-1H-pyrazole-5-carboxamide;
1-ethyl-3-methyl-N-[3-thienyl(thiocarbamoyl)methyl]-1H-pyrazole-5-carboxamide;
N-(cyano-1H-pyrazol-1-ylmethyl)-1,3-dimethyl-1H-pyrazole-5-carboxamide;
N-(cyano-3-furanylmethyl)-1,3-dimethyl-1H-pyrazole-5-carboxamide;
N-(cyano-3-furanylmethyl)-1-ethyl-3-methyl-1H-pyrazole-5-carboxamide;
N-(cyano-3-thienylmethyl)-1-ethyl-3-methyl-1H-pyrazole-5-carboxamide;
N-(cyano-2-thienylmethyl)-1-methyl-1H-pyrazole-4-carboxamide;
3-chloro-N-(cyano-3-thienylmethyl)-1-1H-pyrazole-5-carboxamide; and
N-(cyano-2-furanylmethyl)-3-methoxy-1-methyl-1H-pyrazole-5-carboxamide.

5. A method for preparation of a compound of a general formula (5):

$A-CONH\underset{B}{\underset{|}{C}}HCN$ (5),

characterized by reacting an aminoacetonitrile derivative of a general formula (3):

$H_2N-\underset{B}{\underset{|}{C}}H-CN$ (3)

with an acid halide of a general formula (4):

A-COX (4),

in the presence of an acid acceptor;

in which A represents a 5-membered hetero-aromatic group of a formula:

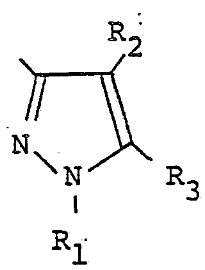 , 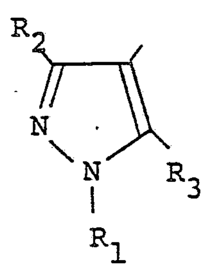 , 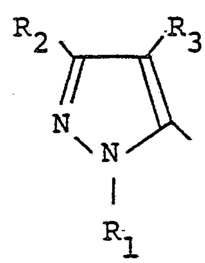

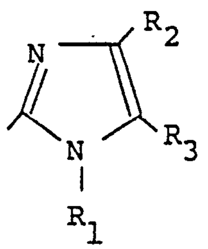 , 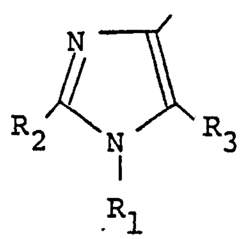 , 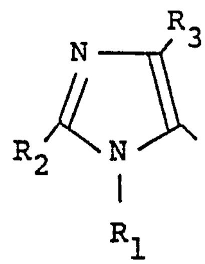 ,

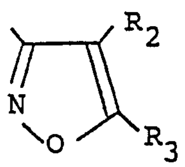 , 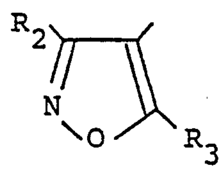 , 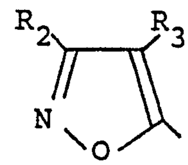 ,

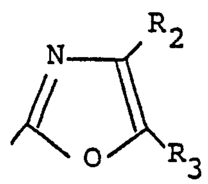 , 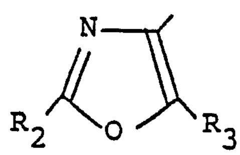 , 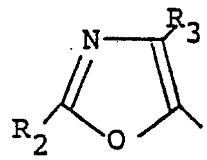 ,

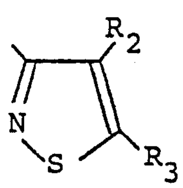 , 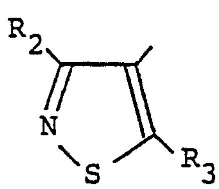 , 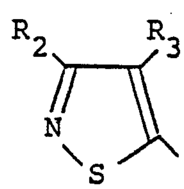 ,

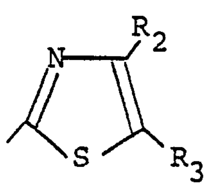 , 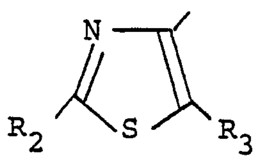 or

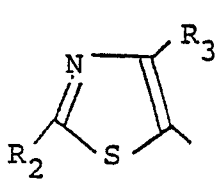 ,

in the groups, $R_1$ represents a hydrogen atom, a lower alkyl group having from 1 to 4 carbon atoms, a halogenated lower alkyl group having from 1 to 4 carbon atoms, an alkenyl group having from 2 to 4 carbon atoms, a halogenated alkenyl group having from 2 to 4 carbon atoms, an alkynyl group having from 2 to 4 carbon atoms or an allenyl group having form 3 to 5 carbon atoms; and
$R_2$ and $R_3$ independently represent a hydrogen atom, a lower alkyl group having from 1 to 4 carbon atoms, a halogen atom, a halogenated alkyl group having from 1 to 4 carbon atoms, an alkoxy group

having from 1 to 4 carbon atoms, a cyano group or a lower alkylthio group having from 1 to 4 carbon atoms;

B represents a 5-membered hetero-aromatic group of a formula:

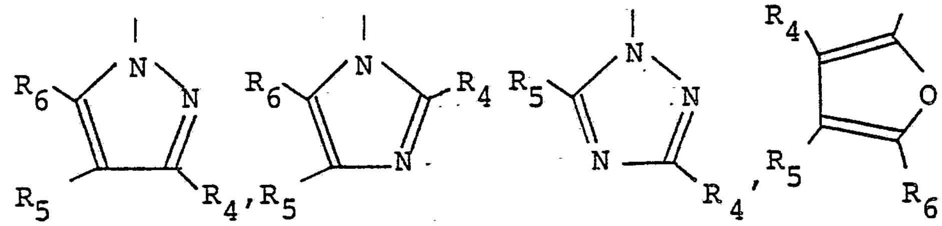

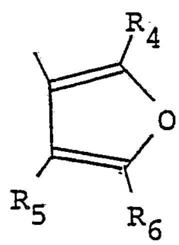

,

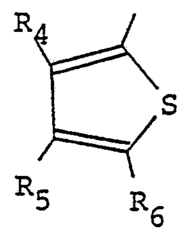

or

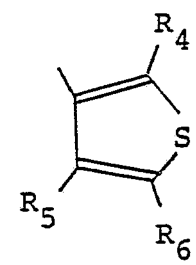

or represents a group of $OR_7$ or SR7;

in the groups, $R_4$, $R_5$ and $R_6$ independently represent a hydrogen atom, a lower alkyl group having from 1 to 4 carbon atoms, a halogen atom or a halogenated alkyl group having from 1 to 4 carbon atoms; and

$R_7$ represents a lower alkyl group having from 1 to 4 carbon atoms, a halogenated alkyl group having from 1 to 4 carbon atoms, an alkenyl group having from 2 to 4 carbon atoms, an alkynyl group having from 2 to 4 carbon atoms, an alkoxyalkyl group having from 3 to 4 carbon atoms, a halogenated alkynyl group having from 3 to 6 carbon aotms, a cyanoalkyl group having from 2 to 4 carbon atoms or a halogenated alkenyl group having from 2 to 4 carbon atoms; and

X represents a halogen atom.

6. A phytopathogenic fungicide containing, as an active ingredient, a compound of a general formula (I):

A-CONH C H-D (I)
B

in which A represents a 5-membered hetero-aromatic group of a formula:

B represents a 5-membered hetero-aromatic group or a formula:

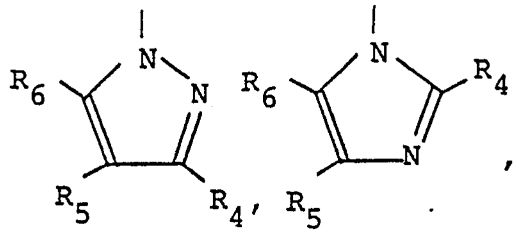

,

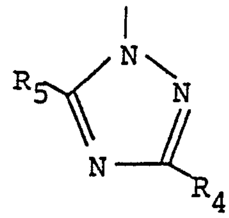

,

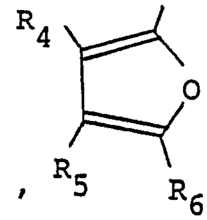

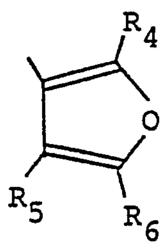

,

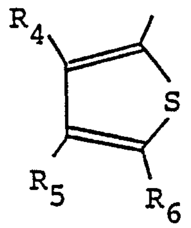

or

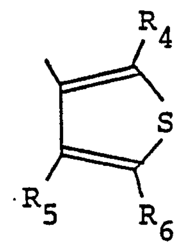

or represents a group of $OR_7$ or $SR_7$;

D represents a cyano, thiocarbamoyl or acylthiocarbamoyl group of -CN, $-CSNH_2$ or $-CSNHCOR_8$;

in the groups, $R_1$ represents a hydrogen atom, a lower alkyl group having from 1 to 4 carbon atoms, a halogenated alkyl group having from 1-4 carbon atoms, an alkenyl group having from 2 to 4 carbon atoms, a halogenated alkenyl group having from 2 to 4 carbon atoms, an alkynyl group having from 2 to 4 carbon atoms or an allenyl group having from 3 to 5 carbon atoms;

$R_2$ and $R_3$ independently represent a hydrogen atom, a lower alkyl group having from 1 to 4 carbon atoms, a halogen atom, a halogenated alkyl group having from 1 to 4 carbon atoms, an alkoxy group having from 1 to 4 carbon atoms, a cyano group or a lower alkylthio group having from 1 to 4 carbon atoms;

$R_4$, $R_5$ and $R_6$ independently represent a hydrogen atom, a lower alkyl group having from 1 to 4 carbon atoms, a halogen atom or a halogenated alkyl group having from 1 to 4 carbon atoms;

$R_7$ represents a lower alkyl group having form 1 to 4 carbon atoms, a halogenated alkyl group having from 1 to 4 carbon atoms, an alkenyl group having from 2 to 4 carbon aotms, an alkynyl group having from 2 to 4 carbon atoms, an alkoxyalkyl group having from 3 to 4 carbon aotms, a halogenated alkynyl group having from 3 to 6 carbon aotms, a cyanoalkyl group having from 2 to 4 carbon aotms or a halogenated alkenyl group haivng from 2 to 4 carbon atoms; and

$R_8$ represents a lower alkyl group having from 1 to 4 carbon atoms, a cyclohexyl group or a phenyl group.

7. Fungicides for agricultural and horticultural use as claimed in claim 6, which contains, as an active ingredient, the compound as claimed in claim 2.

8. Fungicides for agricultural and horticultural use as claimed in claim 6, which contains, as an active ingredient, the compound as claimed in claim 6.

9. Fungicides for agricultural and horticultural use as claimed in claim 1, which contains, as an active ingredient, the compound as claimed in claim 4.

10. A method for protecting against fungi of Phycomycetes, by the use of the compound as claimed in claim 1.

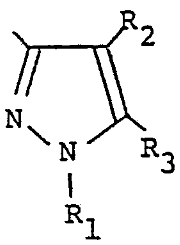 , 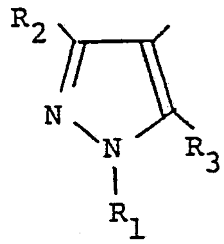 , 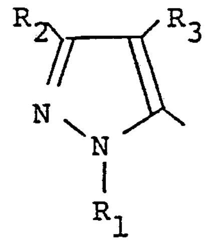 ,

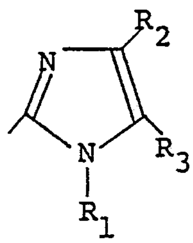 , 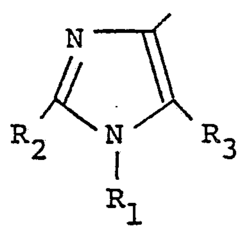 , 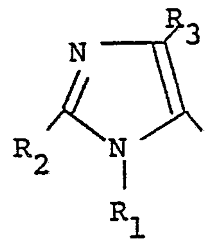 ,

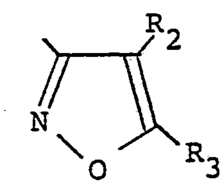 , 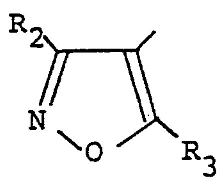 , 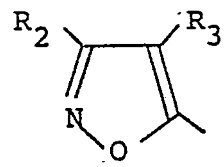 ,

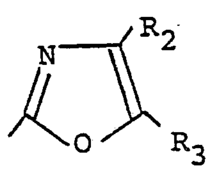 , 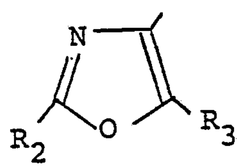 , 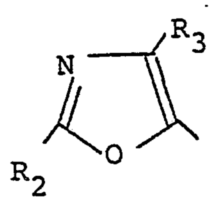 ,

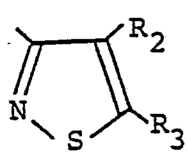 , 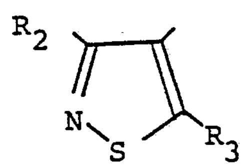 , 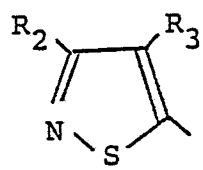 ,

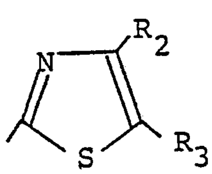 , 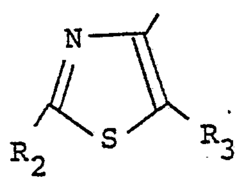 or

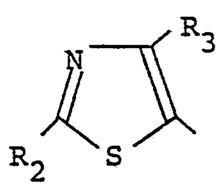 ,